# EUROPEAN PATENT APPLICATION

(11) **EP 4 056 552 A1**
(43) Date of publication of application: **14.09.2022**
(21) Application number: 20884183.3
(22) Date of filing: 08.11.2020
(51) Int. Cl.: C07C 219/00, C07C 229/00, C07C 233/00, C07F 9/09, A61K 48/00, A61P 35/00

(54) **USE OF LIPID IN PREPARATION OF NUCLEIC ACID DELIVERY REAGENT AND RELATED PRODUCT THEREOF**

(30) Priority: 08.11.2019 CN 201911087684
(71) Applicant: Institute of Basic Medical Sciences, Chinese Academy of Medical Sciences, Beijing 100005 (CN)
(72) Inventor: JIANG, Chengyu, Beijing 100005 (CN); QIN, Yuhao, Beijing 100005 (CN); LI, Xiaoyun, Beijing 100005 (CN); ZHANG, Cong, Beijing 100005 (CN)
(74) Representative: Lahrtz, Fritz
(86) International application number: PCT/CN2020/127393
(87) International publication number: WO 2021/089030

(57) **Abstract**

Disclosed are the use of one or more lipid compounds in the delivery of nucleic acids, and a lipid-nucleic acid mixture, a pharmaceutical composition or a kit comprising the lipid compound and nucleic acids. The lipid compounds provided in the present invention can promote the absorption of nucleic acids, particularly via oral absorption, and can promote the entry of nucleic acids at NI target sites in a subject in need thereof.

SEQUENCE LISTING

<110>  INSTITUTE OF BASIC MEDICAL SCIENCES CHINESE ACADEMY OF
       MEDICAL SCIENCES

<120>  USE OF LIPID IN PREPARATION OF NUCLEIC ACID DELIVERY REAGENT AND
       RELATED PRODUCT THEREOF

<130>  074844-8001WO01

<160>  7

<170>  PatentIn version 3.5

<210>  1
<211>  25
<212>  DNA
<213>  Artificial Sequence

<220>
<223>  Synthetic

<400>  1
tccggaatga ttgggcgtaa agcgt                                            25

<210>  2
<211>  50
<212>  DNA
<213>  Artificial Sequence

<220>
<223>  Synthetic

<400>  2
gtcgtatcca gtgcacgctc cgaggtattc gcactggata cgacacgctt                 50

<210>  3
<211>  21
<212>  DNA
<213>  Artificial Sequence

<220>
<223>  Synthetic

<400>  3
tcgcgctccg gaatgattgg g                                                21

<210>  4
<211>  16
<212>  DNA
<213>  Artificial Sequence

<220>
<223>  Synthetic

<400>  4
gtgcacgctc cgaggt                                                      16

<210>  5

<211>  53
<212>  DNA
<213>  Artificial Sequence

<220>
<223>  Synthetic

<400>  5
gtcgtatcca gtgcagggtc cgaggtattc gcactggata cgacaaaaat atg          53


<210>  6
<211>  18
<212>  DNA
<213>  Artificial Sequence

<220>
<223>  Synthetic

<400>  6
gcgcgtcgtg aagcgttc                                                18


<210>  7
<211>  16
<212>  DNA
<213>  Artificial Sequence

<220>
<223>  Synthetic

<400>  7
gtgcagggtc cgaggt                                                  16

## Description

### FIELD OF THE INVENTION

The present invention relates to the field of biology. Particularly, the present invention relates to use of lipid compounds for delivering nucleic acids. These compounds or a variety of combinations thereof can promote the absorption of various nucleic acids in vivo through oral administration, so as to enter a target site, and enter a target cell in a subject in need thereof. These compounds involved in the present invention are extracted and found in traditional Chinese medicines, and can also be synthesized.

### BACKGROUD OF THE INVENTION

Over the past few decades, the idea of using nucleic acid molecules as therapeutic medicaments advanced from concept to clinical practice. In fact, nucleic acid molecules possess many properties that make them useful as therapeutic agents. They can fold into complex conformations to allow them to bind with proteins, small molecules or other nucleic acids, and even form catalytic centers in some cases. For example, small interfering RNA (siRNA) acts as an effector molecule of RNAi, and has an increasingly broad prospect as a therapeutic agent. At present, a variety of siRNA medicaments have entered clinical trials, indicating a good development prospect. Generally, siRNA, miRNA, and other noncoding small RNAs are indiscriminately called small nucleic acids or small RNAs (sRNAs). In addition to small RNAs, nucleic acid molecules that can be used as medicaments also include, for example, mRNA, antisense nucleic acid, and others. However, since nucleic acid molecules such as RNA are prone to degradation, and have a relatively short half-life in the body, they are usually not considered as the optimal choice as a therapeutic agent. Therefore, how to effectively deliver nucleic acid molecules including small RNA, mRNA and others to target organs and target cells in vivo to achieve its biological activity and therapeutic or preventive effect, is a problem in urgent need of solution by those skilled in the art.

### SUMMARY OF THE INVENTION

In an aspect, the present invention provides use of a lipid composition in the manufacture of a reagent for delivering a nucleic acid. The lipid composition includes one or more compounds of Formula (I), or a salt, a hydrate or a solvate thereof: 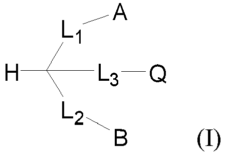 wherein
L₁ is absent, or is -CH₂-O-C(O)-, -CH₂-O- or -CR(OH)-;
L₂ is absent, or is -O-C(O)- or -NH-C(O)-;
L₃ is a linear or branched C₁₋₂₀ alkyl, a linear or branched C₁₋₂₀ alkenyl, a linear or branched C₁₋₂₀ heteroalkyl, a linear or branched C₁₋₂₀ heteroalkenyl or 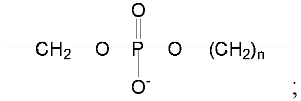
A is a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl;
B is -OH, a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl;
Q is -H, -COOH, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, -N(R)₃⁺ or 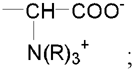
R is each independently H or a linear or branched C₁₋₂₀ alkyl; and
n is 0, 1 or 2.

In certain embodiments, the salt of the compound comprises a pharmaceutically acceptable salt.

In certain embodiments, the compound has the following Formula (II):

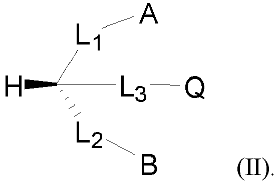

In another aspect, the present invention further provides use of a lipid composition in the manufacture of a reagent for delivering a nucleic acid. The lipid composition comprises one or more compounds selected from the group consisting of: lipids 72, 73, 74, 75, 76, 77, 78, 79, 80, 82, 83, 86, 87, 90, 91, 92, 95, 97, 98, 99, 100, 101, 102, 107, 111, 112, 113, 114, 115, 116, 117, 118, and 119, or a salt, a hydrate or a solvate thereof. The compounds are shown in a Table below:

| **Lipid compound No.** | **Chemical name** |
|---|---|
| 72 | 1 ,2-dioleoyl-sn -glycero-3 -phosphatidylethanolamine |
| 73 | Trilinolenin |
| 74 | 1,2-palmitolein-3 -olein |
| 75 | 1,2-palmitolein-3-palmitin |
| 76 | 1,3 -palmitin-2-myristin |
| 77 | 1,2-stearin-3 -olein |
| 78 | 1,2-olein-3 -arachidin |
| 79 | 1,2-olein-3-behenin |
| 80 | 1,2-myristin-3 -palmitin |
| 82 | Q-10, Ubiquinone 50, Ubiquinone 10 |
| 83 | 1 -palmitoyl-2-hydroxy-sn-glycero-3 -phosphoethanolamine |
| 86 | 1-palmitoyl-2-oleoyl-sn-glycero-3-phosphocholine |
| 87 | 1 -stearoyl-2-oleoyl-sn-glycero-3 -phosphocholine |
| 90 | N-lignoceroyl-D-erythro-sphingosine |
| 91 | N-nervonoyl-D-erythro-sphingosine |
| 92 | 1-arachidoyl-2-hydroxy-sn-glycero-3 -phosphocholine |
| 95 | 1-oleoyl-2-hydroxy-sn-glycero-3 -phosphocholine |
| 97 | D-erythro-sphingosine (C22 group) |
| 98 | D-erythro-sphingosine |
| 99 | D-erythro-sphingosine (C14 group) |
| 100 | D-erythro-sphingosine (C16 group) |
| 101 | D-erythro-sphingosine (C20 group) |
| 102 | D-erythro-sphingosine |
| 107 | 1 ,2-dilinoleoyl-sn -glycero-3 -phosphocholine |
| 111 | 1 -palmitin-3 -stearin |
| 112 | Trieicosanoin |
| 113 | 1 -palmitoyl-2-hydroxy-sn-glycero-3 -phosphatidylcholine |
| 114 | 1-octadecanoyl-2-hydroxy-sn-glycero-3-phosphocholine |
| 115 | 1 -heptadecenoyl-sn-glycero-3 -phosphoethanolamine |
| 116 | 1 -(9Z-octadecenoyl)-sn-glycero-3 -phosphoethanolamine |
| 117 | 1,2-bis-(9Z-octadecenoyl)-sn-glycero-3-phosphocholine |
| 118 | 1-palmitoyl-2-oleoyl-sn-glycero-3 -phosphatidylcholine |
| 119 | 1 ,2-dihexadecanoyl-sn -glycero-3 -phosphocholine |

In certain embodiments, the lipid composition or the reagent can deliver the nucleic acid by oral administration, inhalation or injection. In certain embodiments, the lipid composition or the reagent delivers the nucleic acid by oral administration. In certain embodiments, the delivery comprises in vivo gastrointestinal delivery.

In certain embodiments, the delivery comprises in vitro cell delivery.

In certain embodiments, the lipid composition or the reagent can be used to prepare a lipid-nucleic acid mixture.

The lipid-nucleic acid mixture is prepared by a suitable method, including, but not limited to, a heating method, a reverse evaporation method, or direct mixing.

In certain embodiments, the heating method comprises adding a solution of the lipid composition in an organic solvent to an aqueous solution of the nucleic acid, to obtain a mixed solution, and heating the mixed solution at an appropriate temperature. In certain embodiments, the heating method further comprises cooling the heated mixed solution, to obtain the lipid-nucleic acid mixture.

In some embodiments, the mixed solution is heated at a temperature in a range selected from the group consisting of 25°C to 100°C, 30°C to 100°C, 40°C to 100°C, 50°C to 100°C, 60°C to 100°C, 70°C to 100°C, 80°C to 100°C, 90°C to 100°C, and 95°C to 100°C. In some embodiments, the mixed solution is heated at a temperature selected from the group consisting of 30°C, 35°C, 37°C, 40°C, 45°C, 50°C, 55°C, 60°C, 65°C, 70°C, 75°C, 80°C, 85°C, 90°C, 95°C and 100°C.

In some embodiments, the mixed solution is heated for about 5 min to about 24 hrs, about 5 min to about 20 hrs, about 5 min to about 16 hrs, about 10 min to about 20 hrs, about 10 min to about 16 hrs, about 15 min to about 24 hrs, about 15 min to about 20 hrs, about 30 min to about 24 hrs, about 30 min to about 20 hrs, about 40 min to about 16 hrs, about 50 min to about 12 hrs, about 1 hrs to about 8 hrs, or about 2 hrs to about 4 hrs. In some embodiments, the mixed solution is heated for about 5 min to about 1 hrs, about 5 min to about 30 min, about 5 min to about 15 min, or about 10 min to about 15 min. In some embodiments, the mixed solution is heated for about 5 min, about 10 min, about 15 min, about 20 min, about 25 min, about 30 min, 40 min, 50 min, 1 hr, 2 hrs, 3 hrs, 4 hrs, 5 hrs, 6 hrs, 7 hrs, 8 hrs, 9 hrs, 10 hrs, 12 hrs, 16 hrs, 20 hrs or 24 hrs.

In certain embodiments, the mixed solution is cooled at a temperature in a range selected from the group consisting of 25°C to -80°C, 20°C to -80°C, 15°C to -80°C, 10°C to -80°C, 4°C to -80°C, 0°C to -80°C, -10°C to -80°C, -20°C to -80°C, -30°C to -80°C, and -40°C to -80°C. In some embodiments, the mixed solution is cooled at a temperature selected from the group consisting of 25°C, 20°C, 15°C, 10°C, 4°C or 0°C.

In certain embodiments, the reverse evaporation comprises mixing an aqueous solution of the nucleic acid with a solution of the lipid compound in an organic solvent to obtain a mixed solution. In certain embodiments, the reverse evaporation further comprises removing the organic solvent in the mixed solution, and reconstituting in water, to obtain the lipid-nucleic acid mixture. In certain embodiments, the mixed solution is ultrasonicated and/or evaporated to remove the organic solvent. In certain embodiments, the step of removing the organic solvent in the mixed solution is carried out at an appropriate temperature.

In some embodiments, the organic solvent in the mixed solution is removed at a temperature in a range selected from the group consisting of about 25°C to about 70°C, 30°C to about 70°C, about 30°C to about 65°C, about 40°C to about 65°C, about 40°C to about 60°C, or about 50°C to about 60°C. In some embodiments, the organic solvent in the mixed solution is removed at a temperature selected from the group consisting of about 25°C, about 30°C, about 35°C, about 40°C, about 45°C, about 50°C, about 55°C, about 60°C, about 65°C and about 70°C.

In another aspect, the present invention provides a method for delivering a nucleic acid to an individual in need thereof, which comprises administering the lipid composition and the nucleic acid to the individual by oral administration, inhalation or injection. In certain embodiments, the lipid composition and the nucleic acid are administered as a lipid-nucleic acid mixture.

In certain embodiments, the nucleic acid comprises DNA or RNA. In certain embodiments, the DNA is, for example, non-coding DNA (such as antisense DNA) or coding DNA. In certain embodiments, the RNA is antisense nucleic acid, mRNA, IncRNA, or small RNA (for example, miRNA, siRNA, piRNA, snoRNA, and tsRNA), and so on.

In certain embodiments, the nucleic acid comprises a small nucleic acid at a length of 14-32 bp, 16-28 bp or 18-24 bp.

In certain embodiments, the nucleic acid is single-stranded or double-stranded.

In certain embodiments, the nucleic acid has a stem-loop structure.

In certain embodiments, the nucleic acid is used for treating disease.

In certain embodiments, the nucleic acid is used for treating cancer inflammation, fibrotic disease, autoimmune disease or autoinflammatory disease, bacterial infection, behavioral and psychiatric disorder, hematological disease, chromosomal disorder, congenital and genetic disease, connective tissue disease, digestive disease, ear, nose, and throat disease, endocrine disease, environmental illness, eye disease, female reproductive system disease, fungal infection, heart disease, hereditary cancer syndromes, immune system disorder, kidney and urinary tract disorder, pulmonary disease, male reproductive system disease, metabolic disorder, mouth disease, musculoskeletal disorder, myelodysplastic syndrome, newborn screening, nutritional disease, parasitic disease, rare cancer, rare disease, and skin disease and viral infection.

In certain embodiments, the nucleic acid is used for treating hepatocellular carcinoma, corneal neovascularization, recurrent or refractory anaplastic astrocytoma (WHO grade III) or secondary glioblastoma (WHO grade IV), advanced squamous cell lung cancer, acromegaly, psoriasis, Duchenne muscular dystrophy, advanced non-small cell lung cancer, metastatic castration-resistant prostate cancer, cytomegalovirus retinitis, HIV infection, Hepatitis B, Hepatitis C, hyperlipoproteinemia, total knee replacement, Diabetes mellitus type II, familial amyloid polyneuropathy (FAP), wet macular degeneration (e.g., neovascular age-related macular degeneration, subfoveal neovascular age-related macular degeneration, and exudative age-related macular degeneration), hypercholesterolemia, Crohn's disease, extensive liver fibrosis, infantile spinal muscular atrophy, melanoma, neonatal coronary artery disease, mild allergic asthma, chronic lymphocytic leukemia, hypertriglyceridemia, hepatic veno-occlusive disease complicated with renal or pulmonary dysfunction after hematopoietic stem cell transplantation, and hereditary transthyretin amyloidosis.

In another aspect, the present invention further provides a pharmaceutical composition, comprising a lipid composition and a nucleic acid, wherein the lipid composition comprises one or more compounds of Formula (I), or a pharmaceutically acceptable salt, hydrate or solvate thereof. In certain embodiments, the lipid composition and the nucleic acid exist in the form of a lipid-nucleic acid mixture.

In another aspect, the present invention further provides a pharmaceutical composition, comprising a lipid composition and a nucleic acid, wherein the lipid composition comprises one or more compounds selected from the group consisting of lipids 72, 73, 74, 75, 76, 77, 78, 79, 80, 82, 83, 86, 87, 90, 91, 92, 95, 97, 98, 99, 100, 101, 102, 107, 111, 112, 113, 114, 115, 116, 117, 118, and 119, or a salt, a hydrate or a solvate thereof. In certain embodiments, the lipid composition and the nucleic acid exist in the form of a lipid-nucleic acid mixture.

In another aspect, the present invention further provides use of the pharmaceutical composition provided herein in the manufacture of a medicament for preventing and/or treating disease that can be prevented and/or treated by the nucleic acid, or for in vivo delivering the nucleic acid to a subject in need thereof.

In another aspect, the present invention further provides a kit comprising one or more compounds of Formula (I), or a pharmaceutically acceptable salt, hydrate or solvate provided in a first container, and a nucleic acid provided in a second container.

In another aspect, the present invention further provides a kit comprising one or more compounds, or a pharmaceutically acceptable salt, hydrate or solvate provided in a first container, and a nucleic acid provided in a second container. The one or more compounds are selected from the group consisting of lipids 72, 73, 74, 75, 76, 77, 78, 79, 80, 82, 83, 86, 87, 90, 91, 92, 95, 97, 98, 99, 100, 101, 102, 107, 111, 112, 113, 114, 115, 116, 117, 118, and 119.

In another aspect, the present invention provides use of the kit provided herein in the manufacture of a medicament for preventing and/or treating disease that can be prevented and/or treated by the nucleic acid, or for in vivo delivering the nucleic acid to a subject in need thereof.

In another aspect, the present invention provides a method for delivering a nucleic acid to a target cell, comprising administering, to the target cell, the pharmaceutical composition provided herein, or a lipid-nucleic acid mixture prepared with the kit provided herein.

In another aspect, the present invention provides a method for in vivo delivering a nucleic acid to a subject in need thereof, comprising administering, to the subject, the pharmaceutical composition provided herein, or a lipid-nucleic acid mixture prepared with the kit provided herein.

It should be understood that within the scope of the present invention, the technical features described above and the technical features specifically described below (in the examples) in the present invention may be combined with each other to constitute a new or preferred technical solution. For the sake of brevity, these combinations are not described here.

### BRIEF DESCRIPTION OF THE DRAWINGS

Fig. 1 shows the results of delivering PGY-sRNA-26 to a heat tissue (Fig. 1a), a lung tissue (Fig. 1b), a spleen tissue (Fig. 1c) and blood (Fig. 1d) of mice in a blank group, a free intake group, and a lipid 72 treatment group respectively.
Fig. 2 shows the results of delivering PGY-sRNA-26 to a heat tissue (Fig. 2a), a spleen tissue (Fig. 2b) and blood (Fig. 2c) of mice in a blank group, a free intake group, and a lipid 73 treatment group respectively.
Fig. 3 shows the results of delivering PGY-sRNA-26 to a heat tissue (Fig. 3a), a lung tissue (Fig. 3b), a spleen tissue (Fig. 3c) and blood (Fig. 3d) of mice in a blank group, a free intake group, and a lipid 74 treatment group respectively.
Fig. 4 shows the results of delivering PGY-sRNA-26 to a heat tissue (Fig. 4a), a lung tissue (Fig. 4b), a spleen tissue (Fig. 4c) and blood (Fig. 4d) of mice in a blank group, a free intake group, and a lipid 75 treatment group respectively.
Fig. 5 shows the results of delivering PGY-sRNA-26 to a heat tissue (Fig. 5a), a lung tissue (Fig. 5b), a spleen tissue (Fig. 5c) and blood (Fig. 5d) of mice in a blank group, a free intake group, and a lipid 76 treatment group respectively.
Fig. 6 shows the results of delivering PGY-sRNA-26 to a heat tissue (Fig. 6a), a lung tissue (Fig. 6b), a kidney tissue (Fig. 6c), a spleen tissue (Fig. 6d) and blood (Fig. 6e) of mice in a blank group, a free intake group, and a lipid 77 treatment group respectively.
Fig. 7 shows the results of delivering PGY-sRNA-26 to a heat tissue (Fig. 7a), a lung tissue (Fig. 7b), a kidney tissue (Fig. 7c) and a spleen tissue (Fig. 7d) of mice in a blank group, a free intake group, and a lipid 78 treatment group respectively.
Fig. 8 shows the results of delivering PGY-sRNA-26 to a heat tissue (Fig. 8a), a kidney tissue (Fig. 8b) and a spleen tissue (Fig. 8c) of mice in a blank group, a free intake group, and a lipid 79 treatment group respectively.
Fig. 9 shows the results of delivering PGY-sRNA-26 to a heat tissue (Fig. 9a), a lung tissue (Fig. 9b), a kidney tissue (Fig. 9c) and a spleen tissue (Fig. 9d) of mice in a blank group, a free intake group, and a lipid 80 treatment group respectively.
Fig. 10 shows the results of delivering PGY-sRNA-26 to a heat tissue (Fig. 10a), a kidney tissue (Fig. 10b) and a spleen tissue (Fig. 10c) of mice in a blank group, a free intake group, and a lipid 81 treatment group respectively.
Fig. 11 shows the results of delivering PGY-sRNA-26 to a lung tissue (Fig. 11a), a spleen tissue (Fig. 11b) and a heat tissue (Fig. 11c) of mice in a blank group, a free intake group, and a lipid 82 treatment group respectively.
Fig. 12 shows the results of delivering PGY-sRNA-26 to a spleen tissue (Fig. 12a), a kidney tissue (Fig. 12b) and an intestinal tissue (Fig. 12c) of mice in a blank group, a free intake group, and a lipid 83 treatment group respectively.
Fig. 13 shows the results of delivering PGY-sRNA-26 to a lung tissue (Fig. 13a) and a spleen tissue (Fig. 13b) of mice in a blank group, a free intake group, and a lipid 84 treatment group respectively.
Fig. 14 shows the results of delivering PGY-sRNA-26 to a lung tissue (Fig. 14a), a spleen tissue (Fig. 14b) and a kidney tissue (Fig. 14c) of mice in a blank group, a free intake group, and a lipid 85 treatment group respectively.
Fig. 15 shows the results of delivering PGY-sRNA-26 to an intestinal tissue (Fig. 15a) and a kidney tissue (Fig. 15b) of mice in a blank group, a free intake group, and a lipid 86 treatment group respectively.
Fig. 16 shows the results of delivering PGY-sRNA-26 to a kidney tissue (Fig. 16a), a lung tissue (Fig. 16b) and a spleen tissue (Fig. 16c) of mice in a blank group, a free intake group, and a lipid 87 treatment group respectively.
Fig. 17 shows the results of delivering PGY-sRNA-26 to blood (Fig. 17a), a lung tissue (Fig. 17b), a spleen tissue (Fig. 17c) and a kidney tissue (Fig. 17d) of mice in a blank group, a free intake group, and a lipid 88 treatment group respectively.
Fig. 18 shows the results of delivering PGY-sRNA-26 to blood (Fig. 18a), a lung tissue (Fig. 18b) and a kidney tissue (Fig. 18c) of mice in a blank group, a free intake group, and a lipid 89 treatment group respectively.
Fig. 19 shows the results of delivering PGY-sRNA-26 to blood (Fig. 19a), a lung tissue (Fig. 19b), a spleen tissue (Fig. 19c) and a kidney tissue (Fig. 19d) of mice in a blank group, a free intake group, and a lipid 90 treatment group respectively.
Fig. 20 shows the results of delivering PGY-sRNA-26 to blood (Fig. 20a), a lung tissue (Fig. 20b), a spleen tissue (Fig. 20c) and a kidney tissue (Fig. 20d) of mice in a blank group, a free intake group, and a lipid 91 treatment group respectively.
Fig. 21 shows the results of delivering PGY-sRNA-26 to blood (Fig. 21a), a lung tissue (Fig. 21b), a spleen tissue (Fig. 21c) and a kidney tissue (Fig. 21d) of mice in a blank group, a free intake group, and a lipid 92 treatment group respectively.
Fig. 22 shows the results of delivering PGY-sRNA-26 to blood (Fig. 22a), a lung tissue (Fig. 22b), a spleen tissue (Fig. 22c) and a kidney tissue (Fig. 22d) of mice in a blank group, a free intake group, and a lipid 93 treatment group respectively.
Fig. 23 shows the results of delivering PGY-sRNA-26 to blood (Fig. 23a), a lung tissue (Fig. 23b), a spleen tissue (Fig. 23c) and a kidney tissue (Fig. 23d) of mice in a blank group, a free intake group, and a lipid 94 treatment group respectively.
Fig. 24 shows the results of delivering PGY-sRNA-26 to blood (Fig. 24a), a lung tissue (Fig. 24b), a kidney tissue (Fig. 24c) and a spleen tissue (Fig. 24d) of mice in a blank group, a free intake group, and a lipid 95 treatment group respectively.
Fig. 25 shows the results of delivering PGY-sRNA-26 to blood (Fig. 25a), a lung tissue (Fig. 25b), a kidney tissue (Fig. 25c) and a spleen tissue (Fig. 25d) of mice in a blank group, a free intake group, and a lipid 96 treatment group respectively.
Fig. 26 shows the results of delivering PGY-sRNA-26 to blood (Fig. 26a), a lung tissue (Fig. 26b), a kidney tissue (Fig. 26c) and a spleen tissue (Fig. 26d) of mice in a blank group, a free intake group, and a lipid 97 treatment group respectively.
Fig. 27 shows the results of delivering PGY-sRNA-26 to blood (Fig. 27a), a lung tissue (Fig. 27b), a kidney tissue (Fig. 27c) and a spleen tissue (Fig. 27d) of mice in a blank group, a free intake group, and a lipid 98 treatment group respectively.
Fig. 28 shows the results of delivering PGY-sRNA-26 to blood (Fig. 28a), a lung tissue (Fig. 28b), a kidney tissue (Fig. 28c) and a spleen tissue (Fig. 28d) of mice in a blank group, a free intake group, and a lipid 99 treatment group respectively.
Fig. 29 shows the results of delivering PGY-sRNA-26 to blood (Fig. 29a), a lung tissue (Fig. 29b), a kidney tissue (Fig. 29c) and a spleen tissue (Fig. 29d) of mice in a blank group, a free intake group, and a lipid 100 treatment group respectively.
Fig. 30 shows the results of delivering PGY-sRNA-26 to blood (Fig. 30a), a lung tissue (Fig. 30b), a kidney tissue (Fig. 30c) and a spleen tissue (Fig. 30d) of mice in a blank group, a free intake group, and a lipid 101 treatment group respectively.
Fig. 31 shows the results of delivering PGY-sRNA-26 to blood (Fig. 31a), a kidney tissue (Fig. 31b) and a spleen tissue (Fig. 31c) of mice in a blank group, a free intake group, and a lipid 102 treatment group respectively.
Fig. 32 shows the results of delivering PGY-sRNA-26 to blood (Fig. 32a), a kidney tissue (Fig. 32b) and a lung tissue (Fig. 32c) of mice in a blank group, a free intake group, and a lipid 103 treatment group respectively.
Fig. 33 shows the results of delivering PGY-sRNA-26 to blood (Fig. 33a), a kidney tissue (Fig. 33b), a lung tissue (Fig. 33c) and a spleen tissue (Fig. 33d) of mice in a blank group, a free intake group, and a lipid 104 treatment group respectively.
Fig. 34 shows the results of delivering PGY-sRNA-26 to blood (Fig. 34a), a kidney tissue (Fig. 34b) and a lung tissue (Fig. 34c) of mice in a blank group, a free intake group, and a lipid 105 treatment group respectively.
Fig. 35 shows the results of delivering PGY-sRNA-26 to blood (Fig. 35a), a kidney tissue (Fig. 35b) and a spleen tissue (Fig. 35c) of mice in a blank group, a free intake group, and a lipid 107 treatment group respectively.
Fig. 36 shows the results of delivering PGY-sRNA-26 to blood (Fig. 36a), a lung tissue (Fig. 36b), a kidney tissue (Fig. 36c) and a spleen tissue (Fig. 36d) of mice in a blank group, a free intake group, and a lipid 109 treatment group respectively.
Fig. 37 shows the results of delivering PGY-sRNA-26 to blood (Fig. 37a) and a kidney tissue (Fig. 37b) of mice in a blank group, a free intake group, and a lipid 111 treatment group respectively.
Fig. 38 shows the results of delivering PGY-sRNA-26 to a lung tissue (Fig. 38a), a kidney tissue (Fig. 38b) and a spleen tissue (Fig. 38c) of mice in a blank group, a free intake group, and a lipid 112 treatment group respectively.
Fig. 39 shows the results of delivering PGY-sRNA-26 to a lung tissue (Fig. 39a), a kidney tissue (Fig. 39b) and a spleen tissue (Fig. 39c) of mice in a blank group, a free intake group, and a lipid 113 treatment group respectively.
Fig. 40 shows the results of delivering PGY-sRNA-26 to blood (Fig. 40a), a lung tissue (Fig. 40b), a kidney tissue (Fig. 40c) and a spleen tissue (Fig. 40d) of mice in a blank group, a free intake group, and a lipid 114 treatment group respectively.
Fig. 41 shows the results of delivering PGY-sRNA-26 to blood (Fig. 41a), a lung tissue (Fig. 41b), a kidney tissue (Fig. 41c) and a spleen tissue (Fig. 41d) of mice in a blank group, a free intake group, and a lipid 115 treatment group respectively.
Fig. 42 shows the results of delivering PGY-sRNA-26 to a lung tissue (Fig. 42a), a kidney tissue (Fig. 42b) and a spleen tissue (Fig. 42c) of mice in a blank group, a free intake group, and a lipid 116 treatment group respectively.
Fig. 43 shows the results of delivering PGY-sRNA-26 to a lung tissue (Fig. 43a), a kidney tissue (Fig. 43b) and a spleen tissue (Fig. 43c) of mice in a blank group, a free intake group, and a lipid 117 treatment group respectively.
Fig. 44 shows the results of delivering PGY-sRNA-26 to a lung tissue (Fig. 44a), a kidney tissue (Fig. 44b) and a spleen tissue (Fig. 44c) of mice in a blank group, a free intake group, and a lipid 118 treatment group respectively.
Fig. 45 shows the results of delivering PGY-sRNA-26 to blood (Fig. 45a) and a kidney tissue (Fig. 45b) of mice in a blank group, a free intake group, and a lipid 119 treatment group respectively.
Fig. 46 shows the results of the amount of PGY-sRNA-26 delivered by gavage using lipid 93 to a brain tissue (Fig. 46a), a gastric tissue (Fig. 46b), an intestinal tissue (Fig. 46c), a kidney tissue (Fig. 46d), a lung tissue (Fig. 46e) and a spleen tissue (Fig. 46f) of mice at 0 hr (blank group)/3 hrs/6 hrs/9 hrs/12 hrs/24 hrs respectively.
Fig. 47 shows the results of the amount of PGY-sRNA-26 delivered by gavage using lipid 94 to a brain tissue (Fig. 47a), a heat tissue (Fig. 47b), spleen tissue (Fig. 47c), a lung tissue (Fig. 47d), a kidney tissue (Fig. 47e), an intestinal tissue (Fig. 47f) and a gastric tissue (Fig. 47g) of mice at 0 hr (blank group)/3 hrs/6 hrs/9 hrs/12 hrs/24 hrs respectively.
Fig. 48 shows the results of the amount of PGY-sRNA-26 delivered by gavage using lipid 96 to an intestinal tissue (Fig. 48a) and a kidney tissue (Fig. 48b) of mice at 0 hr (blank group)/3 hrs/6 hrs/9 hrs/12 hrs/24 hrs respectively.
Fig. 49 shows the results of the amount of PGY-sRNA-26 delivered by gavage using lipid 100 to a gastric tissue (Fig. 49A), and an intestinal tissue (Fig. 49b) of mice at 0 hr (blank group)/3 hrs/6 hrs/9 hrs/12 hrs/24 hrs respectively.
Fig. 50 shows the results of the amount of PGY-sRNA-26 delivered by tail vein injection using lipid 94 to a spleen tissue (Fig. 50a), a kidney tissue (Fig. 50b), a brain tissue (Fig. 50c), a gastric tissue (Fig. 50d), and an intestinal tissue (Fig. 50e) of mice at 0 hr (blank group)/3 hrs/6 hrs/9 hrs/12 hrs/24 hrs respectively.
Fig. 51 shows the results of the amount of PGY-sRNA-26 delivered by tail vein injection using lipid 96 to a brain tissue (Fig. 51a), an intestinal tissue (Fig. 51b), a kidney tissue (Fig. 51c), a lung tissue (Fig. 5 1d), a liver tissue (Fig. 51e), and a heart tissue (Fig. 51f) of mice at 0 hr (blank group)/3 hrs/6 hrs/9 hrs/12 hrs/24 hrs respectively.
Fig. 52 shows the results of the amount of PGY-sRNA-26 delivered by tail vein injection using lipid 98 to a kidney tissue (Fig. 52a), an intestinal tissue (Fig. 52b), a gastric tissue (Fig. 52c), and a brain tissue (Fig. 52d), a liver tissue (Fig. 52e), and a heart tissue (Fig. 52f) at 0 hr (blank group)/3 hrs/6 hrs/9 hrs/12 hrs/24 hrs respectively.
Fig. 53 shows the results of the amount of PGY-sRNA-26 delivered by tail vein injection using lipid 100 to a kidney tissue (Fig. 53a), and a gastric tissue (Fig. 53B) of mice at 0 hr (blank group)/3 hrs/6 hrs/9 hrs/12 hrs/24 hrs respectively.
Fig. 54 shows the results of the amount of PGY-sRNA-26 delivered by tail vein injection using lipid 101 to a kidney tissue (Fig. 54a), an intestinal tissue (Fig. 54b), a gastric tissue (Fig. 54c), and a brain tissue (Fig. 54d) of mice at 0 hr (blank group)/3 hrs/6 hrs/9 hrs/12 hrs/24 hrs respectively.

### DETAILED DESCRIPTION OF THE INVENTION

The present invention is at least partially based on the unexpected discovery obtained by the inventors of the present invention after extensive experiments, that is, some lipid components exist in some traditional Chinese medicines (for example, *Rhodiola crenulata, Taraxacum mongolicum, Andrographis paniculata,* and *Lonicera japonica* etc.). These lipids derived from traditional Chinese medicines can promote the uptake/entry of nucleic acids such as small RNAs into cells and/or target sites in a subject in need thereof. To achieve the objects of the present invention, these lipids may be synthetic.

The present invention will be described in detail below with reference to some embodiments of the present invention exemplarily showing the structures and formulas.

Although the present invention will be described in conjunction with the listed embodiments, it should be understood that the present invention is not intended to be limited thereto. On the contrary, all alternatives, modifications and equivalents falling within the scope as defined by the claims are intended to be contemplated in the present invention. Those skilled in the art will appreciate that other methods and materials similar or equivalent to the methods and materials described herein can be used to implement the present invention, and the present invention is not limited to the methods and materials described in any way. When the cited documents and similar materials are different from or conflict with the description of the present invention (including terms for definition, use of terms, and technologies, etc.), the description in the present invention shall prevail.

It should also be understood that certain features described in different embodiments may also be provided in combination in a single embodiment. Conversely, the various features described in a single embodiment can also be provided separately or in any suitable sub-combination.

It should also be understood that the numerical points recited herein are intended to include each numerical point itself, and numerical ranges between any two recited points (as if those numerical ranges had been individually listed).

### Definitions

As used herein, unless otherwise indicated, the following definitions are used. For the purpose of the present invention, chemical elements are identified in accordance with the Periodic Table of the Elements (CAS version), Handbook of Chemistry and Physics (75th Edition). In addition, the general principles of organic chemistry and specific functional moieties and reactivity are described in "Organic Chemistry", Thomas Sorrell, University Science Books, Sausalito: 1999 and "March's Advanced Organic Chemistry", 5th Edition, Smith, M.B. and March, J., John Wiley & Sons, New York: 2001, which are incorporated herein by reference in their entireties.

Linking substituents are described herein. When a structure clearly requires a linking group, it should be understood that the Markush variable given for the group is a linking group. For example, if a structure requires a linking group, and the Markush group definition for this variable gives "alkyl", it should be understood that the "alkyl" means an alkylene linking group.

As used herein, the term "substituted", regardless of whether it is preceded by the term "optionally", means that one or more hydrogen atoms of a specified group is/are replaced by a suitable substituent. Unless otherwise indicated, an " substituted" group may have a suitable substituent at each substitutable position of the group, and when more than one position in any given structure is substituted by more than one substituent selected from particular groups, the substituent may be the same or different at each position. The combinations of substituents envisioned by the present invention are preferably those that result in the formation of stable or chemically feasible compounds. The term "stable" as used herein refers to a compound that remains substantially unchanged when it is amenable to conditions that allow its production, detection, and (in certain embodiments) recovery and purification, and when it is used for one or more of the purposes disclosed herein. Unless specifically indicated as "unsubstituted", the chemical moiety described herein should be understood to include a substituent. For example, when referring to "aryl", it includes substituted aryl and unsubstituted aryl.

When the bond to a substituent is shown to cross the bond linking two atoms in the ring, the substituent may be bonded to any atom in the ring. When a substituent is listed, without specifying the atom via which the substituent is bonded to the remaining moiety of the compound of a given formula, the substituent may be bonded via any atom in the formula. Combinations of substituents and/or variables are allowed, provided that the combination results in a stable compound.

The term "Cᵢ₋ⱼ" as used herein represents the range defining the number of carbon atoms, wherein i and j are integers and j is greater than i, and the range of the number of carbon atoms includes the endpoints (i.e., i and j) and each integer between the endpoints. For example, C₁₋₆ represents the range of 1 to 6 carbon atoms, including 1 carbon atom, 2 carbon atoms, 3 carbon atoms, 4 carbon atoms, 5 carbon atoms and 6 carbon atoms. In some embodiments, the term "C₁₋₁₂" represents 1 to 12, particularly 1 to 10, particularly 1 to 8, particularly 1 to 6, particularly 1 to 5, particularly 1 to 4, particularly 1 to 3, or particularly 1 to 2 carbon atoms.

As used herein, the term " alkyl", whether used as part of another term or used independently, refers to a saturated linear or branched hydrocarbon group. The term "Cᵢ₋ⱼ alkyl" refers to an alkyl having i to j carbon atoms. In some embodiments, the alkyl includes 1 to 20 carbon atoms. In some embodiments, the alkyl includes 5 to 20 carbon atoms. In some embodiments, the alkyl includes 1 to 20 carbon atoms, 1 to 19 carbon atoms, 1 to 18 carbon atoms, 1 to 17 carbon atoms, 1 to 16 carbon atoms, 1 to 15 carbon atoms, 1 to 14 carbon atoms, 1 to 13 carbon atoms, 1 to 12 carbon atoms, 1 to 11 carbon atoms, 1 to 10 carbon atoms, 1 to 9 carbon atoms, 1 to 8 carbon atoms, 1 to 7 carbon atoms, 1 to 6 carbon atoms, 1 to 5 carbon atoms, 1 to 4 carbon atoms, 1 to 3 carbon atoms, or 1 to 2 carbon atoms. Examples of alkyl include, but are not limited to, methyl, ethyl, 1-propyl (n-propyl), 2-propyl (isopropyl), 1-butyl (n-butyl), 2-methyl-1-propyl (isobutyl), 2-butyl (neobutyl), 2-methyl-2-propyl (tert-butyl), 1-pentyl (n-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, 1-heptyl, 1-octyl, 1-nonyl, 1-decyl and the like. Examples of "C₁₋₂₀ alkyl" include, but are not limited to, methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, and eicosyl.

As used herein, the term "alkenyl", whether used as part of another term or used independently, refers to a linear or branched hydrocarbon group having at least one carbon-carbon double bond, which may optionally independently be substituted by one or more substituents described herein, and includes groups having "cis" and "trans" orientations, or "E" and "Z" orientations. In some embodiments, the alkenyl includes 2 to 20 carbon atoms. In some embodiments, the alkenyl includes 5 to 20 carbon atoms. In some embodiments, the alkenyl includes 2 to 20 carbon atoms, 2 to 19 carbon atoms, 2 to 18 carbon atoms, 2 to 17 carbon atoms, 2 to 16 carbon atoms, 2 to 15 carbon atoms, 2 to 14 carbon atoms, 2 to 13 carbon atoms, 2 to 12 carbon atoms, 2 to 11 carbon atoms, 2 to 10 carbon atoms, 2 to 9 carbon atoms, 2 to 8 carbon atoms, 2 to 7 carbon atoms, 2 to 6 carbon atoms, 2 to 5 carbon atoms, 2 to 4 carbon atoms, or 2 to 3 carbon atoms. In some embodiments, the alkenyl includes more than 1 carbon-carbon double bond. It should be understood that in the case that the alkenyl includes more than 1 carbon-carbon double bond, the double bonds may be separated from each other or conjugated. In some embodiments, the alkenyl includes 5 carbon-carbon double bonds, 4 carbon-carbon double bonds, 3 carbon-carbon double bonds, 2 carbon-carbon double bonds, or 1 carbon-carbon double bond.

As used herein, the term "cycloalkyl", whether used as part of another term or used independently, refers to saturated monocyclic and polycyclic ring systems, where all ring atoms are carbon, and which contain at least 3 ring-forming carbon atoms. In some embodiments, the cycloalkyl includes 3 to 20 ring-forming carbon atoms, 3 to 19 ring-forming carbon atoms, 3 to 18 ring-forming carbon atoms, 3 to 17 ring-forming carbon atoms, 3 to 16 ring-forming carbon atoms, 3 to 15 ring-forming carbon atoms, 3 to 14 ring-forming carbon atoms, 3 to 13 ring-forming carbon atoms, 3 to 12 ring-forming carbon atoms, 3 to 11 ring-forming carbon atoms, 3 to 10 ring-forming carbon atoms, 3 to 9 ring-forming carbon atoms, 3 to 8 ring-forming carbon atoms, 3 to 7 ring-forming carbon atoms, 3 to 6 ring-forming carbon atoms, 3 to 5 ring-forming carbon atoms, 4 to 20 ring-forming carbon atoms, 4 to 19 ring-forming carbon atoms, 4 to 18 ring-forming carbon atoms, 4 to 17 ring-forming carbon atoms, 4 to 16 ring-forming-carbon atoms, 4 to 15 ring-forming carbon atoms, 4 to 14 ring-forming carbon atoms, 4 to 13 ring-forming carbon atoms, 4 to 12 ring-forming carbon atoms, 4 to 11 ring-forming carbon atoms, 4 to 10 ring-forming carbon atoms, 4 to 9 ring-forming carbon atoms, 4 to 8 ring-forming carbon atoms, 4 to 7 ring-forming carbon atoms, 4 to 6 ring-forming carbon atoms, or 4 to 5 ring-forming carbon atoms. The cycloalkyl may be optionally substituted at one or more positions on the ring with one or more substituents described herein. The cycloalkyl can be saturated, partially unsaturated or fully unsaturated. In some embodiments, the cycloalkyl may be a saturated cyclic alkyl. In some embodiments, the cycloalkyl may be an unsaturated cyclic alkyl containing at least one double bond or triple bond in the ring system.

As used herein, the term "cycloalkenyl", whether used as part of another term or used independently, refers to monocyclic and polycyclic ring systems containing 3-20 ring-forming carbon atoms and at least one carbon-carbon double bond, as long as the size of the cycloalkenyl ring allows, wherein all ring atoms are carbon. In some embodiments, the cycloalkenyl includes more than 1 carbon-carbon double bond. It should be understood that in the case that the alkenyl includes more than 1 carbon-carbon double bond, the double bonds may be separated from each other or conjugated. In some embodiments, the cycloalkenyl includes 5 carbon-carbon double bonds, 4 carbon-carbon double bonds, 3 carbon-carbon double bonds, 2 carbon-carbon double bonds, or 1 carbon-carbon double bond.

As used herein, the term "heteroatom" refers to nitrogen, oxygen, sulfur or phosphorus, including any oxidized form of nitrogen or sulfur, and any quaternized form of basic nitrogen.

As used herein, the term "heteroalkyl", whether used as part of another term or used independently, refers to an alkyl in which at least one carbon atom is replaced by a heteroatom selected from nitrogen, oxygen, sulfur or phosphorus, wherein the heteroatom may be located at the end or in the middle of the alkyl.

As used herein, the term "heteroalkenyl", whether used as part of another term or used independently, refers to an alkenyl in which at least one carbon atom is replaced by a heteroatom selected from nitrogen, oxygen, sulfur or phosphorus, wherein the heteroatom may be located at the end or in the middle of the alkenyl.

The term "hydroxyl" as used herein refers to -OH.

The term "pharmaceutically acceptable salt" as used herein refers to a salt or zwitterionic form of the compound described herein that is suitable for contact with human or animal tissues without excessive toxicity, irritation, allergic reactions or other problems, and has a reasonable benefit/risk ratio, within the scope of sound medical judgment.

The term "pharmaceutical composition" as used herein refers to a composition comprising the lipid composition of the present invention and a nucleic acid. The pharmaceutical composition optionally further comprises a pharmaceutically acceptable carrier.

The term "pharmaceutically acceptable" as used herein refers to a compound, material, composition and/or dosage form that is suitable for contact with human or animal tissues without excessive toxicity, irritation, allergic reactions or other problems, or complications, and has a reasonable benefit/risk ratio, within the scope of sound medical judgment. In some embodiments, the pharmaceutically acceptable compounds, materials, compositions and/or dosage forms are those approved by regulatory agencies (for example, the U.S. Food and Drug Administration, China National Medical Products Administration, or European Medicines Agency) or listed in recognized pharmacopoeias (e.g., U.S. Pharmacopoeia, Chinese Pharmacopoeia or European Pharmacopoeia) for use in animals (especially humans).

The term "pharmaceutically acceptable carrier" as used herein refers to a pharmaceutically acceptable material, composition or medium, for example, a liquid or solid filler, diluent excipient, solvent or packaging material, which is involved in carrying or delivering a compound provided herein from a position, fluid, tissue, organ (internal or external) or part of the body to another position, fluid, tissue, organ or part of the body. The pharmaceutically acceptable carrier may be a vehicle, diluent excipient or other materials that can be used in contact with animal tissues without excessive toxicity or adverse reactions. Exemplary pharmaceutically acceptable carriers include carbohydrates, starch, cellulose, malt, tragacanth, gelatin, Ringer's solution, alginic acid, isotonic saline, and buffers, etc. The pharmaceutically acceptable carriers that can be used in the present invention include those known in the art, such as those disclosed in "Remington Pharmaceutical Sciences" Mack Pub. Co., New Jersey (1991), which is incorporated herein by reference.

The term "delivery" as used herein includes local and systemic delivery. "Local delivery" refers to the direct delivery of a to-be-delivered therapeutic agent (for example, a nucleic acid) to a target site in an organism. For example, the reagent can be locally delivered by directly injecting to a target site (such as a lesion site, such as a tumor or inflammatory site) or a target organ (such as the heart, spleen, lung, or kidney). "Systemic delivery" is a mode of delivery in which a therapeutic agent (such as a nucleic acid) has a wide biological distribution in an organism, such that an effective amount of the therapeutic agent is exposed to most parts of the body. To obtain a wide range of biological distribution, such a life in blood is usually needed that these therapeutic agents cannot be degraded or cleared quickly before reaching the target site far away from the administration site. The lipid composition can be systemically delivered through any suitable route, including for example, oral administration, or administration by inhalation via the digestive tract, or by intravenous, subcutaneous, or intraperitoneal injection.

The term "lipid" as used herein refers to a class of organic compounds, including, but not limited to, esters of fatty acids, and characterized by being insoluble in water (for example, the solubility in water is less than about 0.01% by weight) but soluble in many organic solvents. The lipid may be, for example, a simple lipid (e.g., fat, oil, and wax), a compound lipid (e.g., phospholipids, and glycolipids), and a derived lipid (e.g., steroids).

The term "nucleic acid" as used herein refers to a polymer containing at least two deoxyribonucleotides or ribonucleotides in single- or double-stranded form. The nucleic acid may include naturally occurring ribonucleotides and deoxyribonucleotides, and may also include non-naturally occurring ribonucleotides and non-naturally occurring deoxyribonucleotides. Naturally occurring ribonucleotides include, for example, adenosine phosphate, guanosine phosphate, cytidine phosphate, uridine phosphate, pseudouridine phosphate, inosinate phosphate, and xanthosine phosphate. Naturally occurring deoxyribonucleotides include, for example, deoxyadenosine phosphate, deoxyguanosine phosphate, deoxycytidine phosphate, and deoxythymidine phosphate. Non-naturally occurring ribonucleotides and deoxyribonucleotides generally have modified nucleobases, modified ribose or deoxyribose, and/or modified phosphate linkages.

A "nucleotide" contains a sugar molecule (such as deoxyribose or ribose), bases, and phosphate groups. Nucleotides are linked together by phosphate groups, to form a polymer. "Bases" include purines and pyrimidines, and further the natural compounds adenine, thymine, guanine, cytosine, uridine, inosine, natural analogs thereof, and synthetic derivatives of purines and pyrimidines, including, but not limited to, those having modifications with amine, alcohol, thiol, carboxylate and alkyl halide, and so on. In the present invention, the nucleic acid may also contain nucleotide analogs or modified nucleotides that may be synthetic, naturally occurring, and non-naturally occurring, and has similar binding performance to a natural nucleic acid. Examples of nucleotide analogs or modified nucleotides include, but are not limited to, nucleotides having phosphorothioate, phosphoramidate, methylphosphate, or chiral methylphosphate, 2'-O-methylribonucleotides and peptide-nucleic acids (PNAs).

Examples of nucleic acids comprise DNA or RNA. DNA includes coding DNA and non-coding DNA, for example, but not limited to, antisense DNA, plasmid DNA, pre-concentrated DNA, PCR products, DNA vectors (P1, PAC, BAC, YAC, and artificial chromosomes), expression cassettes, chimeric sequences, chromosomal DNA, or derivatives and combinations thereof these groups. Examples of RNA include, but are not limited to, antisense RNA, siRNA, asymmetric interfering RNA (aiRNA), microRNA (miRNA), mRNA, tRNA, rRNA, viral RNA (vRNA), long non-coding RNA (IncRNA), Piwi-interacting RNA (piRNA), small nucleolar RNA (snoRNA), tRNA-derived small RNA (tsRNA) or a combination thereof.

Unless otherwise specified, particular nucleic acid sequences also inherently include conservatively modified variants thereof (e.g., degenerate codon substitution), alleles, orthologues, SNPs, complementary sequences and explicitly indicated sequences. Particularly, degenerate codon substitutions can be achieved by generating a sequence in which position(s) 3 in one or more selected (or all) codons is/are substituted by basic and/or deoxyinosine residues (Batzer et al., NucleicAcidRes. 19:5081(1991); Ohtsuka et al., J.Biol.Chem. 260:2605-2608(1985); Rossolini et al., Mol.Cell.Probes, 8:91-98(1994)).

The term "complementary" as used herein in the context of nucleotide pairing includes classical Watson-Crick base pairing, that is, G-C, A-T or A-U pairing. Classical Watson-Crick base pairing also cover situations where one or two nucleotides are modified (for example by ribose modifications or phosphate backbone modifications). "Complementary" sequences as used herein may also include non-Watson-Crick base pairs and/or base pairs formed from non-naturally occurring and modified nucleotides.

As used herein, the term "treat", "treating" or "treatment" means eliminating, reducing or improving a disease or condition and/or symptoms associated therewith. The treatment of a disease or condition does not exclusively require complete elimination of the condition or symptoms associated therewith. The term "treatment" as used herein may include "prophylactic treatment" that is to reduce the potential recurrence of a disease or condition, or reduce the potential relapse of a previously controlled disease or condition in a subject that is not suffering from a disease or condition, but at risk of or prone to recurrence of the disease or condition or at risk of or prone to relapse of the disease or condition. Within the meaning in the present invention, "treat", "treating" or "treatment" also includes recurrence prevention or periodic prevention, as well as the treatment of acute or chronic signs, symptoms and/or dysfunctions. The treatment can be symptom-directed, for example, suppressing the symptoms. It works in a short period of time, in a medium period of time, or in a long period of time, for example in the case of maintenance therapy.

The term "subject" as used herein refers to any organism to which the lipid composition described herein can be administered for therapeutic purposes. In some embodiments, the subject is primates (such as human), dogs, rabbits, guinea pigs, pigs, rats and mice. In certain embodiments, the subject is primates. In other embodiments, the subject is human.

### Method and use of a lipid compound for delivering a nucleic acid.

The present invention provides a method and use of a lipid compound for delivering a nucleic acid. The present invention is based at least partly on the finding that certain lipid compounds have notable effects in nucleic acid delivery. The present invention provides a variety of lipid compounds, found to form a stable nucleic acid-lipid mixture with a nucleic acid, and able to deliver the nucleic acid into cells, especially cells in the body. The lipid compound provided herein can be taken orally to deliver a nucleic acid molecule into the body, and into a target organ in the body, thus achieving a good therapeutic effect. Unexpectedly, different lipid compounds have different effects in delivering the nucleic acids into target organs, and show different preferences for different target organs. Certain lipid compounds exhibit significant effect on nucleic acid delivery to more than one target organs, and can efficiently deliver a nucleic acid to multiple target organs, thus having a broad scope of uses in nucleic acid delivery. Certain lipid compounds exhibit significant nucleic acid delivery to a certain target organ, and can be used to deliver a nucleic acid that acts on this target organ.

In an aspect, the present invention provides use of a lipid composition in the manufacture of a reagent for delivering a nucleic acid, or a method of a lipid composition for delivering a nucleic acid, wherein the lipid composition comprises one or more compounds of Formula (I), or a salt, a hydrate or a solvate thereof: 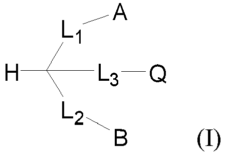 wherein
L₁ is absent, or is -CH₂-O-C(O)-, -CH₂-O- or -CR(OH)-;
L₂ is absent, or is -O-C(O)- or -NH-C(O)-;
L₃ is a linear or branched C₁₋₂₀ alkyl, a linear or branched C₁₋₂₀ alkenyl, a linear or branched C₁₋₂₀ heteroalkyl, a linear or branched C₁₋₂₀ heteroalkenyl or 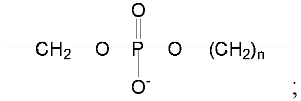
A is a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl;
B is -OH, a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl;
Q is -H, -COOH, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, -N(R)₃⁺ or 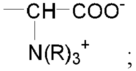
R is each independently H or a linear or branched C₁₋₂₀ alkyl; and
n is 0, 1 or 2.

In certain embodiments, L₁ is -CH₂-O-C(O)-, -CH₂-O- or -CR(OH)-; L₂ is absent, or is -O-C(O)- or -NH-C(O)-; L₃ is 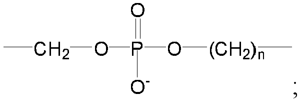 and A, B, Q, R and n are as defined above.

In certain embodiments, L₁ is absent, or is -CH₂-O-C(O)- or -CH₂-O-; L₂ is - O-C(O)-; L₃ is a linear or branched C₁₋₂₀ alkyl or 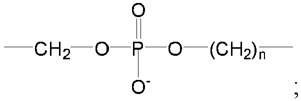 and A, B, Q, R and n are as defined above.

In certain embodiments, L₁ is -CH₂-O-C(O)-; L₂ is absent or is -O-C(O)-; L₃ is 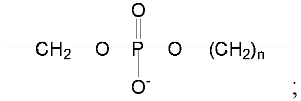 and A, B, Q, R and n are as defined above.

In certain embodiments, L₁ is - CH ₂-O-; L₂ is absent or is -O-C(O)-; L₃ is 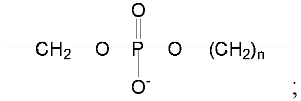 and A, B, Q, R and n are as defined above.

In certain embodiments, L₁ is -CH₂-O-C(O)- or -CH₂-O-; L₂ is absent; L₃ is 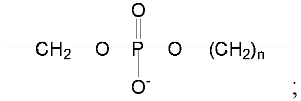 and A, B, Q, R and n are as defined above.

In certain embodiments, L₁ is absent; L₂ is -O-C(O)-; L₃ is a linear or branched C₁₋₂₀ alkyl; A is a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl; B is -OH, a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl; Q is -H, -COOH, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, - N(R)₃⁺ or 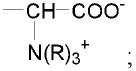 R is each independently H or a linear or branched C₁₋₂₀ alkyl; and n is 0, 1 or 2.

In certain embodiments, L₁ is -CH₂-O-C(O)-; L₂ is absent; L₃ is 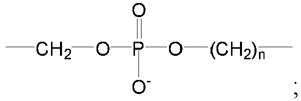 A is a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl; B is -OH, a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl; Q is -H, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, - N(R)₃⁺ or 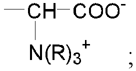 R is each independently H or a linear or branched C₁₋₂₀ alkyl; and n is 0, 1 or 2.

In certain embodiments, L₁ is -CH₂-O-; L₂ is absent; L₃ is 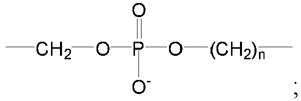 A is a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl; B is -OH, a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl; Q is -H, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, - N(R)₃⁺ or 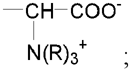 R is each independently H or a linear or branched C₁₋₂₀ alkyl; and n is 0, 1 or 2.

In certain embodiments, L₁ is -CH₂-O-; L₂ is -O-C(O)-; L₃ is 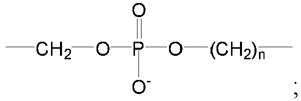 A is a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl; B is -OH, a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl; Q is -H, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, - N(R)₃⁺ or 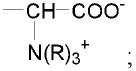 R is each independently H or a linear or branched C₁₋₂₀ alkyl; and n is 0, 1 or 2.

In certain embodiments, L₁ is -CH₂-O-C(O)-; L₂ is -O-C(O)-; L₃ is 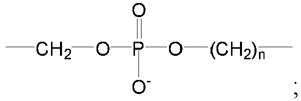 A is a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl; B is a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl; Q is -H, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, -N(R)₃⁺ or

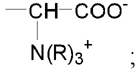

R is each independently H or a linear or branched C₁₋₂₀ alkyl; and n is 0, 1 or 2.

In certain embodiments, L₁ is -CH₂-O-C(O)-; L₂ is -O-C(O)-; L₃ is 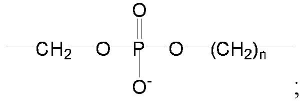 A is a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl; B is -OH, a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl; Q is -H, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, - N(R)₃⁺ or 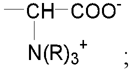 R is each independently H or a linear or branched C₁₋₂₀ alkyl; and n is 0, 1 or 2.

In certain embodiments, L₁ is -CR(OH)-; L₂ is -NH-C(O)-; L₃ is 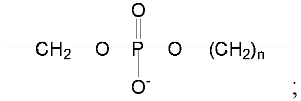 A is a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl; B is -OH, a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl; Q is -H, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, - N(R)₃⁺ or 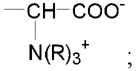 R is each independently H or a linear or branched C₁₋₂₀ alkyl; and n is 0, 1 or 2.

In certain embodiments, the compound has the following Formula (II): 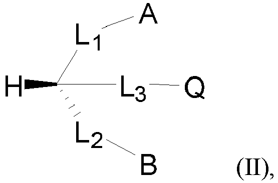 wherein L₁, L₂, L₃, A, B and Q are as defined above.

In certain embodiments, A is a linear or branched C₁₋₂₀ alkyl, or a linear or branched C₁₋₂₀ alkenyl. In certain embodiments, A is a linear or branched C₅₋₂₀ alkyl, or a linear or branched C₅₋₂₀ alkenyl. In certain embodiments, A is a linear or branched C₅₋₂₀ alkyl. In certain embodiments, A is a linear C₅₋₂₀ alkenyl having 1 to 5 double bonds.

In certain embodiments, B is -OH, a linear or branched C₁₋₂₀ alkyl, or a linear or branched C₁₋₂₀ alkenyl. In certain embodiments, B is -OH, a linear or branched C₅₋₂₀ alkyl, or a linear or branched C₅₋₂₀ alkenyl. In certain embodiments, B is -OH. In certain embodiments, B is a linear or branched C₅₋₂₀ alkyl.

In certain embodiments, B is a linear C₅₋₂₀ alkyl. In certain embodiments, B is a linear or branched C₅₋₂₀ alkenyl. In certain embodiments, B is a linear C₅₋₂₀ alkenyl having 1 to 5 double bonds.

In certain embodiments, the double bond is in a Z configuration. In certain embodiments, the double bond in the linear C₅₋₂₀ alkenyl is located at a position selected from: position C1-C2, position C2-C3, position C3-C4, position C4-C5, position C5-C6, position C6-C7, position C7-C8, position C8-C9, position C9-C10, position C10-C11, position C12-C13, position C13-C14 or a combination thereof.

In certain embodiments, Q is -H, -COOH, a linear or branched C₁₋₂₀ alkyl substituted by hydroxyl, a C₃₋₂₀ cycloalkyl substituted by hydroxyl, -N(R)₃⁺ or 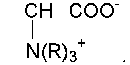 In certain embodiments, Q is a linear or branched C₁₋₂₀ alkyl substituted by hydroxyl. In certain embodiments, Q is a linear or branched C₁₋₁₀ alkyl substituted by hydroxyl. In certain embodiments, Q is a linear C₁₋₅ alkyl substituted by hydroxyl. In certain embodiments, Q is - CH(OH)-CH₂OH. In certain embodiments, Q is a C₃₋₂₀ cycloalkyl substituted by hydroxyl. In certain embodiments, Q is a C₃₋₁₀ cycloalkyl substituted by hydroxyl.

In certain embodiments, Q is

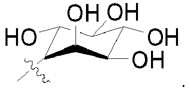

In certain embodiments, Q is -N(R)₃⁺, and R is each independently H or a linear C₁₋₁₀ alkyl.

In certain embodiments, Q is -N(CH₃)₃⁺.

In certain embodiments, Q is 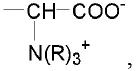 and R is each independently H or a linear C₁₋₁₀ alkyl.

In certain embodiments, Q is 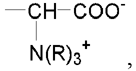 and R is H.

In certain embodiments, the compound has the following Formula (II): 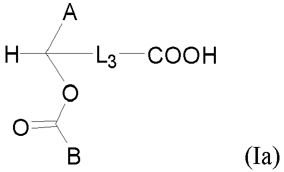 wherein L₃ is a linear or branched C₁₋₂₀ alkyl; A is a linear or branched C₁₋₂₀ alkyl; and B is a linear or branched C₁₋₂₀ alkyl.

In certain embodiments, the compound has the following Formula (Ib): 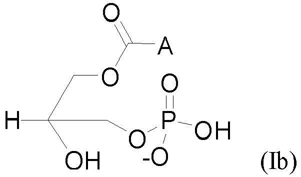 wherein A is a linear or branched C₁₋₂₀ alkyl.

In certain embodiments, the compound has the following Formula (II): 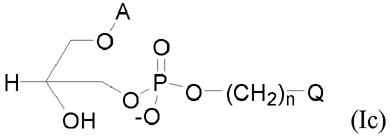 wherein A is a linear or branched C₁₋₂₀ alkyl; Q is -N(R)₃⁺; and R is each independently a linear or branched C₁₋₂₀ alkyl.

In certain embodiments, the compound has the following Formula (Id): 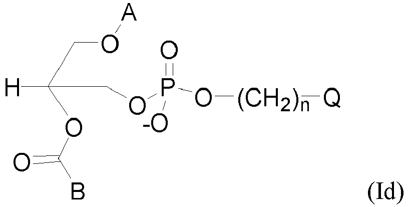 wherein A is a linear or branched C₁₋₂₀ alkyl; B is a linear or branched C₁₋₂₀ alkyl, or a linear or branched C₁₋₂₀ alkenyl; Q is -N(R)₃⁺; and R is each independently a linear or branched C₁₋₂₀ alkyl.

In certain embodiments, the compound has the following Formula (Ie): 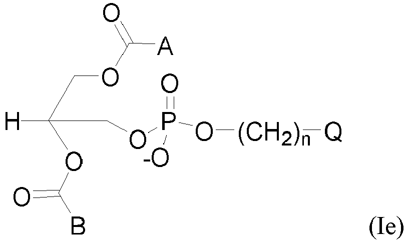 wherein A is a linear or branched C₁₋₂₀ alkyl, or a linear or branched C₁₋₂₀ alkenyl; B is a linear or branched C₁₋₂₀ alkenyl; Q is a linear or branched C₁₋₂₀ alkenyl substituted by hydroxyl, a C₃₋₂₀ cycloalkyl substituted by hydroxyl, or 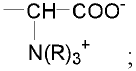 and R is each independently a linear or branched C₁₋₂₀ alkyl.

In certain embodiments, the compound has the following Formula (II): 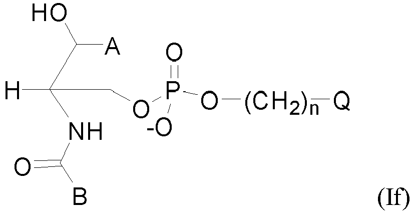 wherein A is a linear or branched C₁₋₂₀ alkenyl; B is a linear or branched C₁₋₂₀ alkyl, or a linear or branched C₁₋₂₀ alkenyl; Q is -N(R)₃⁺; and R is each independently a linear or branched C₁₋₂₀ alkyl.

In certain embodiments, the lipid composition comprises one or more compounds of Formula (I), or a salt, a hydrate or a solvate thereof. The compound is selected from the group consisting of lipids 106, 96, 93, 94, 84, 85, 108, 81, 88, 89, 109, 110, 103, 104 and 105. Data about the chemical names and chemical structures of the lipids is shown in Table 1.

In another aspect, the present invention further provides use of a lipid composition in the manufacture of a reagent for delivering a nucleic acid, or a method of a lipid composition for delivering a nucleic acid, wherein the lipid composition comprises one or more compounds, or a salt, a hydrate or a solvate thereof. The compound is selected from the group consisting of lipids 72, 73, 74, 75, 76, 77, 78, 79, 80, 82, 83, 86, 87, 90, 91, 92, 95, 97, 98, 99, 100, 101, 102, 107, 111, 112, 113, 114, 115, 116, 117, 118 and 119. Data about the chemical names and chemical structures of the lipids is shown in Table 1.

**Table 1. Abbreviations, Structures and chemical names of lipids 72-119**

| **Lipid compound No.** | **Abbreviation** | **Structure and Chemical name** |
|---|---|---|
| 106 | 9-PAHSA | 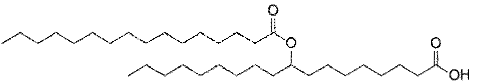 |
| | | 9-(palmitoyloxy)octadecanoic acid |
| 96 | 16:0 Lyso PA | 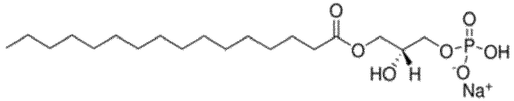 |
| | | 1-palmitoyl-2-hydroxy-sn-glycero-3-phosphate (sodium salt) |
| 93 | C16 Lyso PAF | 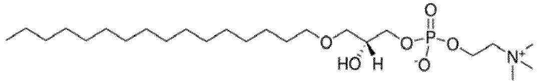 |
| | | 1-O-hexadecyl-2-hydroxy-sn-glycero-3-phosphocholine |
| 94 | C18 Lyso PAF | 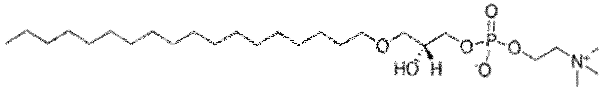 |
| | | 1-O-octadecyl-2-hydroxy-sn-glycero-3-phosphocholine |
| 84 | PAF (16:0p) | 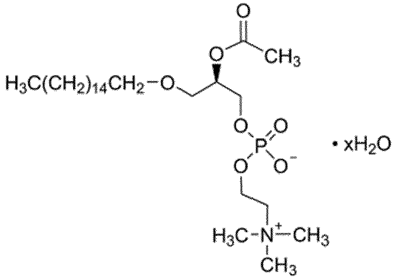 |
| | | β-acetyl-γ-O-hexadecyl-L-α-phosphatidylcholine hydrate |
| 85 | PAF (16:0e) | 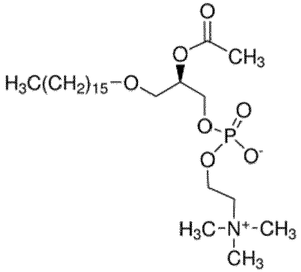 |
| | | β-acetyl-γ-O-alkyl-L-α-phosphatidylcholine |
| 108 | C16:0-20:4 PC | 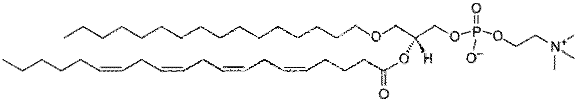 |
| | | 1-O-hexadecyl-2-arachidonoyl-sn-glycero-3-phosphocholine |
| 81 | PG (18:1/18:1) | 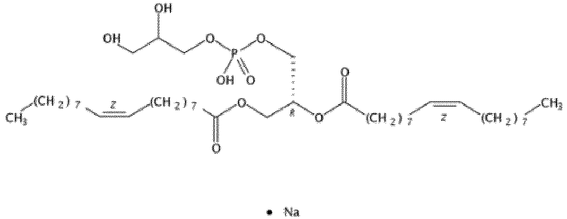 |
| | | 1,2-dioleoyl-sn-glycero-3-phosphatidylglycerol sodium salt |
| 88 | PG (16:0/18:1) | 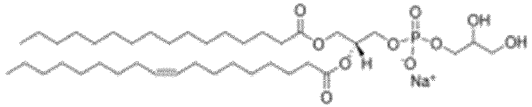 |
| | | 1-palmitoyl-2-oleoyl-sn-glycero-3 -phospho-(1'-rac-glycerol) (sodium salt) |
| 89 | PI (16:0/18:1) | 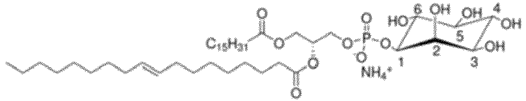 |
| | | 1-palmitoyl-2-oleoyl-sn-glycero-3 -phosphoinositol (ammonium salt), powder |
| 109 | 16:0 PI | 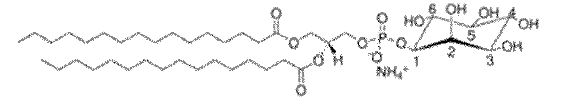 |
| | | 1,2-dipalmitoyl-sn-glycero-3-phospho-(1'-myo-inositol) (ammonium salt) |
| 110 | 16:0-20:4 PS | 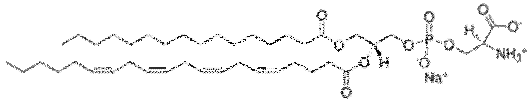 |
| | | 1-palmitoyl-2-arachidonoyl-sn-glycero-3-phospho-L-serine (sodium salt) |
| 103 | 16:1 SM (d18:1/16:1(9Z)) | 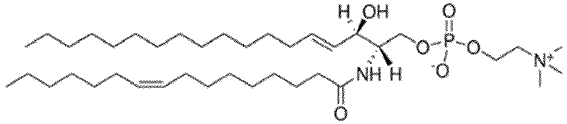 |
| | | N-palmitoleoyl-D-erythro-sphingosylphosphocholine |
| 104 | 18:0 SM (d18:1/18:0) | 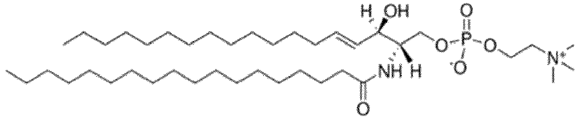 |
| | | N-stearoyl-D-erythro-sphingosylphosphocholine |
| 105 | 16:0 SM (d18:1/16:0) | 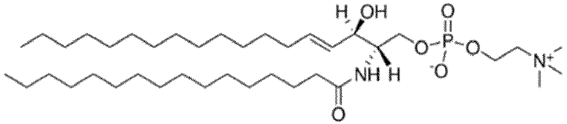 |
| | | N-palmi toyl-D-erythro-sphingosylphosphocholine |
| 72 | PE (18:1/18:1) | 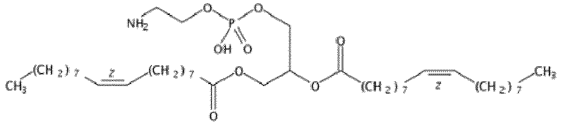 |
| | | 1 ,2-dioleoyl-sn -glycero-3 -phosphatidylethanolamine |
| 73 | TG (18:3/18:3/18:3) | 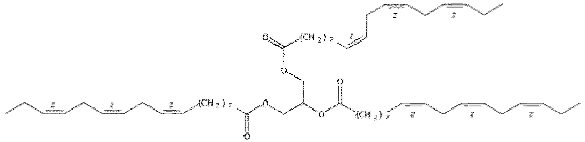 |
| | | Trilinolenin |
| 74 | TG (16:1/16:1/18:1) | 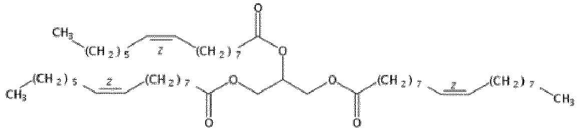 |
| | | 1,2-palmitolein-3 -olein |
| 75 | TG (16:0/16:1/16:1) | 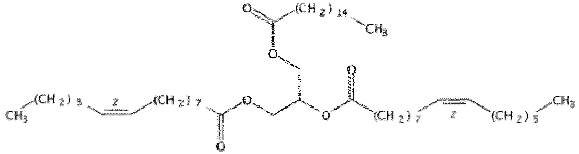 |
| | | 1,2-palmitolein-3-palmitin |
| 76 | TG (16:0/14:0/16:0) | 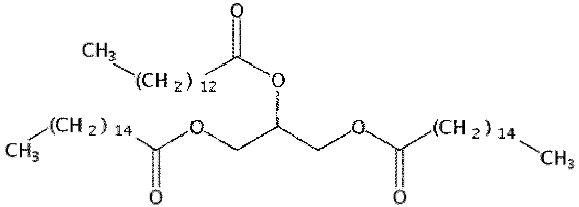 |
| | | 1,3 -palmitin-2-myristin |
| 77 | TG (18:0/18:0/18:1) | 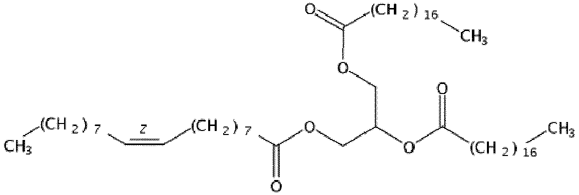 |
| | | 1 ,2-stearin-3 -olein |
| 78 | TG (20:0/18:1/18:1) | 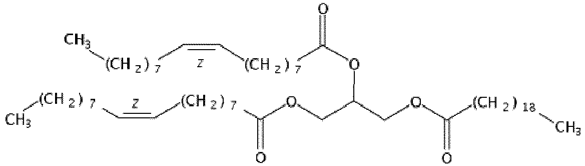 |
| | | 1,2-olein-3 -arachidin |
| 79 | TG (18:1/18:1/22:0) | 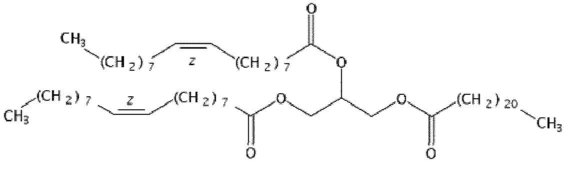 |
| | | 1,2-olein-3-behenin |
| 80 | TG (16:0/14:0/14:0) | 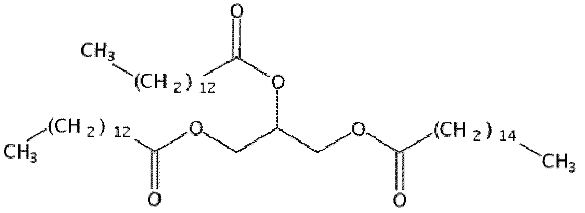 |
| | | 1,2-myristin-3 -palmitin |
| 82 | Co(Q10) | 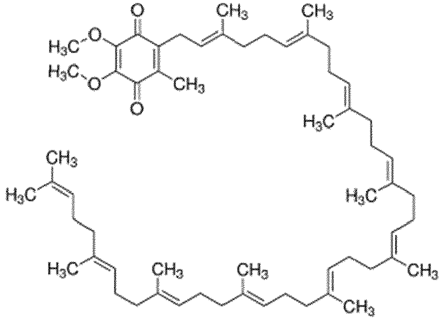 |
| | | Q-10, Ubiquinone 50, Ubiquinone 10 |
| 83 | LPE(16:0) | 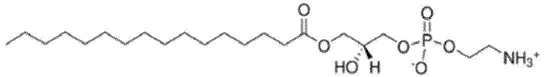 |
| | | 1 -palmitoyl-2-hydroxy-sn-glycero-3 -phosphoethanolamine |
| 86 | PC(16:0e/18:1) | 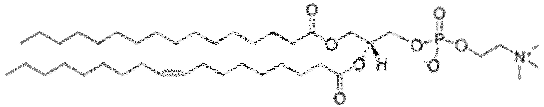 |
| | | 1-palmitoyl-2-oleoyl-sn-glycero-3-phosphocholine, powder |
| 87 | PC (18:0/18:1) | 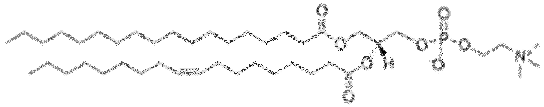 |
| | | 1 -stearoyl-2-oleoyl-sn-glycero-3 -phosphocholine |
| 90 | C24 ceramide (d18:1/24:0) | 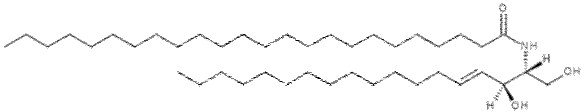 |
| | | N-lignoceroyl-D-erythro-sphingosine |
| 91 | C24:1 ceramide (d18:1/24:1(15Z)) | 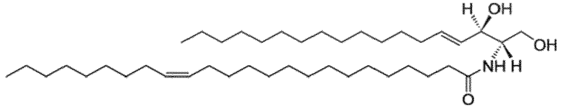 |
| | | N-nervonoyl-D-erythro-sphingo sine |
| 92 | 20:0 Lyso PC | 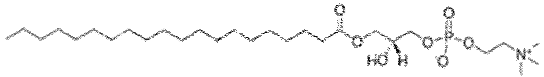 |
| | | 1-arachidoyl-2-hydroxy-sn-glycero-3 -phosphocholine |
| 95 | 18:1 Lyso PC | 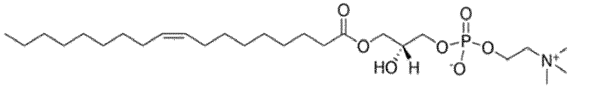 |
| | | 1-oleoyl-2-hydroxy-sn-glycero-3 -phosphocholine |
| 97 | Sphingosine (d22:1) | 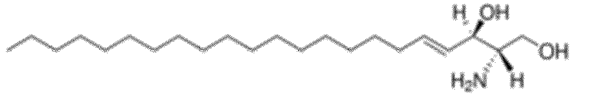 |
| | | D-erythro-sphingosine (C22 group) |
| 98 | Sphingosine (d18:1) | 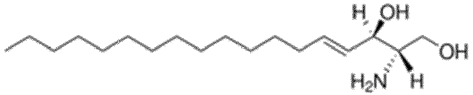 |
| | | D-erythro-sphingosine |
| 99 | Sphingosine (d14:1) | 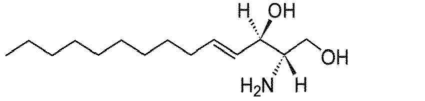 |
| | | D-erythro-sphingosine (C14 group) |
| 100 | Sphingosine (d16:1) | 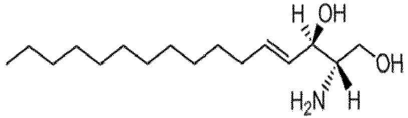 |
| | | D-erythro-sphingosine (C16 group) |
| 101 | Sphingosine (d20:0) | 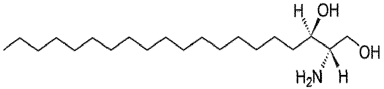 |
| | | D-erythro-sphingosine (C20 group) |
| 102 | Sphingosine (d18:0) | 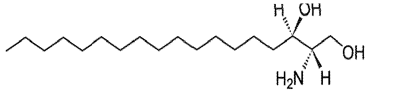 |
| | | D-erythro-sphingosine |
| 107 | 18:2 (Cis) PC (DLPC) | 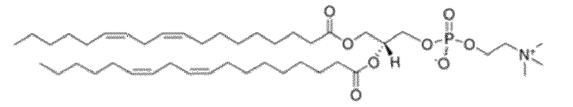 |
| | | 1 ,2-dilinoleoyl-sn -glycero-3 -phosphocholine |
| 111 | DG (18:0/16:0) | 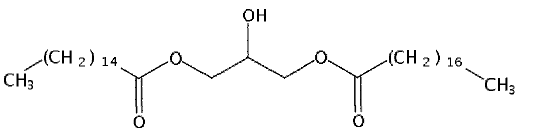 |
| | | 1 -palmitin-3 -stearin |
| 112 | TG (20:0/20:0/20:0) | 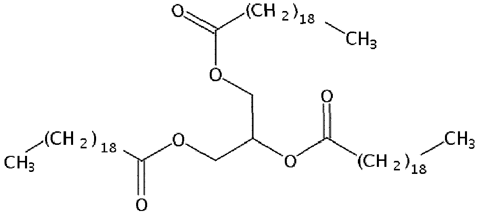 |
| | | Trieicosanoin |
| 113 | LPC(16:0) | 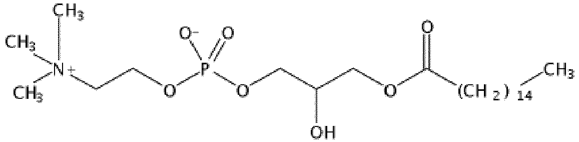 |
| | | 1 -palmitoyl-2-hydroxy-sn-glycerol-3 -phosphatidylcholine |
| 114 | LPC(18:0) | 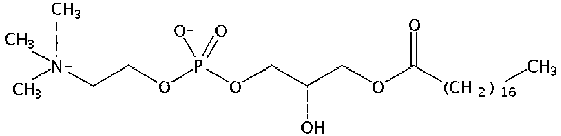 |
| | | 1-octadecanoyl-2-hydroxy-sn-glycero-3-phosphocholine |
| 115 | LPE(1 7: 1) | 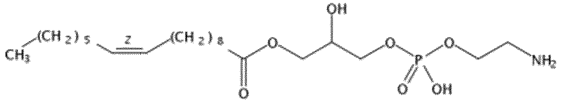 |
| | | 1 -heptadecenoyl-sn-glycero-3 -phosphoethanolamine |
| 116 | LPE(18: 1) | 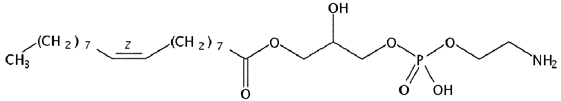 |
| | | 1 -(9Z-octadecenoyl)-sn-glycero-3 -phosphoethanolamine |
| 117 | PC (18:1/18:1) | 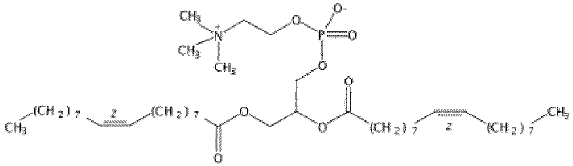 |
| | | 1,2-bis-(9Z-octadecenoyl)-sn-glycero-3-phosphocholine |
| 118 | PC (17:0/17:1) | 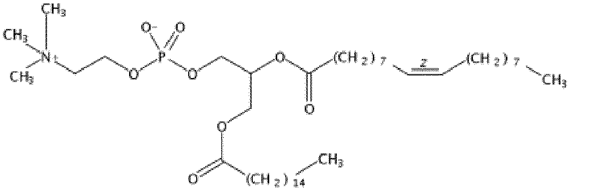 |
| | | 1-palmitoyl-2-oleoyl-sn-glycero-3 -phosphatidylcholine |
| 119 | PC (16:0/16:0) | 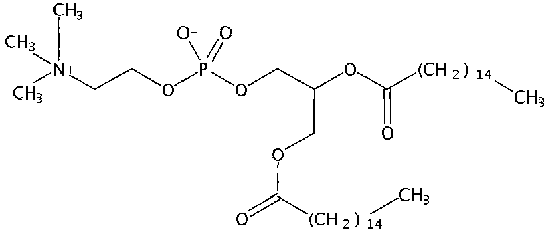 |
| | | 1 ,2-dihexadecanoyl-sn -glycero-3 -phosphocholine |

In certain embodiments, the compounds are synthetic. According to the chemical structures of the compounds, those skilled in the art can synthesize the above compounds by means of chemical synthesis, or some of the compounds described hereincan also be obtained commercially.

In certain embodiments, the compounds are derived from a traditional Chinese medicine extract. "Traditional Chinese medicine extract" as used herein refers to an extract obtained from a traditional Chinese medicine material or medicinal plant by suitable extraction methods. In certain embodiments, the traditional Chinese medicine material or medicinal plant is selected from *Rhodiola crenulata, Taraxacum mongolicum, Lonicera japonica,* and *Andrographis paniculata,* or decoction pieces thereof. The traditional Chinese medicine extract can be obtained through any suitable extraction methods. For example, decoction pieces of *Rhodiola crenulata, Taraxacum mongolicum, Lonicera japonica* or *Andrographis paniculata* are soaked in water, and heated intensely and slowly sequentially; the heated traditional Chinese medicine soup is concentrated; and then chloroform-methanol, chloroform, and water are added sequentially followed by mixing fully, and the chloroform layer is collected and extracted to obtain the traditional Chinese medicine extract. In certain embodiments, individual compounds can be further isolated or purified from the traditional Chinese medicine extract.

In certain embodiments, the traditional Chinese medicine extract is obtained by extracting fat-soluble components by Bligh&Dyer method (Bligh E.G. and Dyer, W. J., A rapid method for total lipid extraction and purification, Can. J. Biochem. Physiol., 1959, 37: 911-917), or obtained by boiling traditional Chinese medicines.

In certain embodiments, the traditional Chinese medicine extract is obtained by boiling and extracting the traditional Chinese medicine selected from the group consisting of decoction pieces of *Rhodiola crenulata, Taraxacum mongolicum, Andrographis paniculata* and *Lonicera japonica.*

In certain embodiments, the traditional Chinese medicine extract is obtained by soaking a traditional Chinese medicine in water, heating intensely and slowly sequentially; concentrating the heated traditional Chinese medicine soup; and sequentially adding chloroform-methanol, chloroform, and water followed by stirring, collecting and extracting chloroform layer.

### Lipid composition

In certain embodiments, the lipid composition provided herein comprises one or more compounds provided herein, or a salt, a hydrate or a solvate thereof. The lipid composition can be a compound provided herein, a salt, a hydrate or a solvate thereof, or a mixture of two or more compounds provided herein.

In certain embodiments, the lipid composition may also comprise one or more other lipid compounds than the compounds provided herein. The other lipid compounds may be, for example, neutral lipids, charged lipids, steroids and polymer conjugated lipids. "Neutral lipid" refers to a lipid compound that exists in an uncharged or neutral zwitterionic form at a selected pH (e.g., physiological pH). "Charged lipid" refers to a lipid compound in a positively or negatively charged form that is not affected by pH values within a useful physiological range (e.g. about pH 3 to 9).

In certain embodiments, the lipid composition may further comprise one or more solvents, which can be mixed with the compound provided herein or a salt, a hydrate or a solvate thereof to form a homogeneous mixture.

The solvent contained in the lipid composition may comprise an organic solvent or a solvent mixture, for example, chloroform, dichloromethane, diethyl ether, cyclohexane, cyclopentane, benzene, toluene, methanol or other aliphatic alcohols, for example, ethanol, propanol, isopropanol, butanol, t-butanol, iso-butanol, pentanol and hexanol. These solvents can be used alone, in mixture, and/or optionally together with a suitable buffer as a solvent in the lipid composition. The choice of solvent often takes the polarity of the solvent, the difficulty in removing the solvent at a later stage in the formation of lipid-nucleic acid mixture, and/or pharmaceutically acceptable properties into consideration. In certain embodiments, the solvent is non-toxic, or pharmaceutically acceptable. Exemplary pharmaceutically acceptable solvents comprise lower alcohols (having 1-6 carbon atoms), for example, methanol, ethanol, n-propanol, iso-propanol, and n-butanol. In certain embodiments, an appropriate amount of solvent can be used, so that the nucleic acid and lipid can form a clear single-phase mixture.

The lipid compositions provided herein can be used for delivering a nucleic acid, or preparing a reagent for nucleic acid delivery. In certain embodiments, the nucleic acid comprises DNA or RNA. In certain embodiments, the nucleic acid may comprise, for example, coding DNA, non-coding DNA, antisense nucleic acid, messenger RNA (mRNA), long non-coding RNA (IncRNA), small RNA (e.g. microRNA (miRNA), small interfering RNA (siRNA), Piwi-interacting RNA (piRNA), small nucleolar RNA (snoRNA), tRNA-derived small RNA (tsRNA)), and others.

In certain embodiments, the nucleic acid is single-stranded or double-stranded. Single-stranded nucleic acid comprises, for example, miRNA, mRNA, antisense DNA, antisense RNA, and others. Double-stranded nucleic acid comprises, for example, siRNA, double-stranded DNA, double-stranded RNA, and others.

In certain embodiments, the nucleic acid has a stem-loop structure. The stem-loop structure refers to a structure wherein reverse complementary sequences, existing in two regions of a single-stranded nucleic acid, form a double strand by base pairing, and the non-complementary region between the two reverse complementary regions protrudes to form a loop. The stem-loop structure is also known as the hairpin structure.

In certain embodiments, the nucleic acid comprises small nucleic acids. A small nucleic acid refers to a nucleic acid that is short in length (e.g., less than 200 nucleotides). The small nucleic acid can be non-coding, such as small RNA (e.g. miRNA, siRNA, piRNA, snoRNA, and tsRNA), and so on; and can be single-stranded, or double-stranded. In certain embodiments, the nucleic acid has a length of 14-32 bp, 16-28 bp or 18-24 bp.

In certain embodiments, the nucleic acid is a nucleic acid medicament. Nucleic acid medicaments can be of various types, for example, antisense nucleic acids, siRNA, CpG oligodeoxynucleotides, nucleic acid aptamers, mRNA or DNA encoding a target protein, or miRNA etc. Exemplary nucleic acid medicaments include, AEG35156, aganirsen, AP 12009, Apatorsen, ATL1103, AVT-02 UE, Bevasiranib Sodium, BMN 044, BMN 053, CpG 7909, Custirsen, Drisapersen, Eteplirsen, Fomivirsen, Pegaptanib, Mipomersen, Eteplirsen, Defibrotide, Nusinersen, Patisiran, Tegsedi and Fovista.

In certain embodiments, the nucleic acid is used for treating disease.

In certain embodiments, the nucleic acid is used for treating cancer, inflammation, fibrotic disease, autoimmune disease or autoinflammatory disease, bacterial infection, behavioral and psychiatric disorder, hematological disease, chromosomal disorder, congenital and genetic disease, connective tissue disease, digestive disease, ear, nose, and throat disease, endocrine disease, environmental illness, eye disease, female reproductive system disease, fungal infection, heart disease, hereditary cancer syndromes, immune system disorder, kidney and urinary tract disorder, pulmonary disease, male reproductive system disease, metabolic disorder, mouth disease, musculoskeletal disorder, myelodysplastic syndrome, newborn screening, nutritional disease, parasitic disease, rare cancer, rare disease, and skin disease and viral infection.

In certain embodiments, the nucleic acid is used for treating hepatocellular carcinoma, corneal neovascularization, recurrent or refractory anaplastic astrocytoma (WHO grade III) or secondary glioblastoma (WHO grade IV), advanced squamous cell lung cancer, acromegaly, psoriasis, Duchenne muscular dystrophy, advanced non-small cell lung cancer, metastatic castration-resistant prostate cancer, cytomegalovirus retinitis, HIV infection, Hepatitis B, Hepatitis C, hyperlipoproteinemia, total knee replacement, Diabetes mellitus type II, familial amyloid polyneuropathy (FAP), wet macular degeneration (e.g., neovascular age-related macular degeneration, subfoveal neovascular age-related macular degeneration, and exudative age-related macular degeneration), hypercholesterolemia, Crohn's disease, extensive liver fibrosis, infantile spinal muscular atrophy, melanoma, neonatal coronary artery disease, mild allergic asthma, chronic lymphocytic leukemia, hypertriglyceridemia, hepatic veno-occlusive disease complicated with renal or pulmonary dysfunction after hematopoietic stem cell transplantation, and hereditary transthyretin amyloidosis.

In certain embodiments, the lipid composition can form a lipid-nucleic acid mixture with a nucleic acid. The nucleic acid is delivered by the lipid-nucleic acid mixture. The "lipid-nucleic acid mixture" in the present invention means a lipid-based mixture for nucleic acid delivery, for example, liposomes. Examples of liposomes include, for example, lipid particles or lipid vesicles that encapsulate a nucleic acid. The lipid-nucleic acid mixture is prepared by a suitable method, including, for example, direct mixing, a heating method, and a reverse evaporation method.

The direct mixing comprises the step of mixing a compound provided herein, or a salt, a hydrate or a solvate thereof, with a nucleic acid to be delivered. In certain embodiments, the mixing can be directly mixing of the lipid composition provided herein with the nucleic acid to be delivered (for example, mixing dry powders of the lipid composition and nucleic acid, and then adding a suitable solvent to form a lipid-nucleic acid mixture where the lipid encapsulates the nucleic acid). In certain embodiments, the mixing can be mixing of the lipid composition of the present invention dissolved in a suitable solvent with the nucleic acid, or mixing of the nucleic acid dissolved in a suitable solvent with the lipid composition of the present invention, or mixing of the lipid composition of the present invention dissolved in a suitable solvent with the nucleic acid dissolved in a suitable solvent. Examples of suitable solvents that can be used in the lipid compositions of the present invention are as described above in the specification of the present invention. Suitable solvents that can be used with the nucleic acid include water (DEPC-treated water, double distilled water), a buffer, physiological saline, or a glucose solution, etc. The mixing step can be carried out in any suitable steps, for example, the nucleic acid (or a solution thereof) can be added to the lipid composition (or a solution thereof), or the lipid composition (or a solution thereof) can be added to the nucleic acid (or a solution thereof). In certain embodiments, the mixing may also include, for example, a swirling, ultrasonication and other steps, to facilitate uniform mixing.

In certain embodiments, the direct mixing comprises adding a solution of the lipid composition of the present invention in ethanol to an appropriate volume of an aqueous buffer of the nucleic acid to be delivered, mixing uniformly by swirling or ultrasonicating followed by incubating to obtain the lipid-nucleic acid mixture. In certain embodiments, the direct mixing comprises combining a solution of the lipid composition of the present invention in ethanol with an appropriate volume of a solution of the nucleic acid to be delivered in ethanol, mixing uniformly by swirling or ultrasonicating, incubating to obtain the lipid-nucleic acid mixture ethanol, then removing ethanol, and re-suspending the obtained lipid-nucleic acid mixture in an aqueous buffer.

In certain embodiments, the nucleic acid and the lipid compound can be mixed at a certain ratio. The ratio depends on the nucleic acid to be delivered and its amount, and the lipid compound needed to achieve the effect of nucleic acid encapsulation. The ratio can be any ratio that meets the therapeutic needs, as long as a stable lipid-nucleic acid mixture can be formed, and a required amount of the nucleic acid can be provided. In certain embodiments, the ratio of the nucleic acid to the lipid compound may be, for example, 0.1 nmol : 100 µg to 10 nmol : 100 µg, 0.2 nmol : 100 µg to 10 nmol : 100 µg, 0.3 nmol : 100 µg to 10 nmol : 100 µg, 0.4 nmol : 100 µg to 10 nmol : 100 µg, 0.5 nmol : 100 µg to 10 nmol : 100 µg, 1 nmol : 100 µg to 10 nmol : 100 µg, 2 nmol : 100 µg to 10 nmol : 100 µg, 3 nmol : 100 µg to 10 nmol : 100 µg, 4 nmol : 100 µg to 10 nmol : 100 µg, 5 nmol : 100 µg to 10 nmol : 100 µg, 6 nmol : 100 µg to 10 nmol, 7 nmol : 100 µg to 10 nmol, 8 nmol : 100 µg to 10 nmol, or 9 nmol : 100 µg to 10 nmol : 100 µg. In certain embodiments, the ratio of the nucleic acid to the lipid compound may be 1 nmol : 100 µg, 2 nmol : 100 µg, 3 nmol : 100 µg, 4 nmol : 100 µg, 5 nmol : 100 µg, 6 nmol : 100 µg, 7 nmol : 100 µg, 8 nmol : 100 µg, 9 nmol : 100 µg or 10 nmol : 100 µg. In certain embodiments, the ratio of the nucleic acid to the lipid compound may be 10 nmol : 100 µg.

The direct mixing can be carried out at any suitable temperature, as long as a lipid-nucleic acid mixture that can deliver nucleic acid can be formed. In certain embodiments, the nucleic acid and the lipid compound can be mixed at an appropriate temperature, for example, but not limited to, 0°C to 100°C, 4°C to 100°C, 10°C to 100°C, 15°C to 100°C, 20°C to 100°C, 25°C to 100°C, 30°C to 100°C, 35°C to 100°C, 40°C to 100°C, 45°C to 100°C, 50°C to 100°C, 55°C to 100°C, 60°C to 100°C, 65°C to 100°C, 70°C to 100°C, 75°C to 100°C, 80°C to 100°C, 85°C to 100°C, 90°C to 100°C, or 95°C to 100°C. In some embodiments, the temperature is 0°C, 4°C, 10°C, 20°C, 30°C, 40°C, 50°C, 55°C, 60°C, 65°C, 70°C, 75°C, 80°C, 85°C, or 90°C. In some embodiments, the temperature is 90°C.

In certain embodiments, the lipid-nucleic acid mixture is prepared by a heating method. The heating method comprises the step of mixing a solution of the compound provided herein or a salt, a hydrate or a solvate thereof dissolved in a suitable solvent with an aqueous solution of the nucleic acid to be delivered to obtain a mixed solution, and heating the mixed solution at a suitable temperature. In certain embodiments, the step of heating the mixed solution is carried out at a temperature in a range selected from the group consisting of: 25°C to 100°C, 30°C to 100°C, 40°C to 100°C, 50°C to 100°C, 60°C to 100°C, 70°C to 100°C, and 80°C to 100°C, 90°C to 100°C or 95°C to 100°C. In certain embodiments, the step of heating the mixed solution is carried out at a temperature selected from the group consisting of: about 30°C, about 35°C, about 37°C, about 40°C, about 45°C, about 50°C, about 55°C, about 60°C, about 65°C, about 70°C, about 75°C, about 80°C, about 85°C, about 90°C, about 95°C and about 100°C. In some embodiments, the step of heating the mixed solution is carried out at 90°C.

In certain embodiments, the heating method comprises heating the mixed solution for an appropriate period of time. Those skilled in the art can choose a suitable heating time according to the properties of the lipid compound and nucleic acid used and the desired lipid-nucleic acid mixture. The heating time may be, for example, about 5 min to about 24 hrs, about 5 min to about 20 hrs, about 5 min to about 16 hrs, about 10 min to about 20 hrs, about 10 min to about 16 hrs, about 15 min to about 24 hrs, about 15 min to about 20 hrs, about 30 min to about 24 hrs, about 30 min to about 20 hrs, about 40 min to about 16 hrs, about 50 min to about 12 hrs, about 1 hr to about 8 hrs, or about 2 hrs to about 4 hrs. In certain embodiments, the heating time may be, for example, about 5 min, about 10 min, about 15 min, about 20 min, about 30 min, about 40 min, about 50 min, about 1 hrs, about 2 hrs, about 3 hrs, about 4 hrs, about 5 hrs, about 6 hrs, about 7 hrs, about 8 hrs, about 9 hrs, about 10 hrs, about 12 hrs, about 16 hrs, about 20 hrs or about 24 hrs. In some embodiments, the heating time is about 15 min.

In certain embodiments, the heating method further comprises a cooling step. the cooling can be carried out at an appropriate temperature after the step of heating the mixed solution, as long as the formed lipid-nucleic acid mixture is not destroyed. Exemplary cooling temperatures include, but are not limited to, 25°C to -80°C, 20°C to -80°C, 15°C to - 80°C, 10°C to -80°C, 4°C to -80°C, 0°C to -80°C, -10°C to -80°C, -20°C to -80°C, -30°C to - 80°C, -40°C to -80°C. In some embodiments, after the step of heating the mixed solution, the mixed solution is naturally cooled at room temperature, for example, 25°C or 20°C.

In certain embodiments, the lipid-nucleic acid mixture is prepared by reverse evaporation. The reverse evaporation comprises adding an aqueous solution of the nucleic acid to a solution of the lipid compound dissolved in a suitable solvent, and then removing the solvent by ultrasonication, evaporation, and others. In certain embodiments, the reverse evaporation method further comprises hydration after removing the solvent, to obtain the lipid-nucleic acid mixture. In some embodiments, the hydration comprises adding water or a suitable medium into the system. In some embodiments, the suitable medium is, for example, OPTI-MEM medium.

In certain embodiments, the step of removing the solvent is carried out at an appropriate temperature, for example, about 25°C to about 70°C, 30°C to about 70°C, about 30°C to about 65°C, about 40°C to about 65°C, about 40°C to about 60°C, or about 50°C to about 60°C. In certain embodiments, the step of removing the solvent is performed at about 25°C, 30°C about 35°C, about 37°C, about 40°C, about 45°C, about 50°C, about 55°C, about 60°C, about 65°C or about 70°C. In certain embodiments, the step of removing the solvent is performed at about 55°C.

In certain embodiments, the nucleic acid is delivered by oral administration, inhalation or injection. In certain embodiments, the nucleic acid is delivered by oral administration. In certain embodiments, the reagent is used to deliver the nucleic acid by oral administration. In certain embodiments, the nucleic acid delivery comprises in vivo gastrointestinal delivery.

In certain embodiments, the lipid-nucleic acid mixture, after oral administration to an individual, can deliver the nucleic acid to a target organ or a target tissue of interest in the individual.

In certain embodiments, the lipid-nucleic acid mixture, after oral administration to an individual, can deliver the nucleic acid to a heart tissue in the individual. In certain embodiments, the lipid-nucleic acid mixture comprises lipid 73 and/or lipid 80.

In certain embodiments, the lipid-nucleic acid mixture, after oral administration to an individual, can deliver the nucleic acid to a lung tissue in the individual. In certain embodiments, the lipid-nucleic acid mixture comprises one or more lipid compounds selected from the group consisting of lipid 72, lipid 76, lipid 92, lipid 94, lipid 95, lipid 97, lipid 98 and lipid 103.

In certain embodiments, the lipid-nucleic acid mixture, after oral administration to an individual, can deliver the nucleic acid to a kidney tissue in the individual. In certain embodiments, the lipid-nucleic acid mixture comprises one or more lipid compounds selected from the group consisting of: lipid 88, lipid 91, lipid 93, lipid 94, lipid 96, lipid 97 and lipid 98.

In certain embodiments, the nucleic acid is delivered to the liver. In certain embodiments, the lipid-nucleic acid mixture, after oral administration to an individual, can deliver the nucleic acid to a spleen tissue in the individual. In certain embodiments, the lipid-nucleic acid mixture comprises one or more lipid compounds selected from the group consisting of lipid 76, lipid 77, lipid 79, lipid 80, lipid 81, lipid 95, lipid 96, lipid 98, lipid 99, lipid 100, lipid 101 and lipid 102.

In certain embodiments, the lipid-nucleic acid mixture, after oral administration to an individual, can deliver the nucleic acid to the blood in the individual. In certain embodiments, the lipid-nucleic acid mixture comprises one or more lipid compounds selected from the group consisting of lipid 75, lipid 102, lipid 104 and lipid 105.

In certain embodiments, the lipid-nucleic acid mixture, after oral administration to an individual, can deliver the nucleic acid to an intestinal tissue in the individual. In certain embodiments, the lipid-nucleic acid mixture comprises the lipid compound lipid 83.

In certain embodiments, the lipid-nucleic acid mixture, after oral administration to an individual, can deliver the nucleic acid to two or more tissues. In certain embodiments, the lipid-nucleic acid mixture comprises a lipid compound selected from the group consisting of lipid 76, lipid 80, lipid 94, lipid 95, lipid 96, lipid 97, lipid 98 and lipid 102.

In certain embodiments, the nucleic acid delivery comprises in vitro cell delivery. "In vitro" as used herein means outside a multicellular organism, for example, outside a human or animal body. In vitro cells include, for example, in vitro cell cultures, ex-vivo tissue or cells, etc. The in vitro delivery includes the delivery of nucleic acid into cells in vitro, including for example, contacting the lipid-nucleic acid mixture with in vitro cells under conditions that allow the nucleic acid to enter the cells.

### Pharmaceutical composition

In another aspect, the present invention further provides a pharmaceutical composition, comprising a lipid composition and a nucleic acid, wherein the lipid composition comprises one or more compounds of Formula (I), or a pharmaceutically acceptable salt, hydrate or solvate thereof. In certain embodiments, the compound is selected from lipids 106, 96, 93, 94, 84, 85, 108, 81, 88, 89, 109, 110, 103, 104, and 105.

In another aspect, the present invention further provides a pharmaceutical composition, comprising a lipid composition and a nucleic acid. The lipid composition comprises one or more compounds selected from the group consisting of lipids 72, 73, 74, 75, 76, 77, 78, 79, 80, 82, 83, 86, 87, 90, 91, 92, 95, 97, 98, 99, 100, 101, 102, 107, 111, 112, 113, 114, 115, 116, 117, 118, and 119, or a salt, a hydrate or a solvate thereof.

The pharmaceutical composition provided herein may be in a solid or liquid form, including a semi-solid, semi-liquid, suspension and gel form. The solid form includes, for example, a tablet or powder form. The liquid form includes, for example, an oral solution, oral syrup, injectable liquid, or aerosol, suitable for, for example, administration by inhalation.

In certain embodiments, the pharmaceutical composition is formulated for oral administration, or administration by inhalation, via the digestive tract, or via the respiratory tract. In certain embodiments, the pharmaceutical composition is formulated to deliver the nucleic acid by in vivo gastrointestinal delivery. In certain embodiments, the pharmaceutical composition is an oral pharmaceutical composition. When administered orally, the pharmaceutical composition is preferably in a solid or liquid form.

As a solid pharmaceutical composition for oral administration, the pharmaceutical composition can be formulated into the form of powders, granules, compressed tablets, pills, capsules, and chewable gums, etc. Such solid compositions generally further comprises one or more inert diluents or edible carriers (such as starch, lactose or dextrin), and one or more adjuvants selected from the group consisting of a binder (such as carboxymethyl cellulose, ethyl cellulose, microcrystalline cellulose, tragacanth or gelatin), a disintegrant (alginic acid, sodium alginate, Primogel, corn starch, etc.), a lubricant (such as magnesium stearate or Sterotex), a glidant (e.g. colloidal silica), a sweetener (such as sucrose or saccharin), a flavoring agent, and a colorant.

As a liquid pharmaceutical composition for oral administration, the pharmaceutical composition can be, for example, formulated into, an elixir, a syrup, a solution, an emulsion or a suspension. When administered orally, the liquid pharmaceutical composition comprises one or more adjuvants selected from the group consisting of a sweetener, a preservative, a dye/colourant and a flavour enhancer.

The pharmaceutical composition of the present invention may also be formulated for administration by inhalation via the respiratory tract. Suitable preparations for inhalation may include those administered as an aerosol. The aerosols can be delivered in a single-phase, a two-phase system or a three-phase system. For example, the aerosol can be delivered by a liquefied and compressed gas, or by a suitable system for dispersing the active ingredient. The delivery of an aerosol involves a necessary container, activator, valve, and sub-container, etc., which are be formed into a kit.

In certain embodiments, the pharmaceutical composition of the present invention can also be formulated for parenteral administration, for example, administration by injection. The liquid composition for injection can be a solution, a suspension or an injection powder, which may include one or more of a surfactant, a preservative, a moisturizer, a dispersant, a suspension, a buffer, and a stabilizer and an isotonic agent.

In certain embodiments, the pharmaceutical composition may also be used to deliver the nucleic acid by in vitro cell delivery.

In certain embodiments, at least part of or all of the lipid composition and the nucleic acid exist in the form of a mixture in the pharmaceutical composition. In certain embodiments, the mixture is prepared by a heating method, a reverse evaporation method, or direct mixing.

In another aspect, the present invention also provides use of the pharmaceutical composition in the manufacture of a medicament for preventing and/or treating disease that can be prevented and/or treated by the nucleic acid, or for in vivo delivering the nucleic acid to a subject in need thereof.

The pharmaceutical composition provided herein can be used to treat disease that can be treated by the nucleic acid. Common disease include, for example, but are not limited to, cancer, inflammation, fibrotic disease, autoimmune disease or autoinflammatory disease, bacterial infection, behavioral and psychiatric disorder, hematological disease, chromosomal disorder, congenital and genetic disease, connective tissue disease, digestive disease, ear, nose, and throat disease, endocrine disease, environmental illness, eye disease, female reproductive system disease, fungal infection, heart disease, hereditary cancer syndromes, immune system disorder, kidney and urinary tract disorder, pulmonary disease, male reproductive system disease, metabolic disorder, mouth disease, musculoskeletal disorder, myelodysplastic syndrome, newborn screening, nutritional disease, parasitic disease, rare cancer, rare disease, and skin disease and viral infection.

Examples of cancer include, but are not limited to, gastric cancer, lung cancer, colorectal cancer, liver cancer, pancreatic cancer, cervical cancer, breast cancer, leukemia, and pultiple myeloma.

Examples of inflammation include, but are not limited to, pneumonia, myocarditis, acute and chronic gastritis, acute and chronic enteritis, acute and chronic hepatitis, acute and chronic nephritis, dermatitis, encephalitis, lymphangitis, conjunctivitis, keratitis, iridocyclitis, otitis media, allergic rhinitis, asthma, pulmonary fibrosis, chronic obstructive pulmonary disease, allergic dermatitis, sickle cell disease, multiple sclerosis, systemic lupus erythematosus, and lupus nephritis.

Known nucleic acid medicaments are able to treat a variety of diseases. Known indications of the nucleic acid medicaments include, for example, hepatocellular carcinoma, corneal neovascularization, recurrent or refractory anaplastic astrocytoma (WHO grade III) or secondary glioblastoma (WHO grade IV), advanced squamous cell lung cancer, acromegaly, psoriasis, Duchenne muscular dystrophy, advanced non-small cell lung cancer, metastatic castration-resistant prostate cancer, cytomegalovirus retinitis, HIV infection, Hepatitis B, Hepatitis C, hyperlipoproteinemia, total knee replacement, diabetes mellitus type II, familial amyloid polyneuropathy (FAP), wet macular degeneration (e.g., neovascular age-related macular degeneration, subfoveal neovascular age-related macular degeneration, and exudative age-related macular degeneration), hypercholesterolemia, Crohn's disease, extensive liver fibrosis, infantile spinal muscular atrophy, melanoma, neonatal coronary artery disease, mild allergic asthma, chronic lymphocytic leukemia, hypertriglyceridemia, hepatic veno-occlusive disease complicated with renal or pulmonary dysfunction after hematopoietic stem cell transplantation, and hereditary transthyretin amyloidosis.

### Kit

In another aspect, the present invention further provides a kit comprising one or more compounds of Formula (I), or a pharmaceutically acceptable salt, hydrate or solvate provided in a first container, and a nucleic acid provided in a second container. In certain embodiments, the compound is selected from the group consisting of lipids 106, 96, 93, 94, 84, 85, 108, 81, 88, 89, 109, 110, 103, 104, and 105.

In another aspect, the present invention further provides a kit comprising one or more compounds, or a pharmaceutically acceptable salt, hydrate or solvate provided in a first container, and a nucleic acid provided in a second container. The one or more compounds is/are selected from the group consisting of lipids 72, 73, 74, 75, 76, 77, 78, 79, 80, 82, 83, 86, 87, 90, 91, 92, 95, 97, 98, 99, 100, 101, 102, 107, 111, 112, 113, 114, 115, 116, 117, 118, and 119.

In certain embodiments, in the kit, at least part of or all of the lipid composition and the nucleic acid are formulated into a lipid-nucleic acid mixture prior to use. In certain embodiments, in the kit, the lipid-nucleic acid mixture is prepared by a heating method, a reverse evaporation method, or direct mixing.

In certain embodiments, the kit is prepared into an oral pharmaceutical composition.

In certain embodiments, the kit is prepared for oral administration, or administration by inhalation, via the digestive tract, or via the respiratory tract.

In certain embodiments, the kit is prepared for delivering the nucleic acid by in vitro cell delivery, or by in vivo gastrointestinal delivery.

In another aspect, the present invention provides use of the kit in the manufacture of a medicament for preventing and/or treating disease that can be prevented and/or treated by the nucleic acid, or for in vivo delivering the nucleic acid to a subject in need thereof.

### Method for delivering nucleic acid

In another aspect, the present invention provides a method for delivering a nucleic acid to a target cell, comprising administering, to the target cell, the pharmaceutical composition provided herein, or a lipid-nucleic acid mixture prepared with the kit provided herein.

In another aspect, the present invention provides a method for in vivo delivering a nucleic acid to a subject in need thereof, comprising administering, to the subject, the pharmaceutical composition provided herein, or a lipid-nucleic acid mixture prepared with the kit provided herein.

In certain embodiments, the subject is a human or an animal, such as a mammal.

In certain embodiments, the nucleic acid is delivered in vivo to the blood circulation or to a target tissue/cell in the subject.

In certain embodiments, the method comprises administering the medicament by oral administration, inhalation or injection.

In certain embodiments, the method comprises administering the medicament by administration via the digestive tract, or via the respiratory tract.

In certain embodiments, the method comprises administering the medicament by oral administration.

Through the above technical solutions provided herein, the efficient targeted delivery of nucleic acids can be significantly improved, to overcome the defects of low encapsulation rate, poor safety, poor stability, complicated production process, non-uniform product, difficulty in reproduction, and to-be-further-improved targeting performance associated with the nucleic acid-liposome in the prior art.

The features mentioned herein, or the features mentioned in the examples can be combined arbitrarily. All the features disclosed in the present specification can be used in any combination, and each feature disclosed in the specification can be replaced by any alternative features providing the same, equivalent or similar purposes. Therefore, unless otherwise specified, the disclosed features are merely general examples of equivalent or similar features.

The present invention is further described below in conjunction with specific examples. It should be understood that these examples are merely illustrative of the present invention and are not intended to limit the scope of the present invention.

### Example 1: Extraction, identification, and synthesis of traditional Chinese medicine-derived lipids

### 1.1. Boiling of traditional Chinese medicine

1) 100 g of decoction pieces of traditional Chinese medicine (*Rhodiola crenulata,* purchased from Ningbo Haishu Heavenlyherba Biochem Co., Ltd; and *Taraxacum mongolicum, Lonicera japonica,* and *Andrographis paniculata,* purchased from Beijing Tongrentang Pharmacy), was added to 1000 mL of ddH₂O and soaked for 30 min.
2) The traditional Chinese medicine was heated intensely for 15 min, and then slowly for 20 min.
3) 400 mL of the heated traditional Chinese medicine soup was added to a rotary evaporator, and concentrated to 100 mL at 60°C and 60 rpm, for 30 min.

### 1.2. Extraction of traditional Chinese medicine-derived lipids

1) 600 mL of a chloroform-methanol mixture (chloroform:methanol=1:2, v/v) was added to 160 mL of the traditional Chinese medicine soup (concentrated by a rotary evaporator) prepared in section 1.1, to give a ratio of chloroform:methanol:water of 1:2:0.8, and mixed uniformly by stirring for 10-15 min.
2) 200 mL of chloroform was added to the conical flask and mixed uniformly by stirring for 10 min.
3) 200 ml of ddH₂O was added to the conical flask, to give a ratio of chloroform:methanol:water of 2:2:1.8, and mixed uniformly by stirring for 10 min.
4) The liquid in the upper layer and the insoluble substances in the middle layer were removed, and the lower chloroform layer was collected, and stored at -40°C.

### 1.3. Identification of traditional Chinese medicine-derived lipids

The lipid components derived from traditional Chinese medicine were identified by HPLC-MS/MS.

### Instrument conditions

### 1) Chromatographic conditions:

Instrument: Ultimate 3000; Chromatography column: Kinetex C18 (100 x 2.1 mm, 1.9 µm); Column temperature: 45°C; Mobile phase: A: acetonitrile:water (V/V, 60:40), containing 10 mmol/L ammonium formate, Mobile phase B: acetonitrile:isopropanol (10:90, V/V), containing 10 mmol/L ammonium formate and 0.1% formic acid. Flow rate: 0.4 mL/min; Volume of injection: 4 µL.

### 2) MS parameters:

a) Positive mode: heater temperature: 300°C, sheath gas flow rate: 45 arb, auxiliary gas flow rate: 15 arb, purge gas flow rate: 1 arb, nozzle voltage: 3.0 KV, capillary temperature: 350°C, S-Lens RF Level: 30%, and sweep range: 200-1500.
b) Negative mode: heater temperature: 300°C, sheath gas flow rate: 45 arb, auxiliary gas flow rate: 15 arb, purge gas flow rate: 1 arb, nozzle voltage: 2.5KV, capillary temperature: 350°C, S-Lens RF Level: 60%, and sweep range: 200-1500.

### 1.4. Synthesis of traditional Chinese medicine-derived lipids

For the identified lipids 72-119, each lipid is commercially available except for the lipids 106, 108, and 110.

### Example 2: Preparation of lipid-nucleic acid mixture (heating method):

The concentration of a lipid solution in chloroform was 10 mg/ml (stock solution), and grouping was performed according to the lipid compound number.

1 nmol of small RNA (as shown in Table 2) was dissolved in 400 µl of DEPC-treated water in a glass tube, and 10 µl of a corresponding lipid stock solution was added, to give a system containing 100 µg of lipid. The system was well mixed, heated for 15 min in a water bath at 90°C, and then naturally cooled, to obtain a lipid-nucleic acid mixture of the lipid and small RNA.

**Table 2. Sequence information of small RNA (obtained from Guangzhou Ribo Biotechnology Co., Ltd.)**

| **Name** | **Sequence** | **Modification** |
|---|---|---|
| PGY-sRNA-26 | TCCGGAATGATTGGGCGTAAAGCGT (SEQ ID No. 1) | 3 '-methylated |
| PGY-sRNA-26 Reverse transcription primer | GTCGTATCCAGTGCACGCTCCGAGGTATTC GCACTGGATACGACACGCTT (SEQ ID No. 2) | |
| PGY-sRNA-26 Forward primer | TCGCGCTCCGGAATGATTGGG (SEQ ID No. 3) | |
| PGY-sRNA-26 Reverse primer | GTGCACGCTCCGAGGT (SEQ ID No. 4) | |

### Example 3: Study on in vivo delivery of lipid-nucleic acid mixture

### 1. Test animals:

The C57BL/6 male mice used in the experiment were purchased from Beijing Vital River Laboratory Animal Technology Co., Ltd., about 6 weeks old, and weighed 20-24 g. They were kept in an SPF animal room of the Animal Center of the Institute of Basic Medical Sciences, Chinese Academy of Medical Sciences. The mice were fasted for 12 hrs before gavage.

### 2. Preparation of lipid-small RNA mixture:

The lipid-nucleic acid mixture was prepared following the method as described in Example 2. The prepared lipid-nucleic acid mixture was orally administered at a dose of 100 µg lipid: 1 nmol single-stranded small RNA PGY-sRNA-26 per mouse.

### 3. Detection of relative intake:

### 3.1. Experimental groups:

1) Blank group: Mice (n=4) were given 500 µl of normal saline by gavage, and this group served as a blank control group;
2) Free intake group: Mice (n=4) were given sRNA solution (1 nmol/animal, 500 µl) by direct gavage, and this group served as a negative control group;
3) Lipid treatment group: Mice (n=4) were given 500 µl of the lipid-nucleic acid mixture prepared in step 2 by gavage.

### 3.2. RNA extraction from tissue samples:

12 hrs after gavage in each group of mice, 500 µl of blood was taken from the eyeball, and 1.5 ml of Trizol Reagent LS (purchased from Invitrogen) was added and mixed well for lysis. The tissue sample was added with 3 ml of Trizol Reagent LS. and homogenized for full lysis, wherein the tissue sample included lung/kidney/spleen/heart/gut. 1 ml of blood or tissue lysate was taken and RNA was extracted according to the following steps.

The blood or tissue lysate was centrifuged at 4°C, and 12,000 rpm for 5 min, the pellet was discarded, and Trizol was transferred to a new centrifuge tube.

Chloroform was added in an amount of 200 µL chloroform/mL Trizol, shaken fully until uniform, and allowed to stand at room temperature for 5 min.

The sample was centrifuged at 4°C, and 12,000 rpm, for 15 min.

The upper aqueous phase was pipetted to another centrifuge tube, and isopropanol was added in an amount of 0.4 mL isopropanol/mL Trizol, mixed well, and allowed to stand at a low temperature for 10-20 min.

The sample was centrifuged at 4°C, and 12,000 rpm, for 15 min, the supernatant was discarded, and the RNA pellet was at the bottom of the tube.

1 mL of 75% ethanol was added, and the centrifuge tube was shaken gently, to suspend the pellet.

The sample was centrifuged at 4°C, and 12,000 rpm, for 10 min, the supernatant was discarded, 1 mL of 75% ethanol was added, and the centrifuge tube was shaken gently, to suspend the pellet.

The sample was centrifuged at 4°C, and 12,000 rpm, for 10 min, the supernatant was discarded, and the sample was air dried at room temperature. The RNA sample was dissolved in 50-100 µL of RNase-free H₂O, and the RNA concentration was quantified by determining the OD value.

### 3.3. Reverse transcription of sRNA into cDNA:

The sRNA was reversely transcribed into cDNA using a reverse transcription kit (High-Capacity cDNA Reverse Transcription Kits, Applied Biosystems, cat. no. 4368813), following the stem-loop method (see, for example Real-time quantification of microRNAs by stem-loop RT-PCR, Nucleic Acids Res. 2005 Nov 27;33(20):e179, incorporated herein by reference). System for reverse transcription: template RNA (200 ng/µL) 10 µL, 10X RT buffer 2.0 µL, 25X dNTP Mix (100 mM) 0.8 µL, U6 RT stem-loop primer 2.0 µL, PGY-sRNA-26 RT stem-loop primer 2.0 µL, MultiScribe^{™} reverse transcriptase 1.0 µL, RNAase inhibitor 1.0 µL, and nuclease-free water H₂O 1.2 µL. After transient centrifugation, the system was fed to a PCR machine, and reacted under the following conditions: (1) 25°C for 10 min; (2) 37°C for 120 min; (3) 85°C for 5 min; and (4) termination at 4°C. After the reaction, 20 µL of RNase-free ddH₂O was added, to make up the final volume to 40 µL. The stem-loop primers used in the reverse transcription process were synthesized by Beijing Hongxun Biotechnology Co., Ltd. (U6 RT primer, because the quantification of small RNA by RT-qPCR reaction can be only relative quantification, U6 was used as a standard reference gene, to calculate the relative expression):
U6 RT stem-loop primer:
   GTCGTATCCAGTGCAGGGTCCGAGGTATTCGCACTGGATACGACAAAAATATG (SEQ ID No. 5);
PGY-sRNA-26 RT stem-loop primer:
   GTCGTATCCAGTGCACGCTCCGAGGTATTCGCACTGGATACGACACGCTT(SEQ ID No. 2).

### 3.4. Quantitative PCR amplification reaction:

The total volume of the qPCR reaction system was 10 µl, comprising: 5 µL 2 x SYBR Green Master Mix, 0.5 µl forward primer (10 µM), 0.5 µl reverse primer (10 µM), 1 µl cDNA obtained by reverse transcription, and 3 µl RNAase-free ddH20. The LightCycler 480 fluorescence quantitative PCR machine was used, the PCR reaction conditions were: pre-denaturation at 95°C for 5 min, then PCR amplification cycles, including 40 cycles of (1) 95°C for 10 s; (2) 55°C for 10 s; and (3) 72°C for 20 s; and final cooling at 40°C for 10 s. The forward primer and reverse primer for the amplification reaction were designed and synthesized by Beijing Tsingke Biotechnology Co., Ltd. (U6 F primer: GCGCGTCGTGAAGCGTTC (SEQ ID No. 6), U6 R primer: GTGCAGGGTCCGAGGT (SEQ ID No. 7); PGY-sRNA-26 forward primer: TCGCGCTCCGGAATGATTGGG (SEQ ID No. 3), reverse primer miR all rev: GTGCACGCTCCGAGGT (SEQ ID No. 4)).

### 3.5. The relative expression level was calculated by the 2-ΔCt method.

### 4. The experimental results are shown graphically.

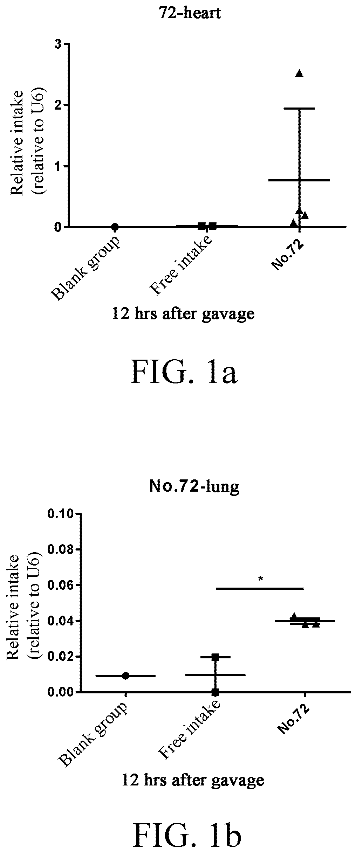

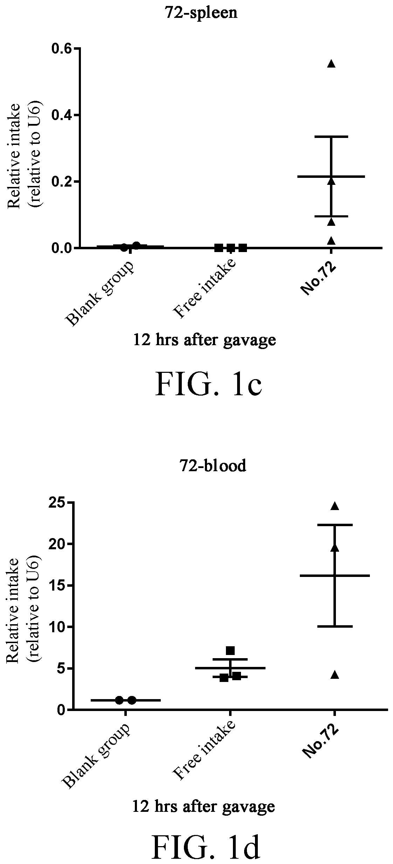

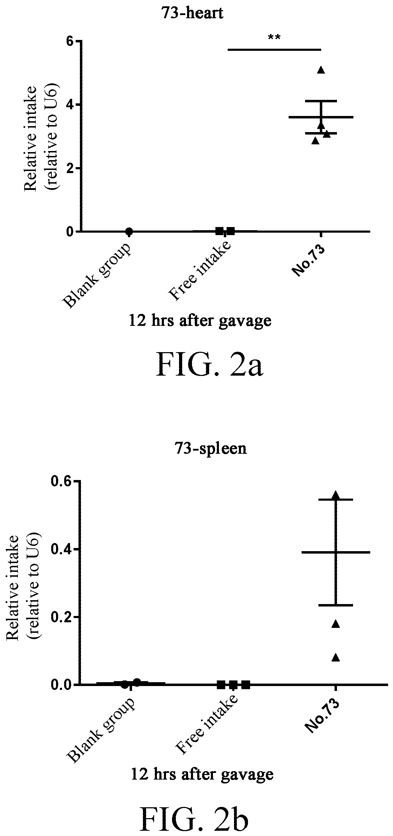

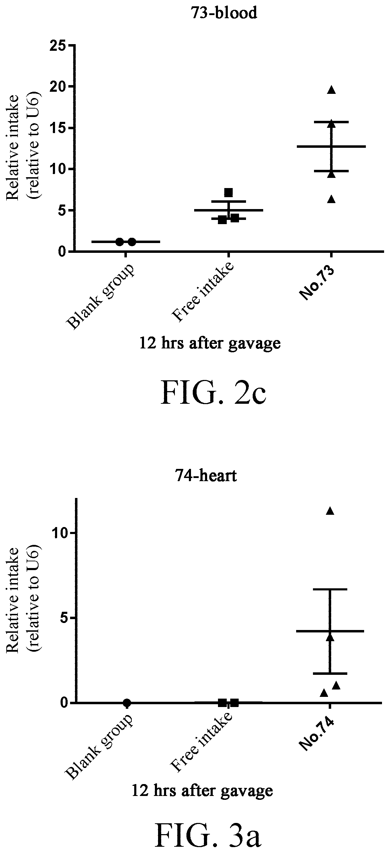

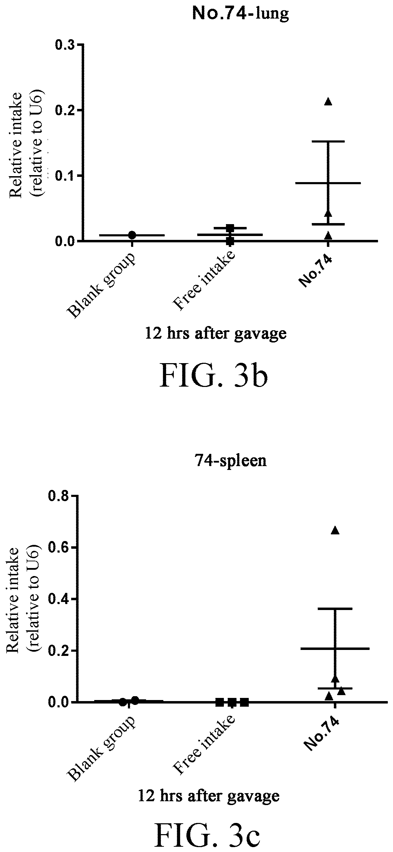

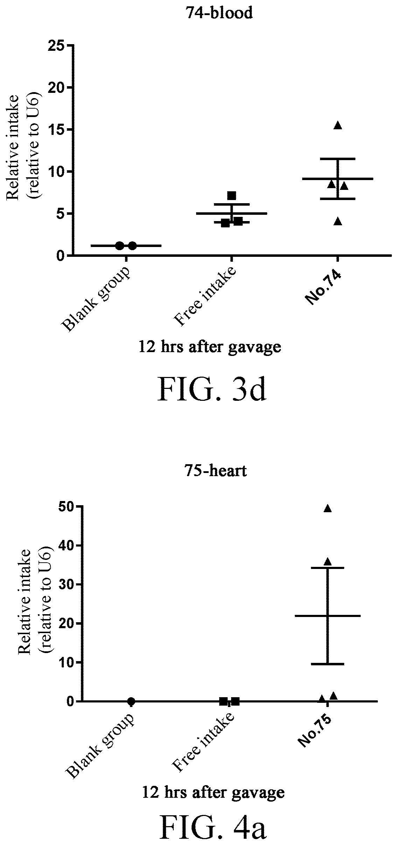

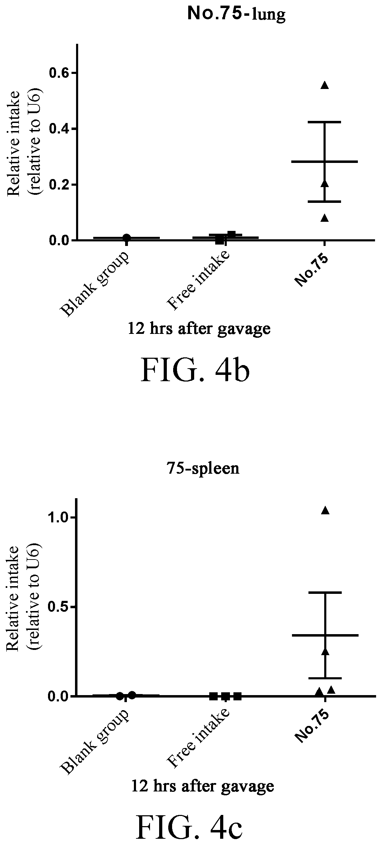

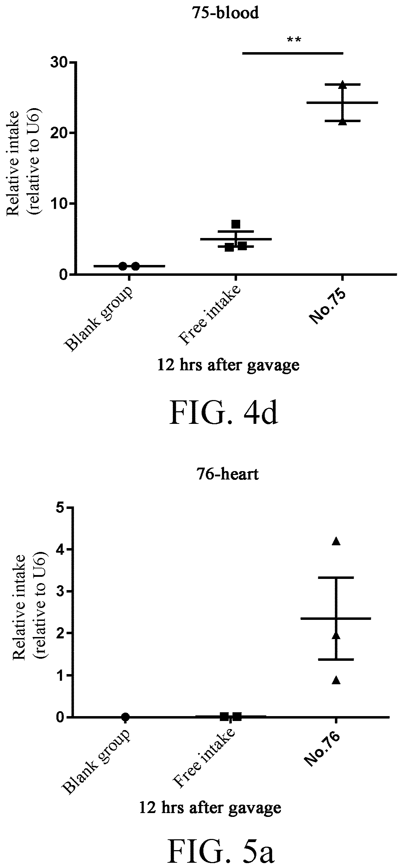

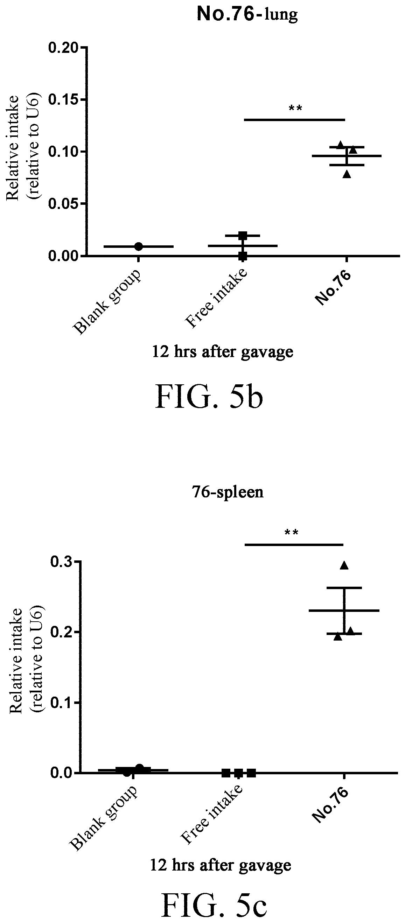

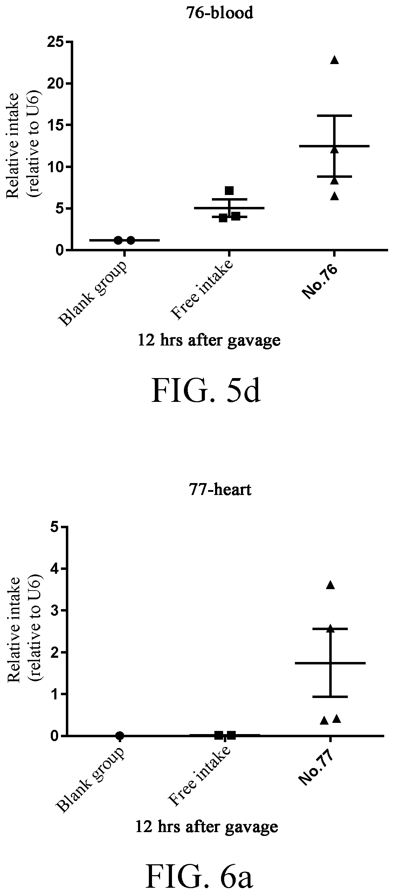

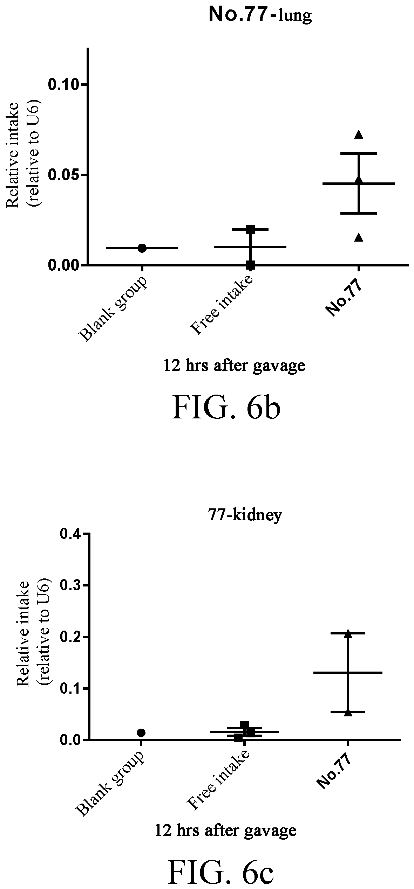

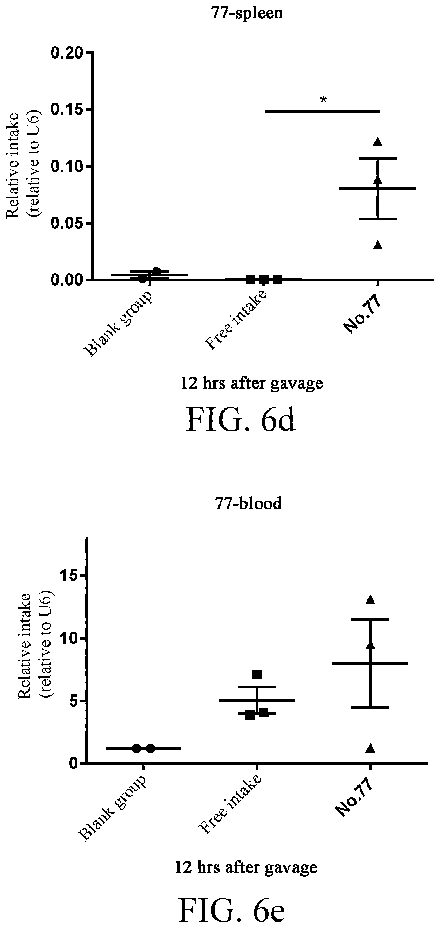

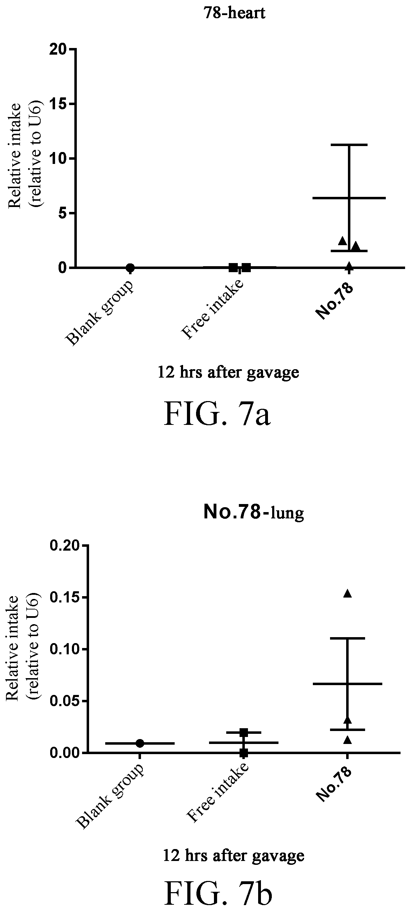

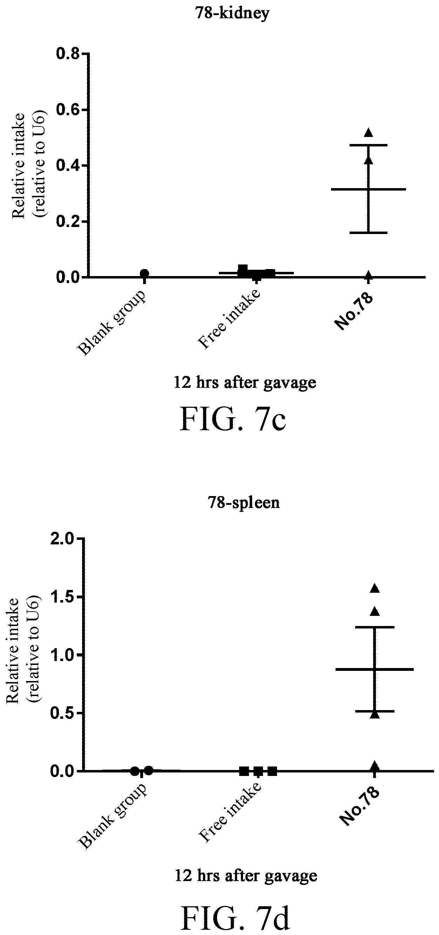

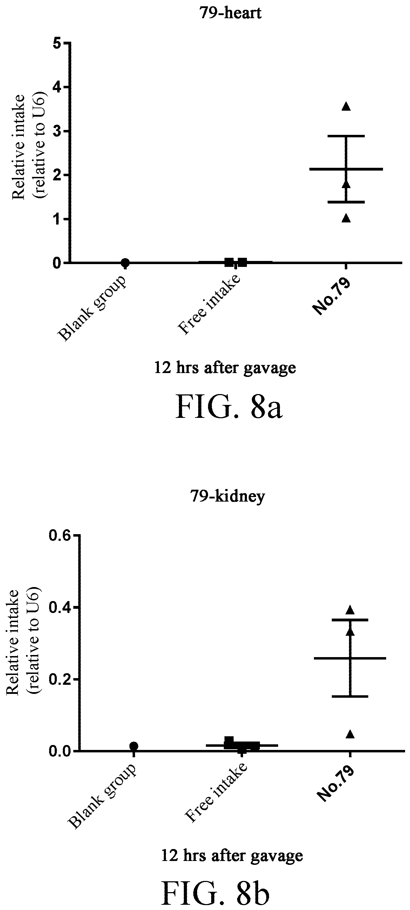

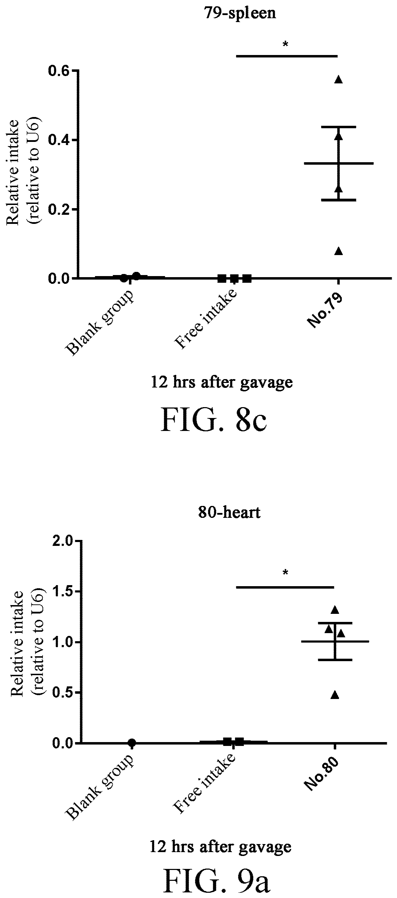

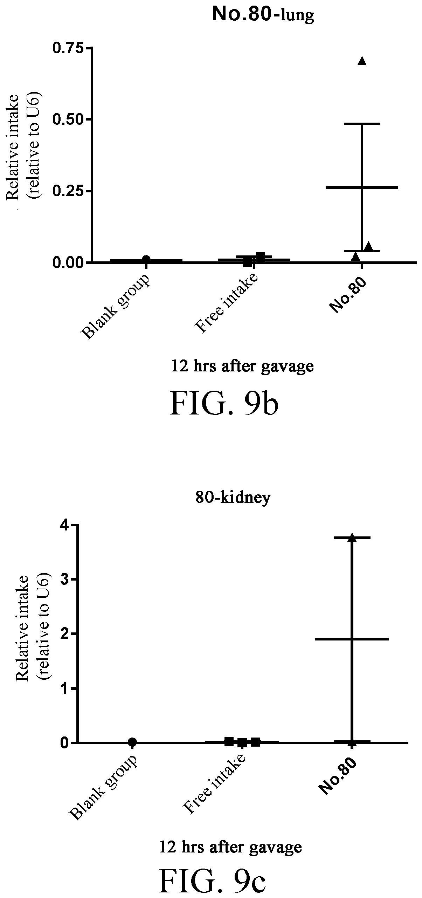

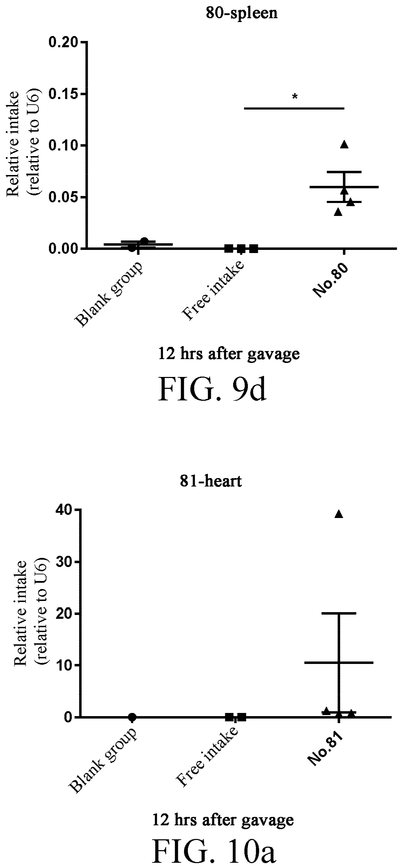

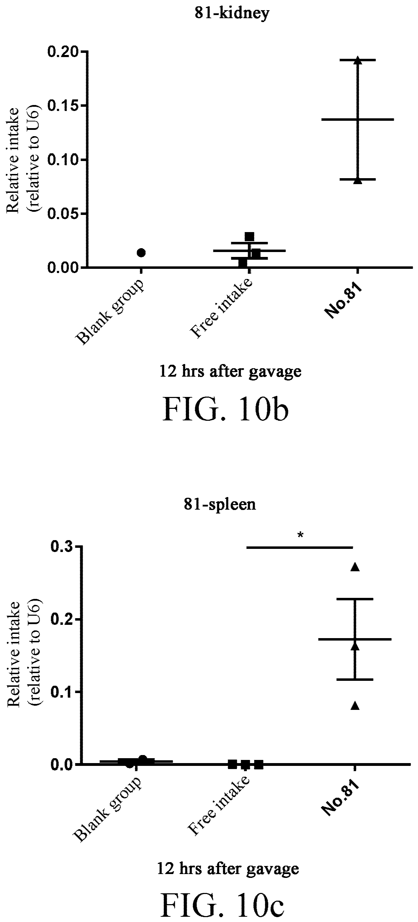

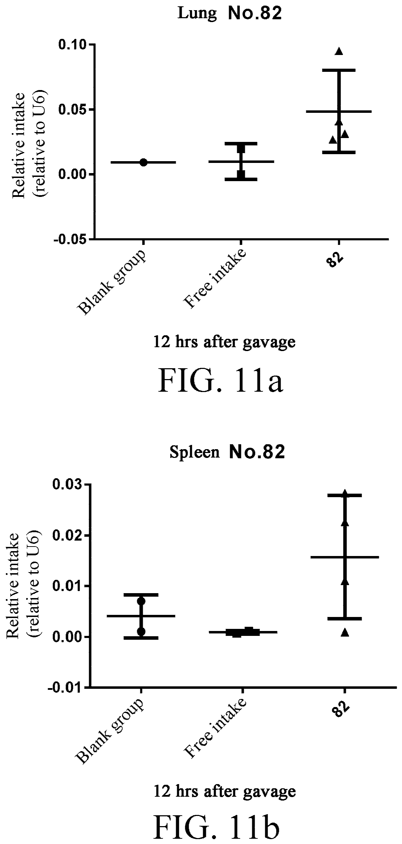

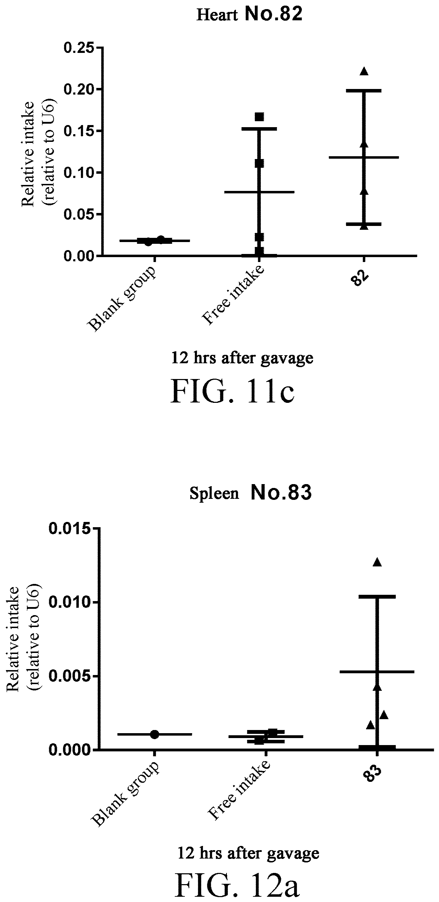

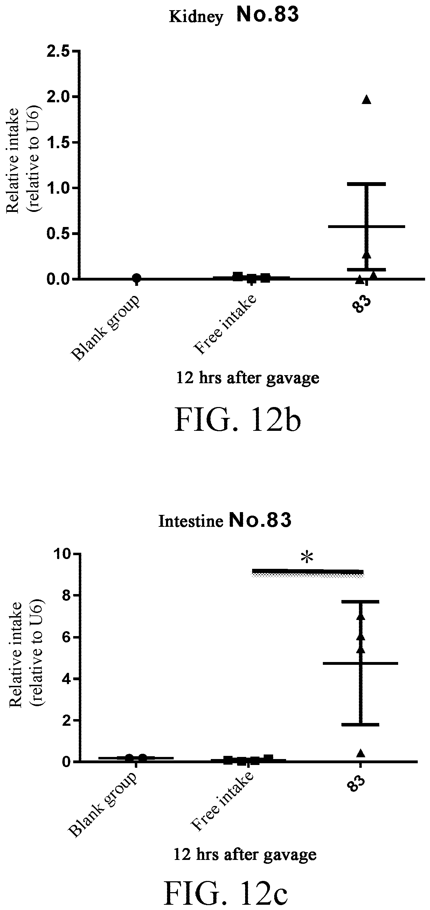

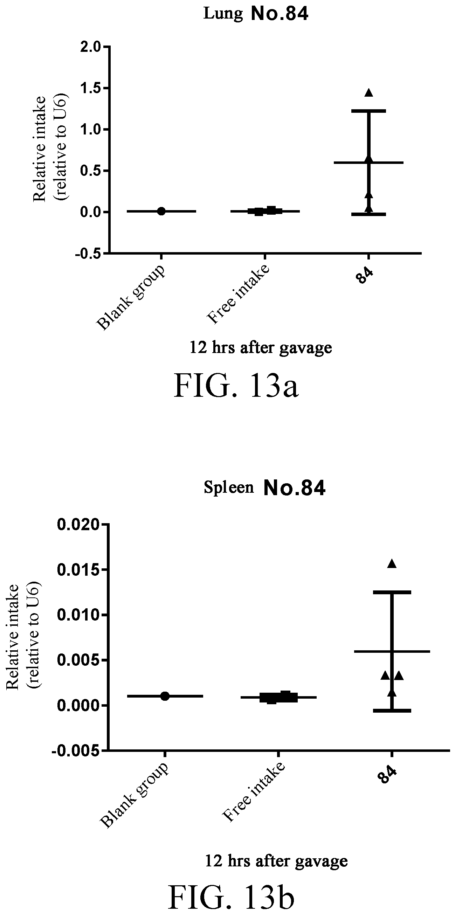

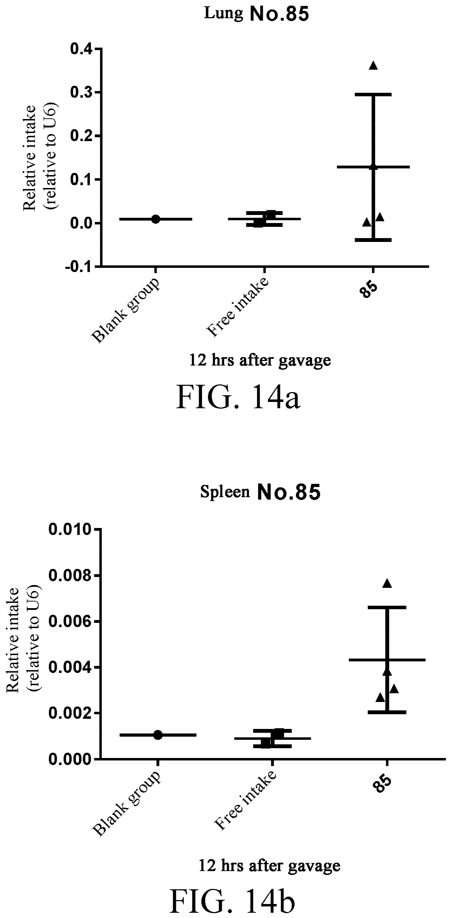

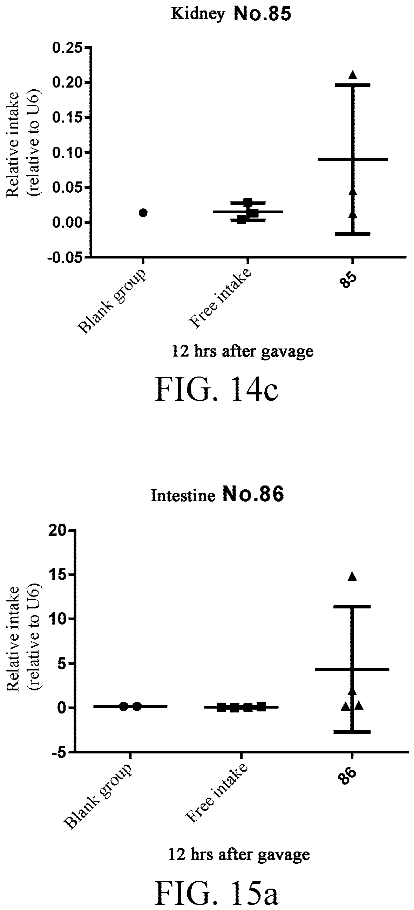

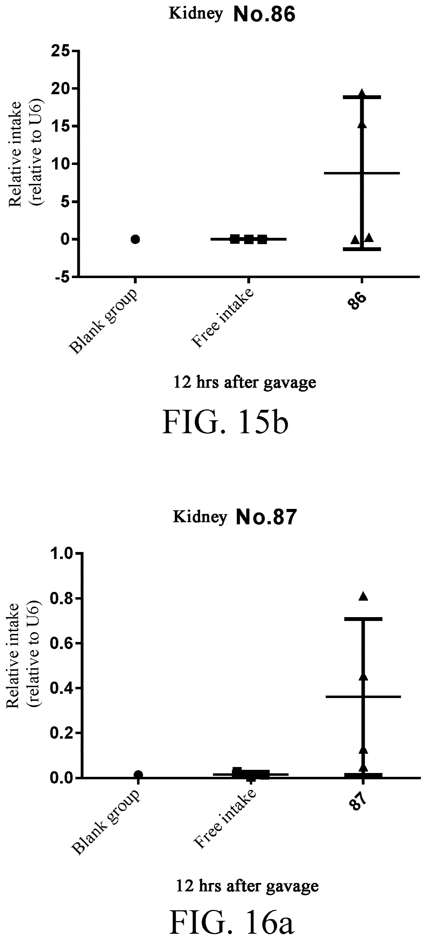

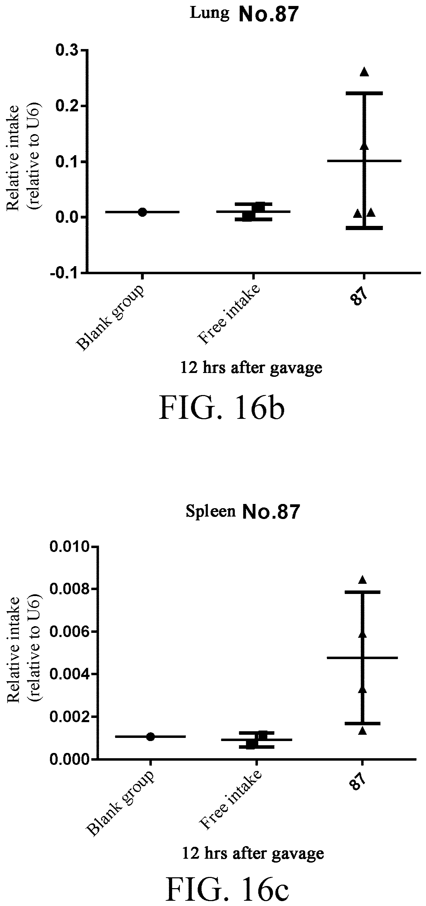

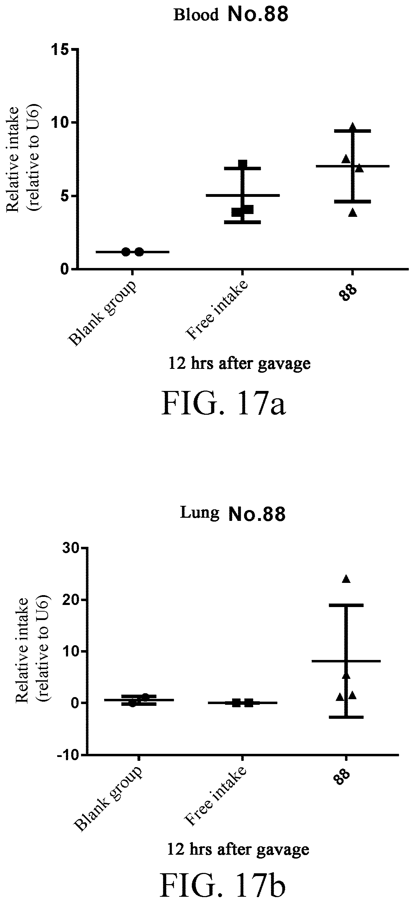

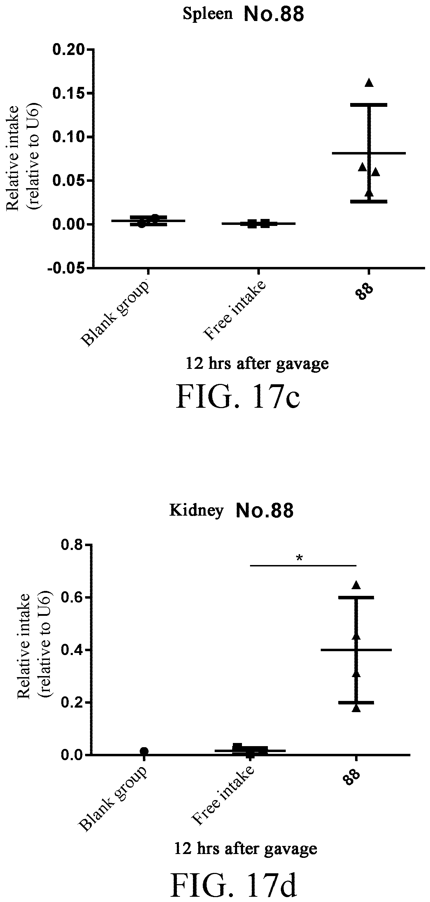

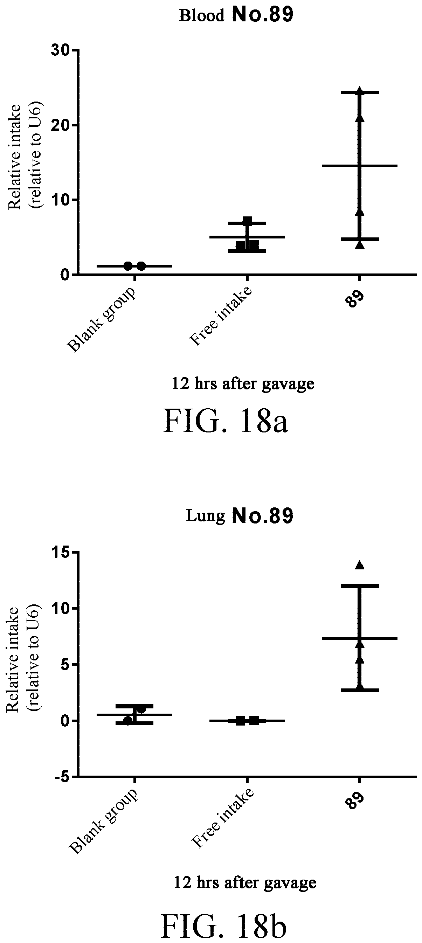

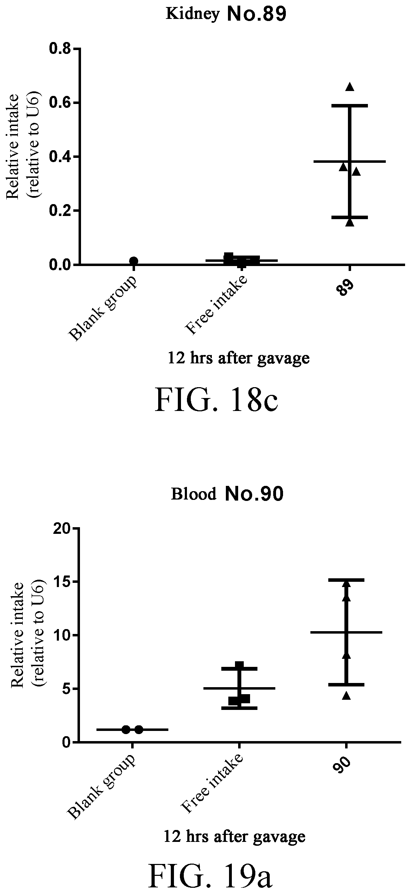

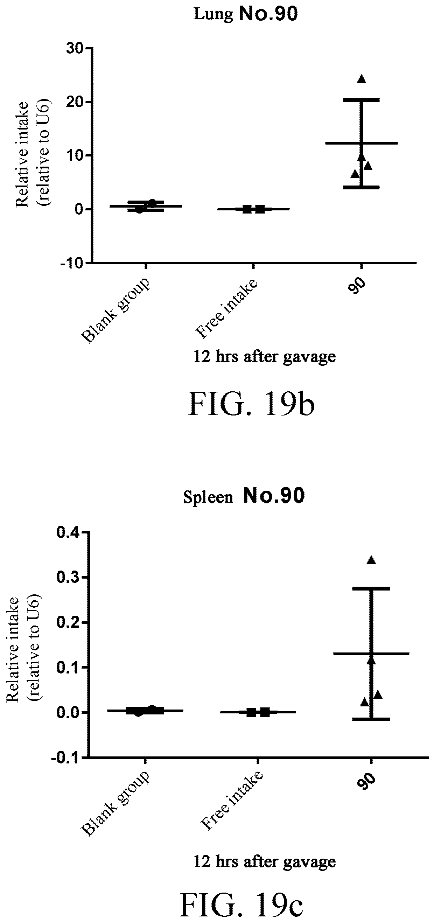

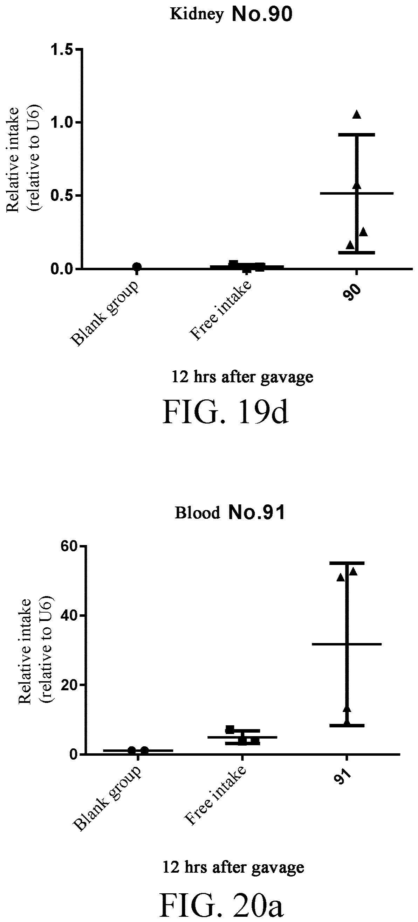

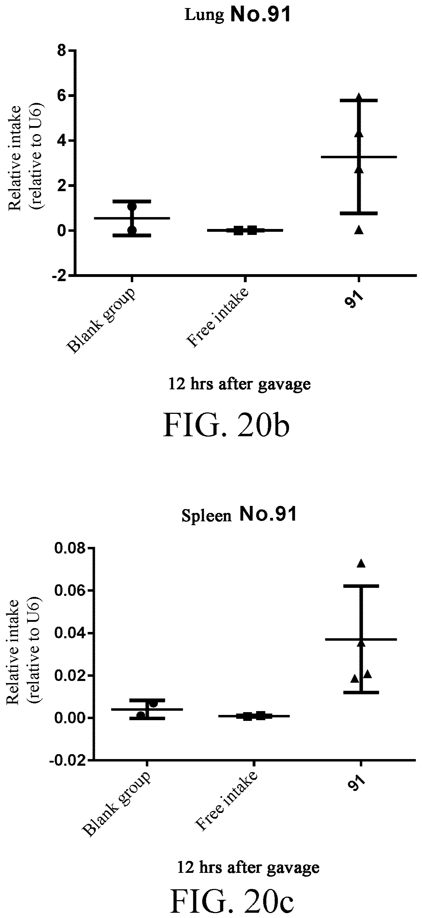

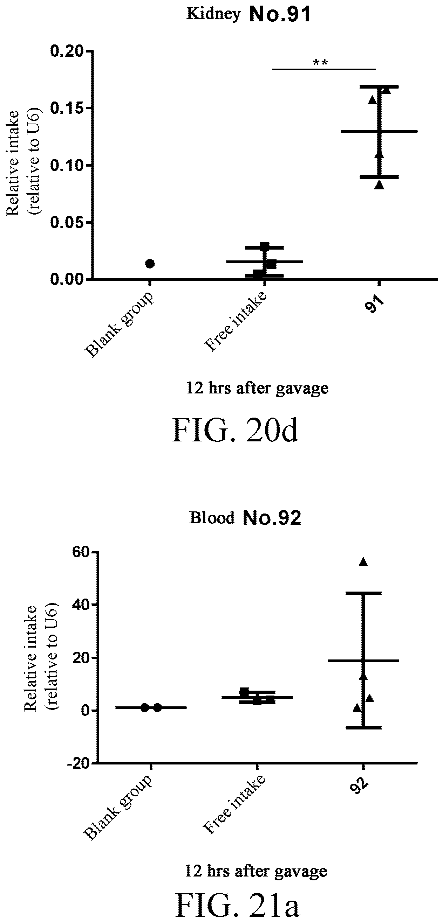

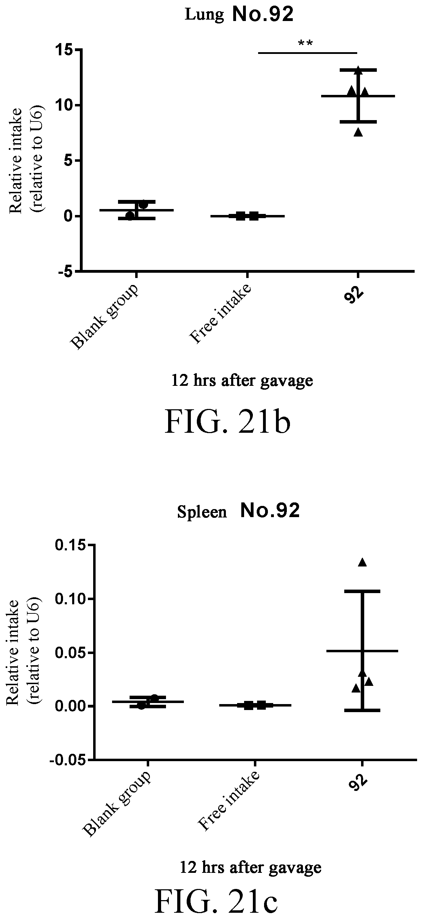

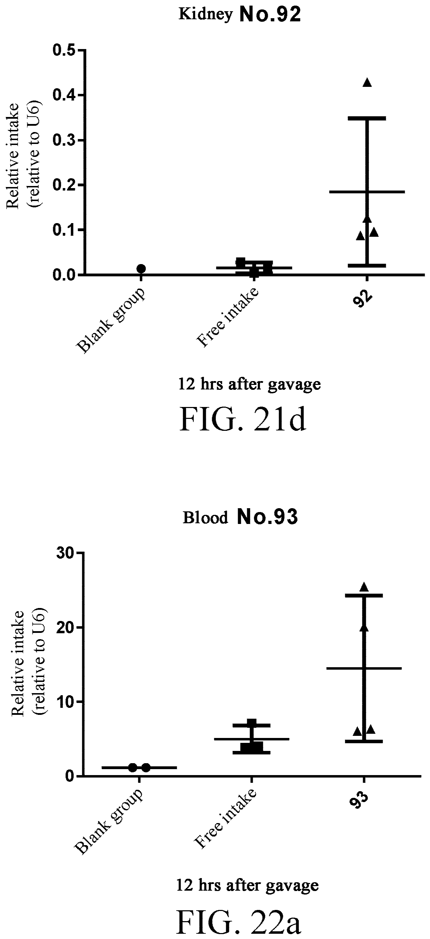

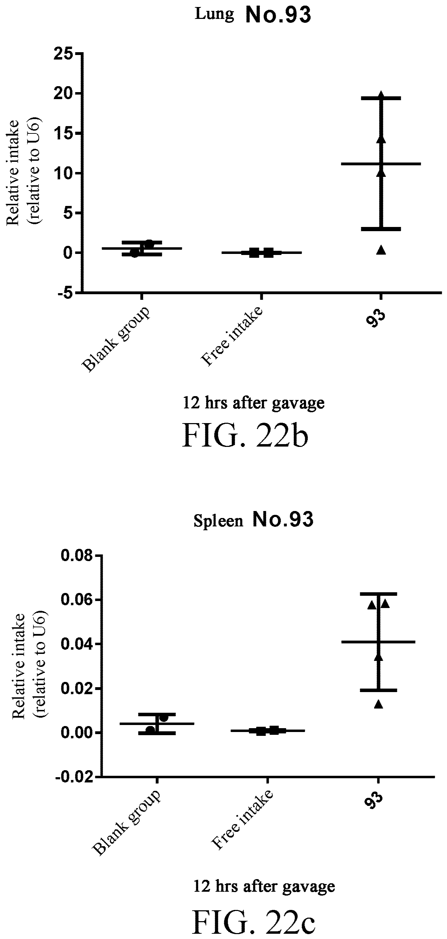

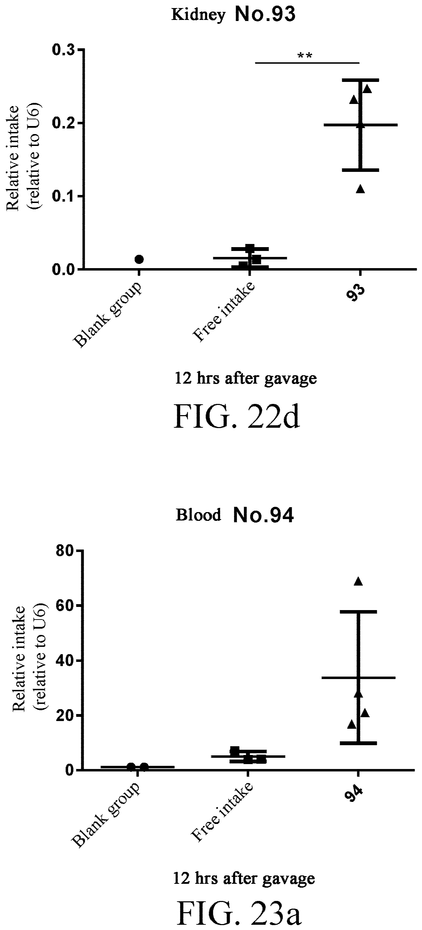

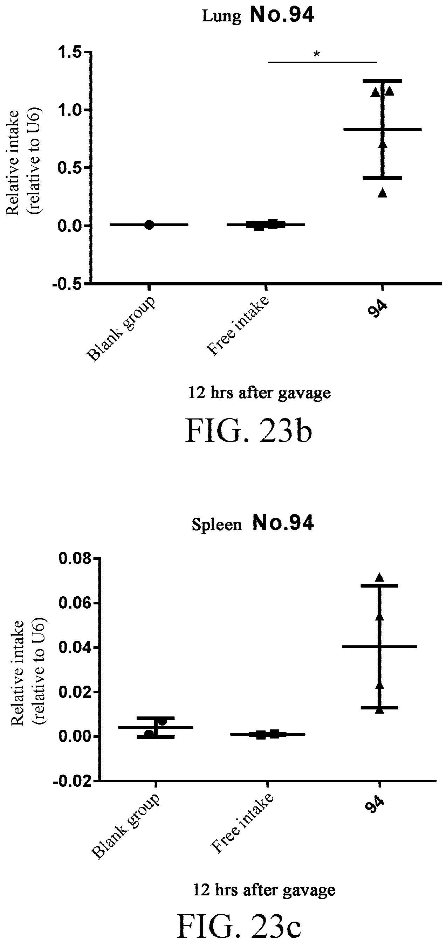

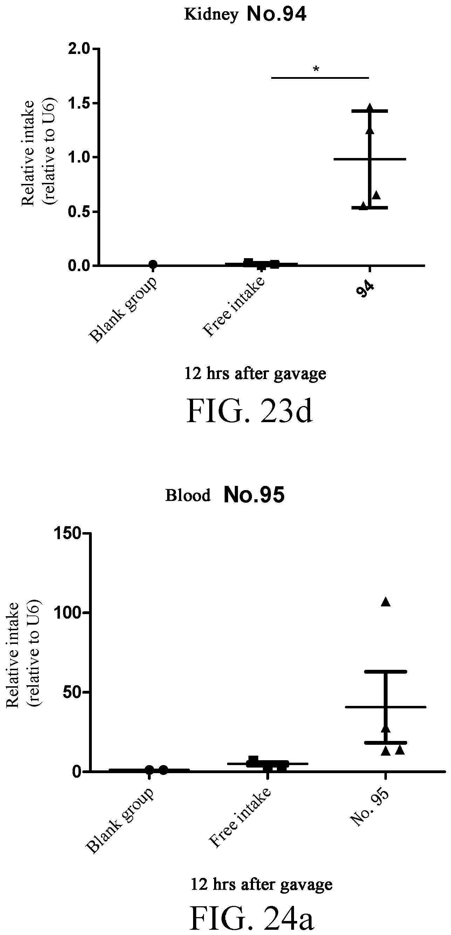

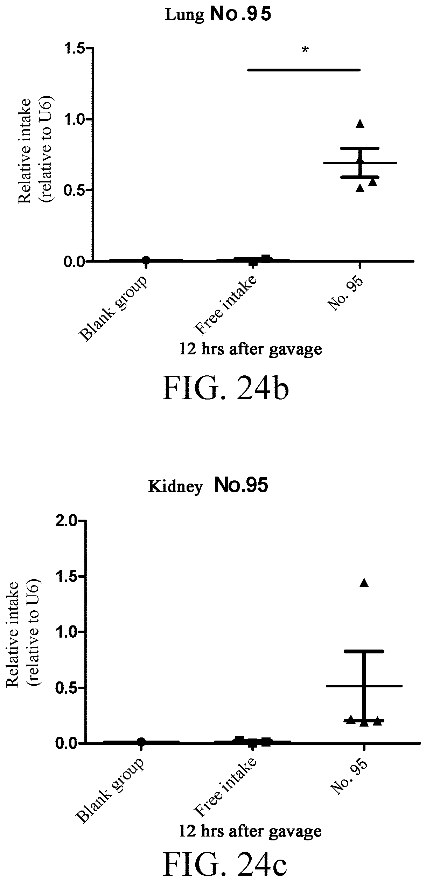

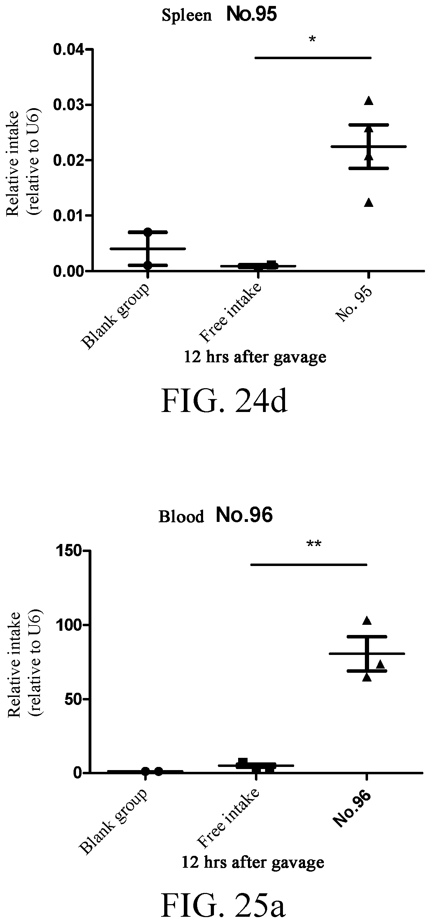

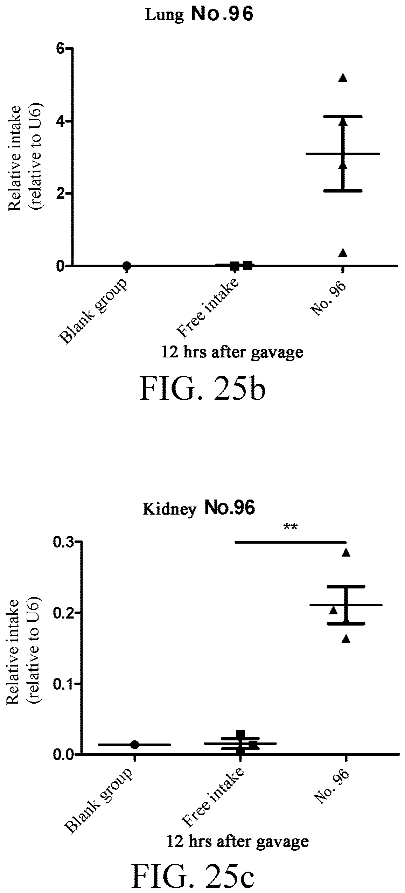

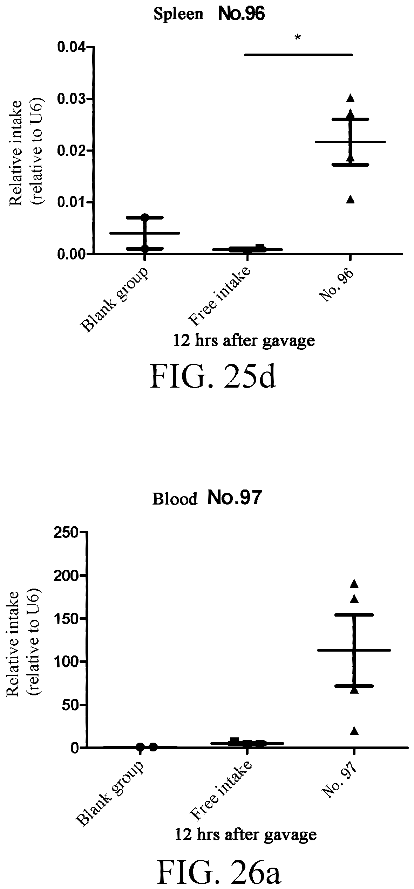

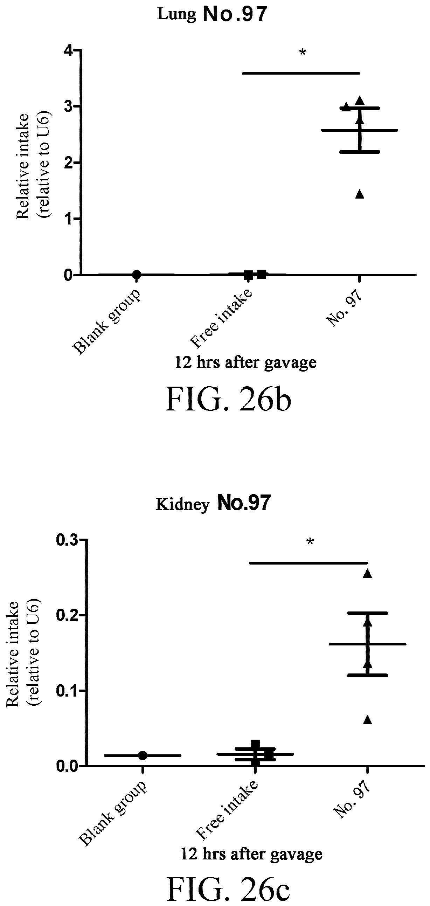

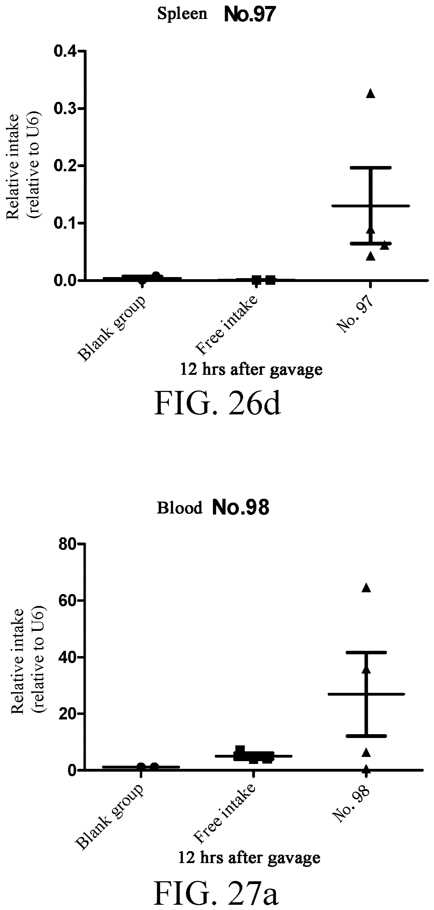

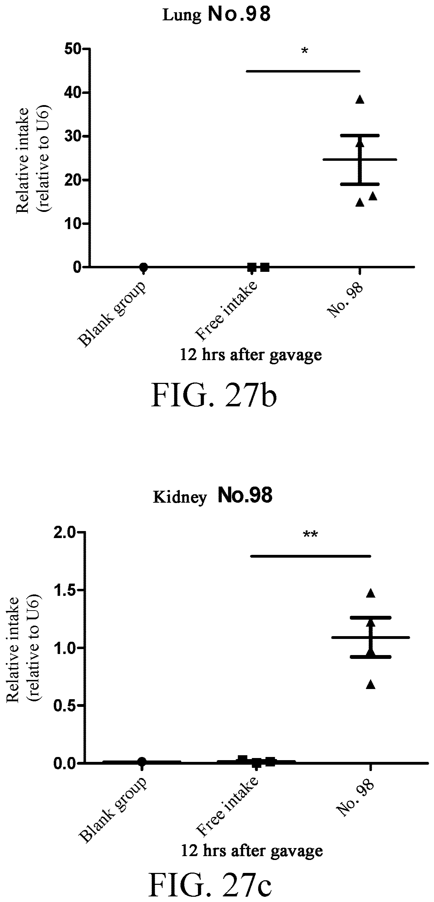

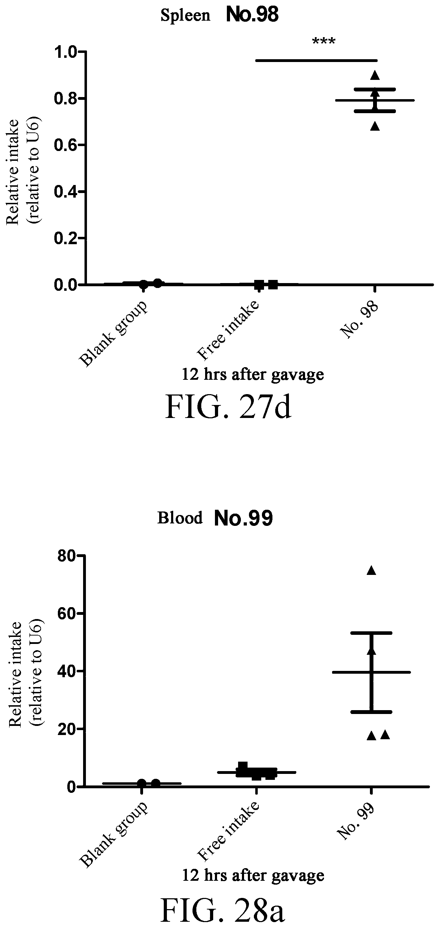

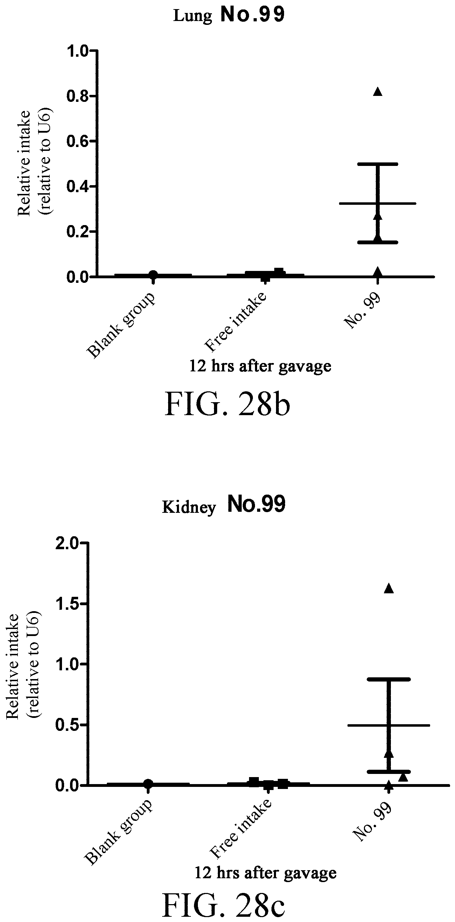

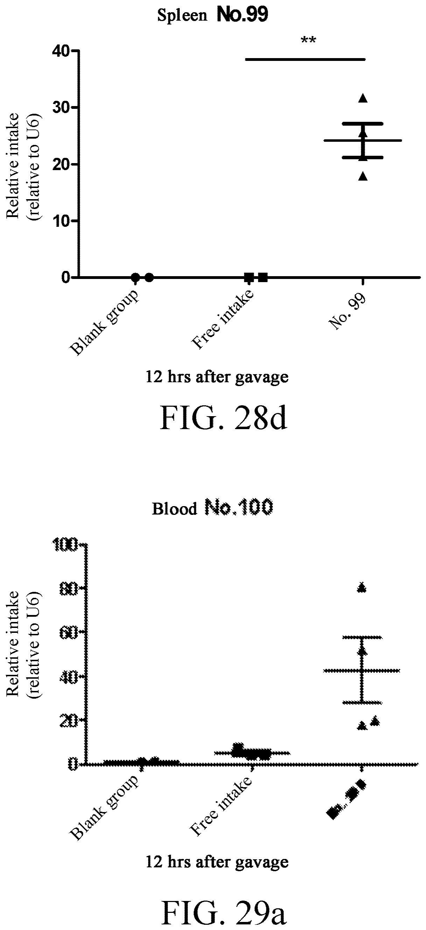

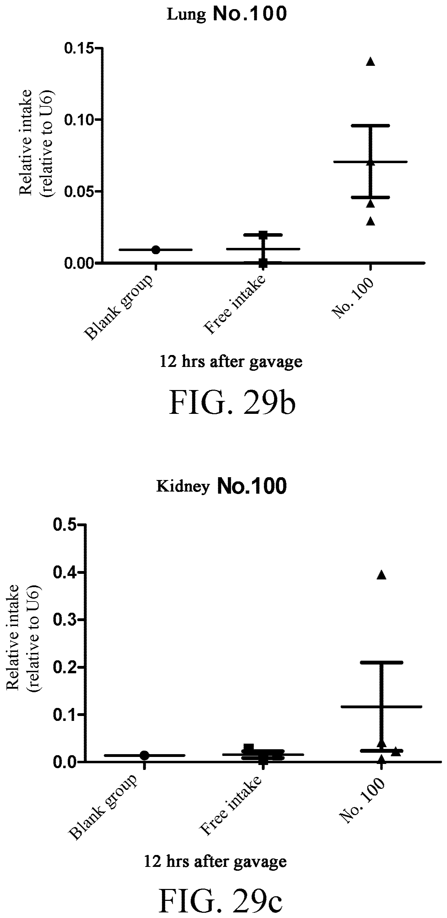

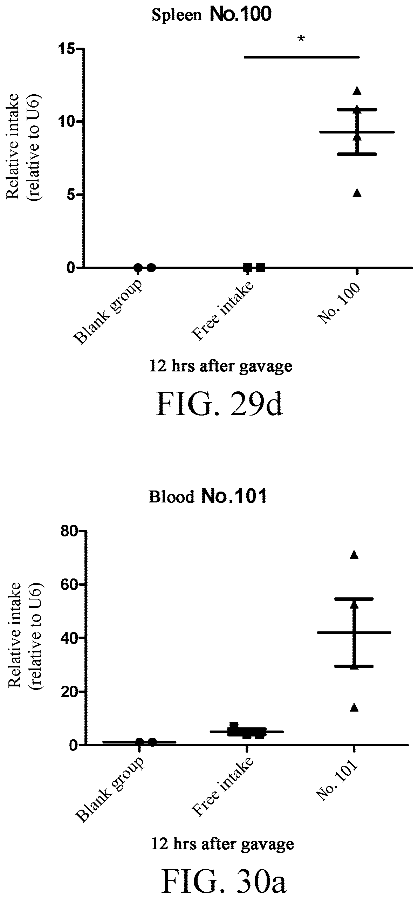

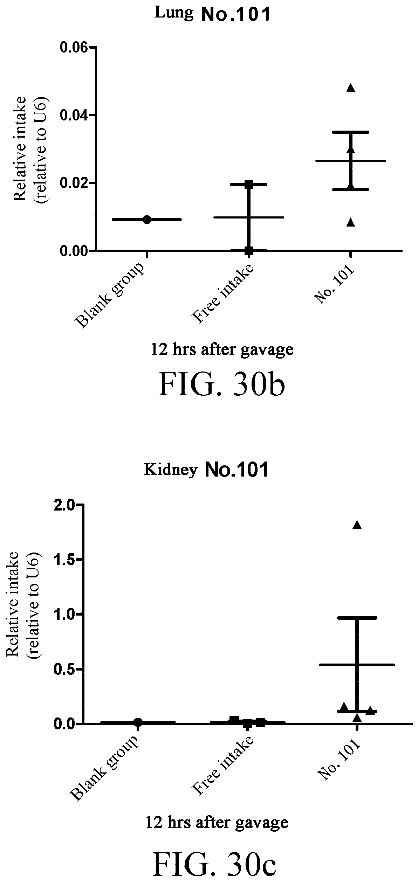

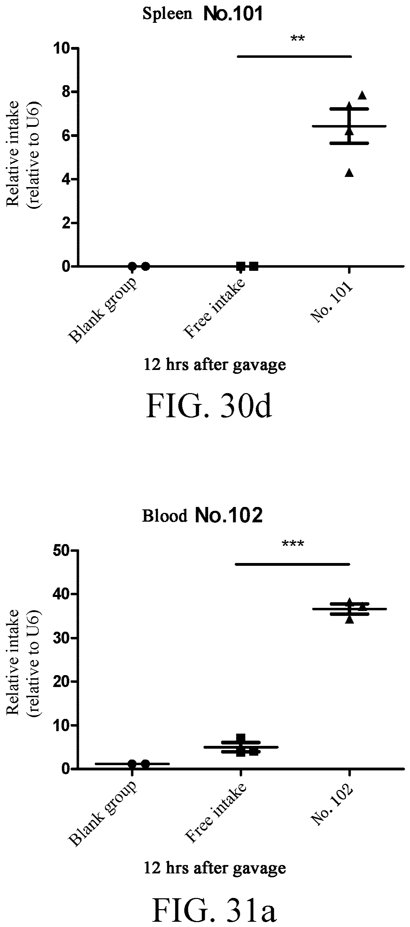

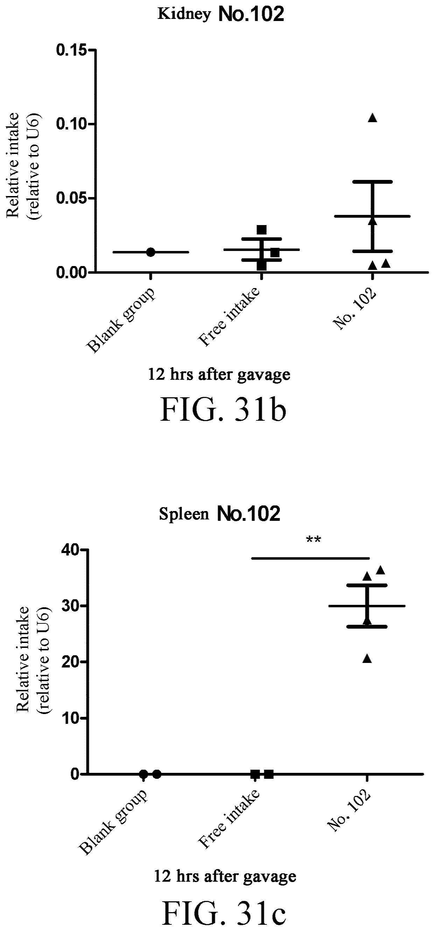

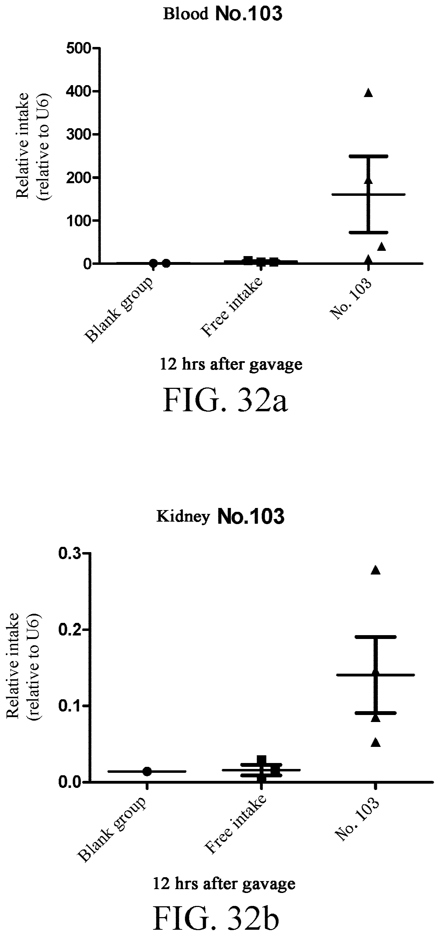

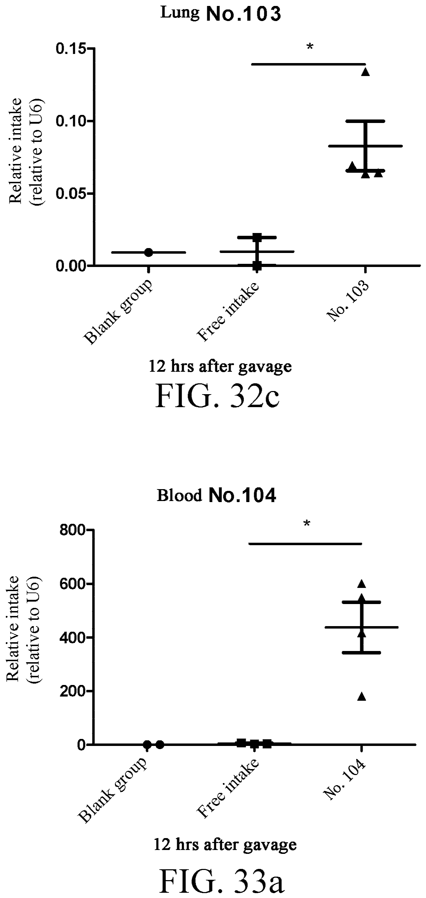

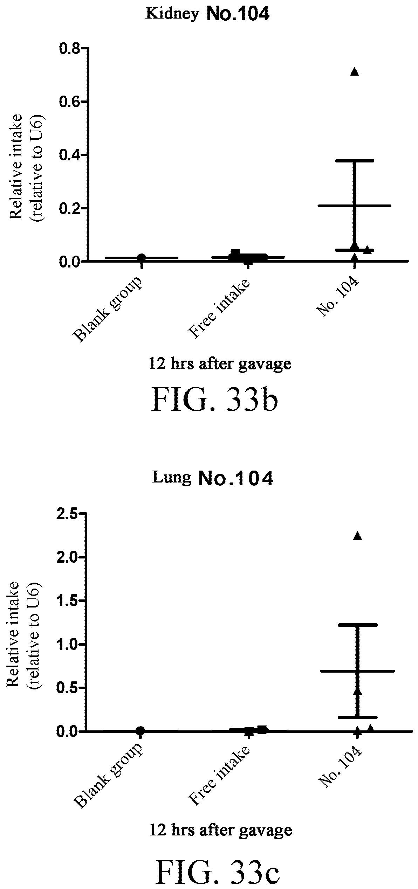

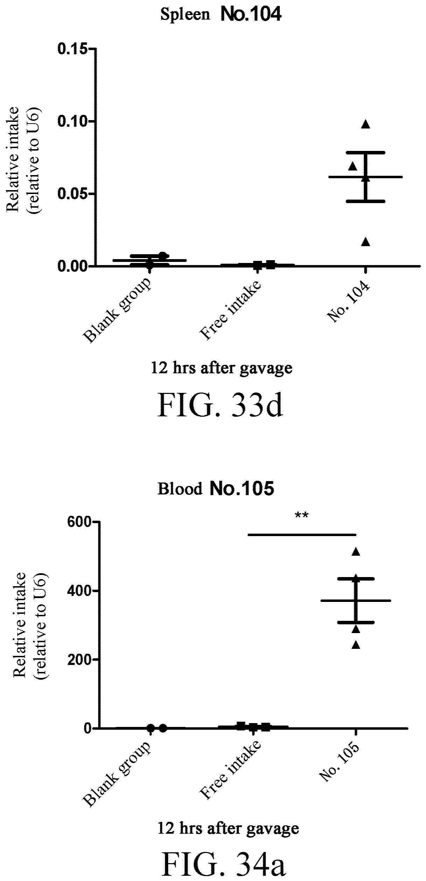

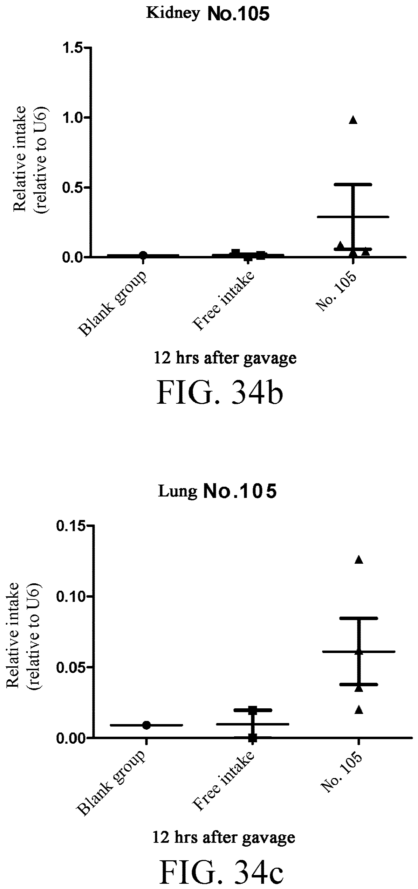

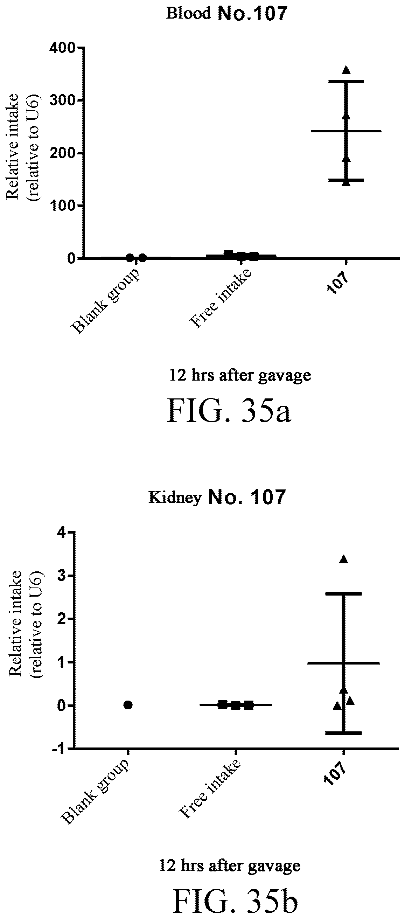

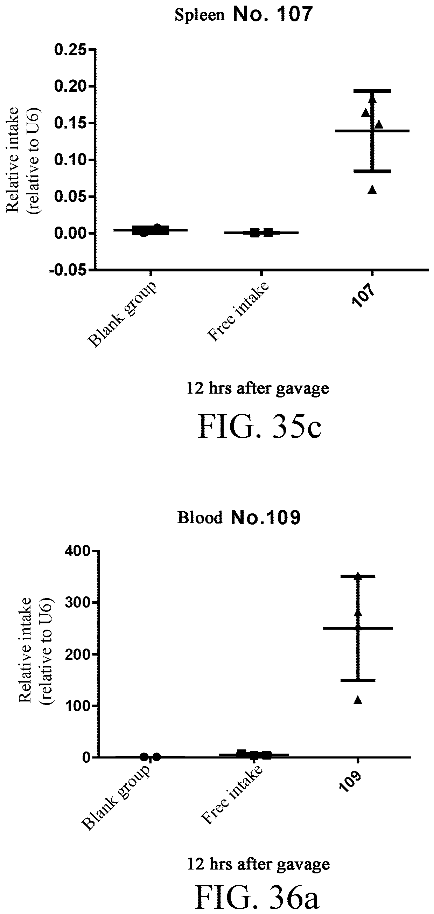

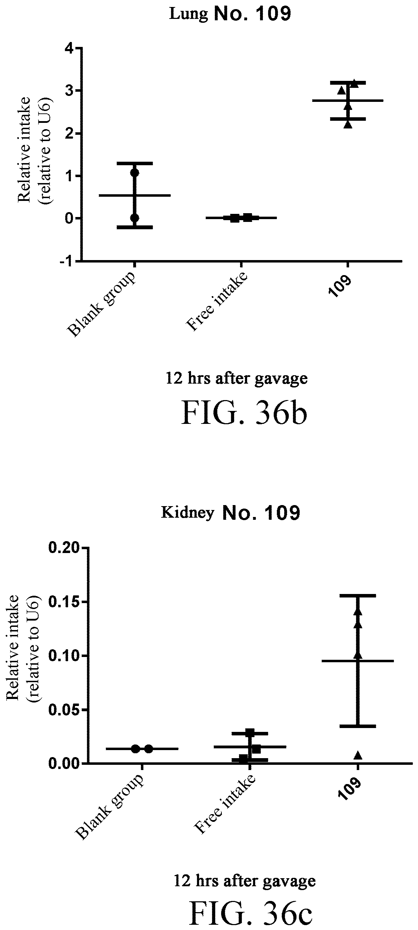

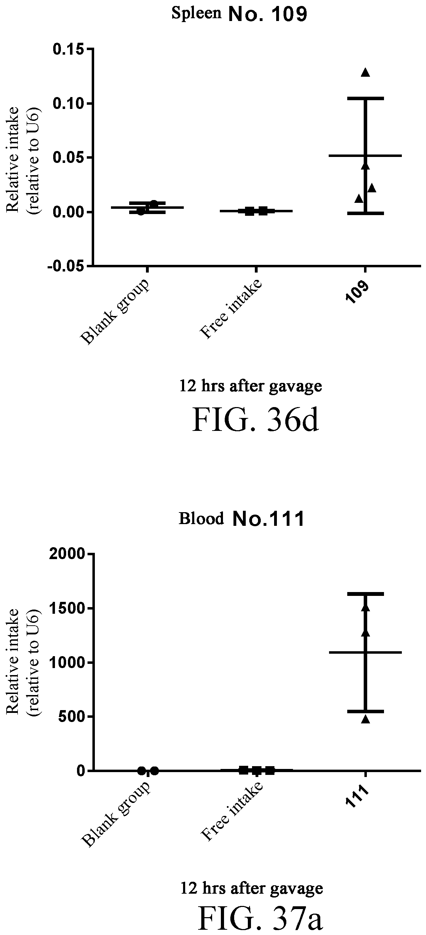

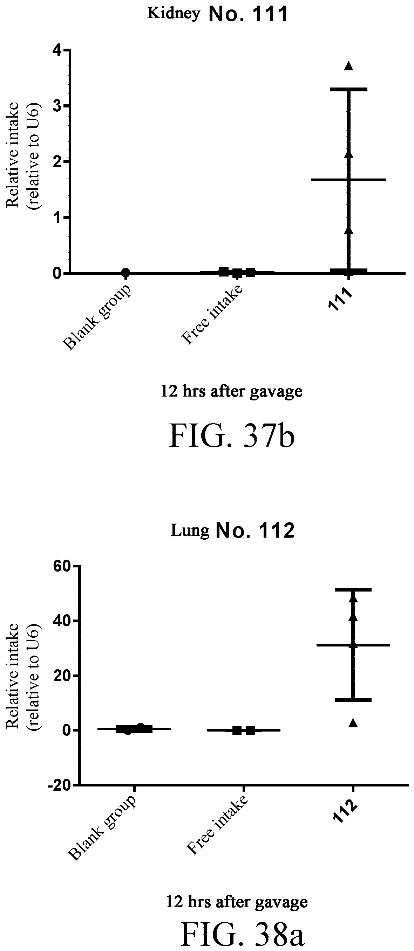

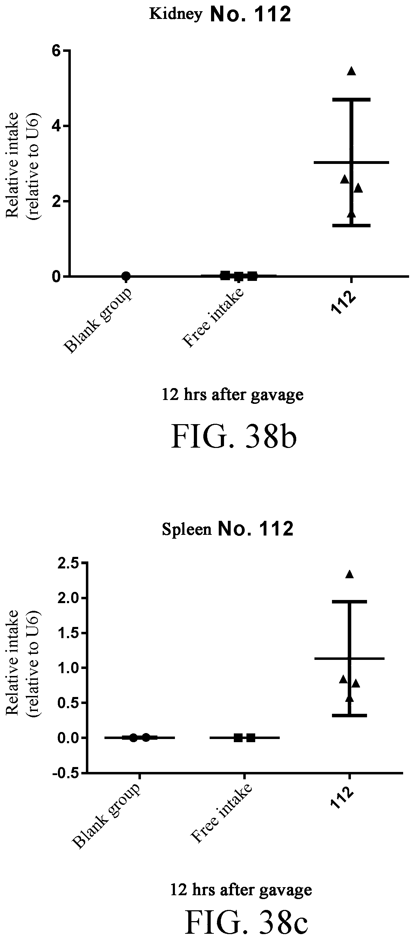

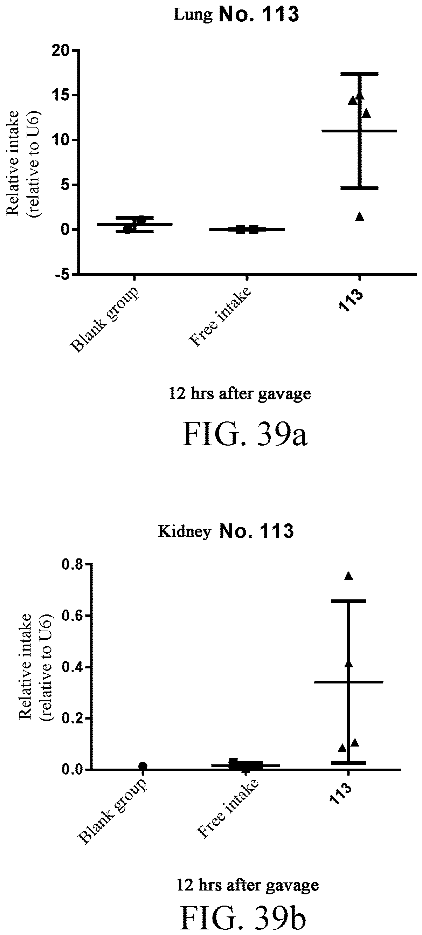

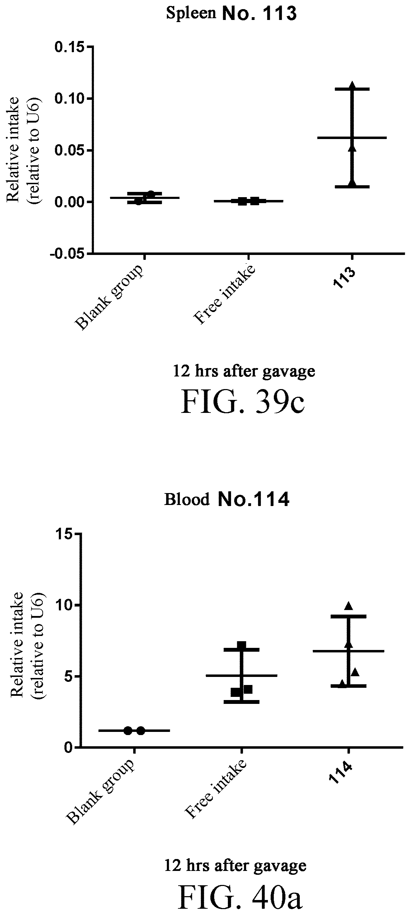

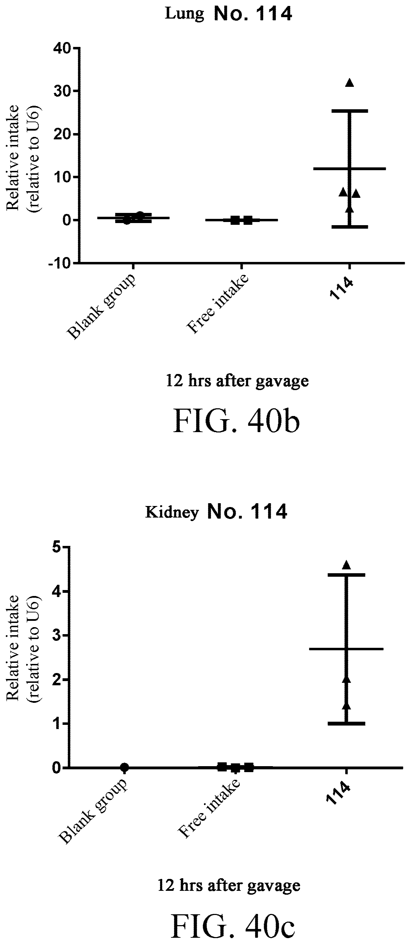

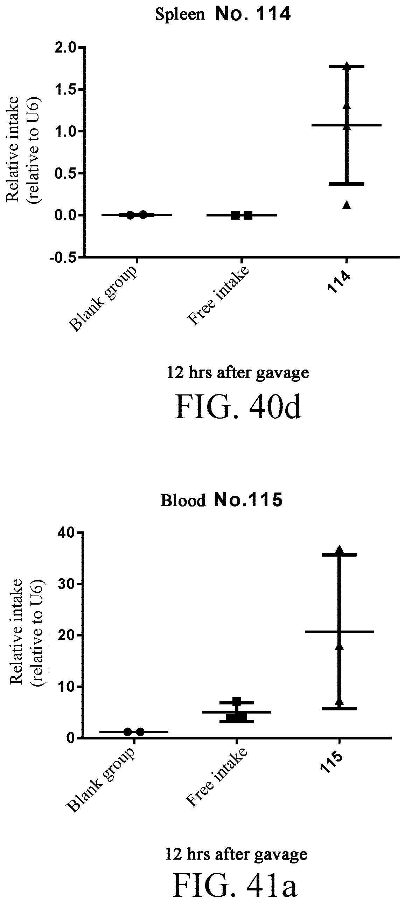

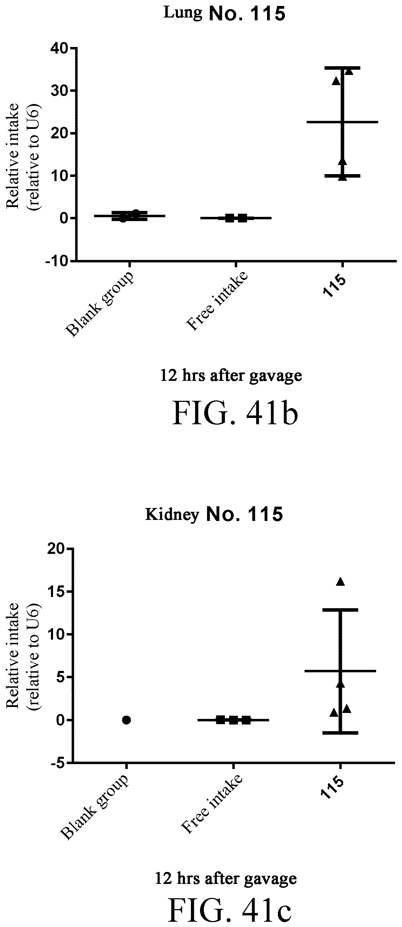

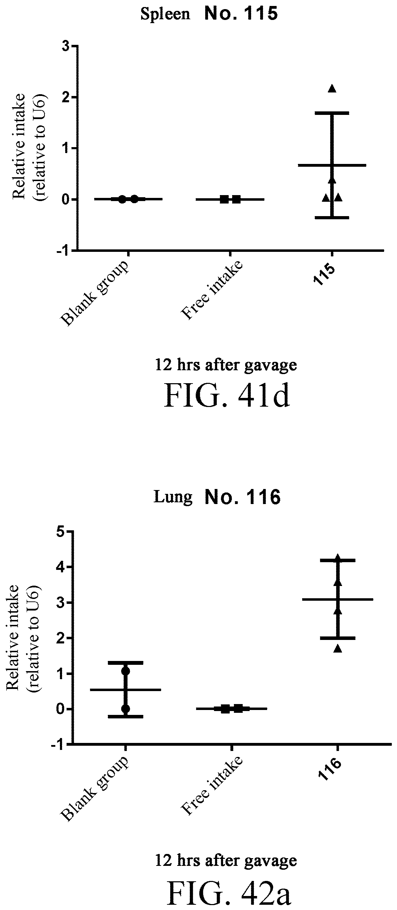

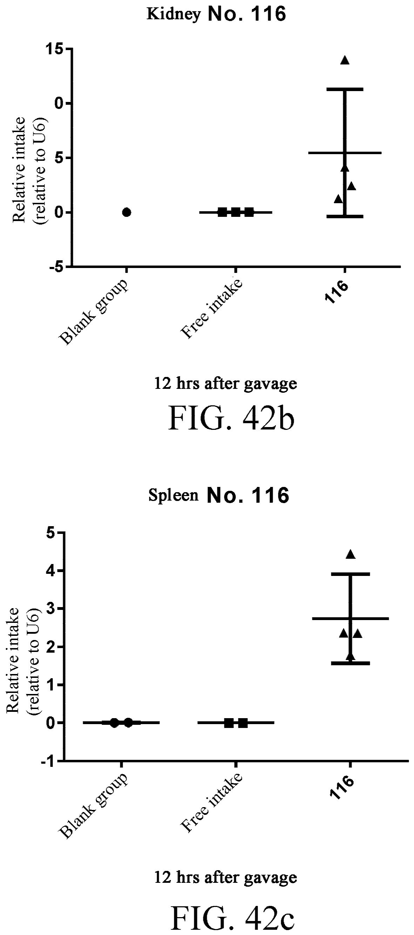

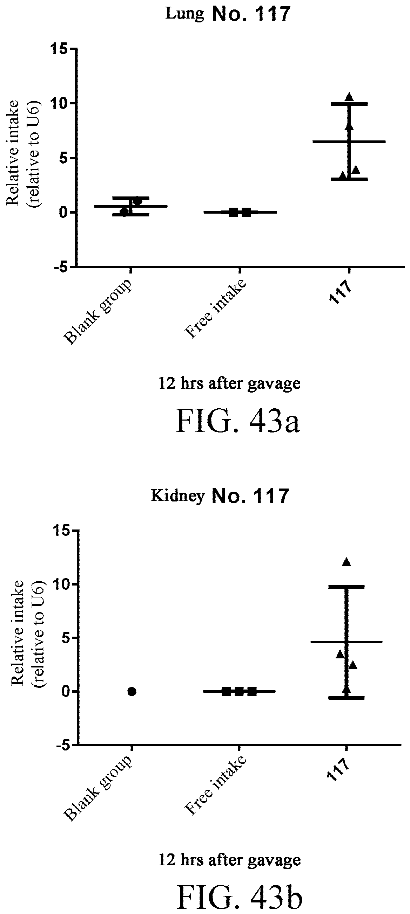

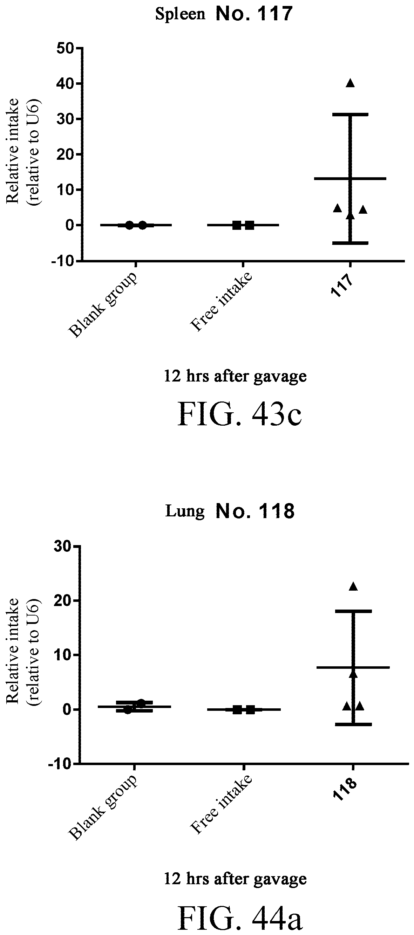

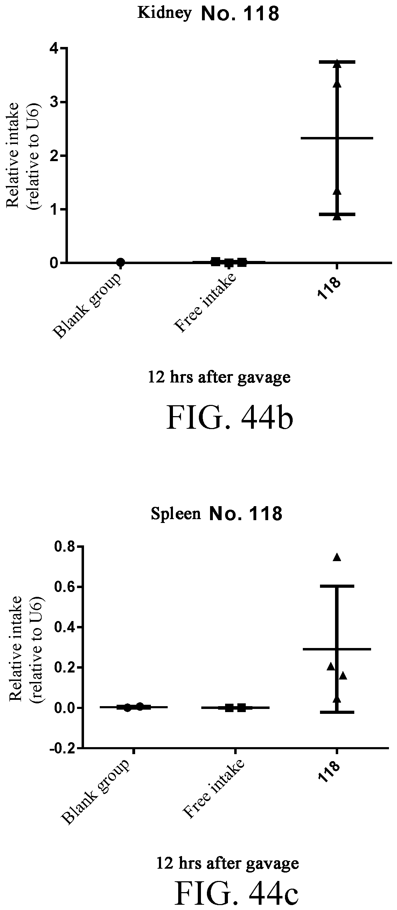

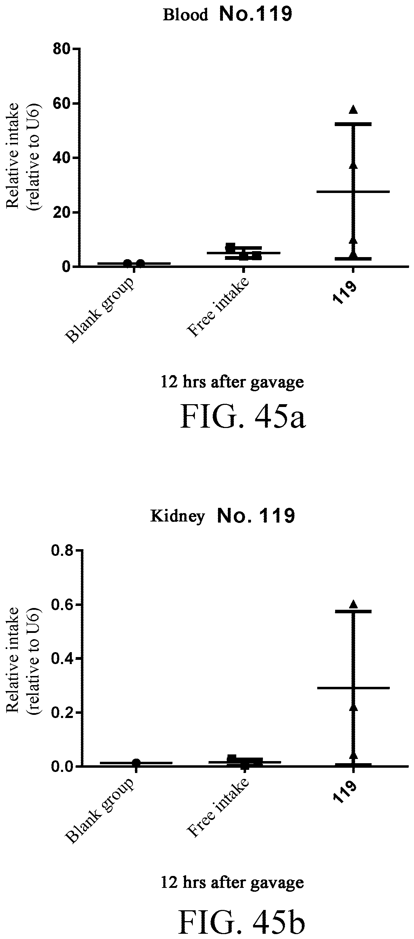

Figs. 1-45 respectively show the results of delivering single-stranded small RNA PGY-sRNA-26 into different target tissues in the blank group, the free intake group, and treatment groups with different lipids. All data in the figures were calculated for the significance of difference by two-tailed t-test. Statistically significant results are marked with an asterisk in the figures. ∗ represents P<0.05, ^{∗∗} represents P<0.01, a larger number of asterisks indicates a higher significance. Due to the large individual differences, some data shows a clear trend, but there is no significant difference after calculation.

### Example 4: Pharmacokinetic study of lipid-nucleic acid mixture

### 1. Test animals:

The C57BL/6 male mice used in the experiment were purchased from Beijing Vital River Laboratory Animal Technology Co., Ltd., about 6 weeks old, and weighed 20-24g. They were kept in an SPF animal room of the Animal Center of the Institute of Basic Medical Sciences, Chinese Academy of Medical Sciences. The mice were fasted for 12 hrs before gavage.

### 2. Preparation of lipid-small RNA mixture:

The lipid-nucleic acid mixture was prepared following the method as described in Example 2. The prepared lipid-nucleic acid mixture was orally administered or injected via the tail vein at a dose of 100 µg lipid: 1 nmol single-stranded small RNA PGY-sRNA-26 per mouse. The oral dose was half that of the tail vein injection.

### 3. Detection of relative amount delivered:

### 3.1. Experimental groups:

1) Blank group: Mice (n=6) were given 500 µl of normal saline by gavage, and this group served as a blank control group;
2) Gavage group: Mice (n=15) were given 200 µl of the lipid-nucleic acid mixture prepared in step 2 by gavage.
3) Intravenous injection group: Mice (n=15) were given 100 µl of the lipid-nucleic acid mixture prepared in step 2 by injection via the tail vein.

### 3.2. RNA extraction from tissue sample:

At 3 hrs, 6 hrs, 9 hrs, 12 hrs and 24 hrs after administration in the gavage group and the intravenous injection group, 3 mice were taken and the RNA was extracted from each organ according to the method in Example 3.

### 3.3. Reverse transcription of sRNA into cDNA:

The RNA extracted from each organ was reverse transcribed into cDNA according to the method in Example 3.

### 3.4. Quantitative PCR amplification reaction:

A quantitative PCR amplification reaction was performed according to the method in Example 3.

### 3.5. The relative expression level was calculated by the 2-ΔCt method.

### 4. The experimental results are shown graphically.

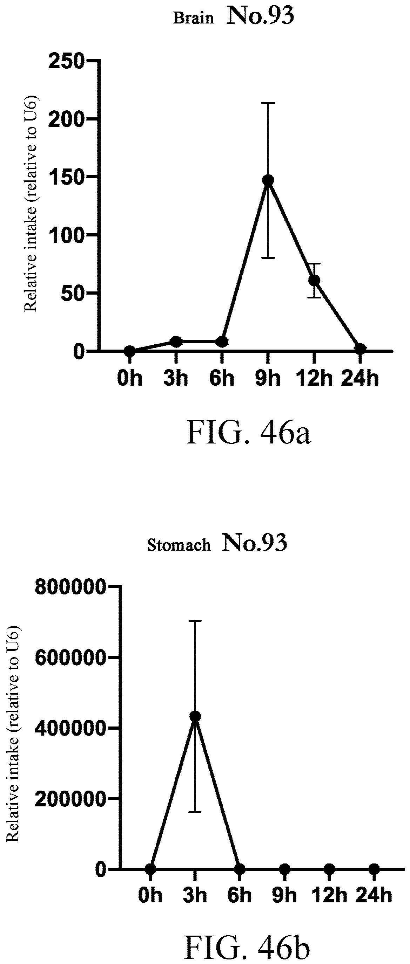

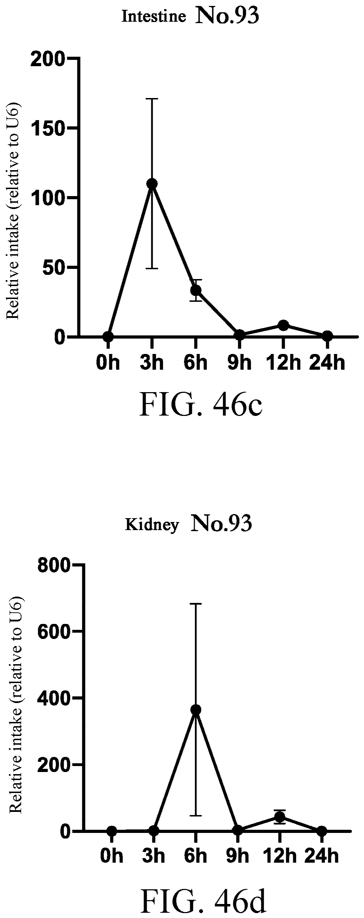

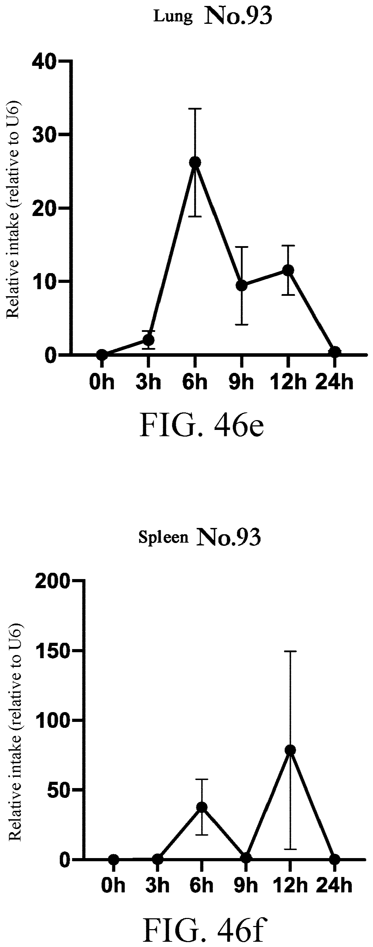

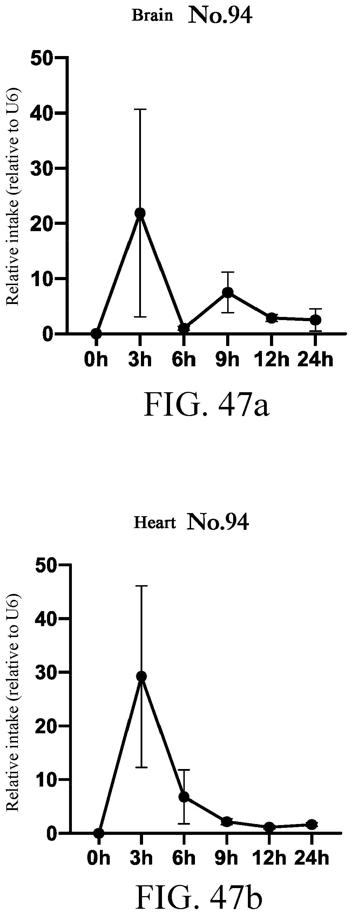

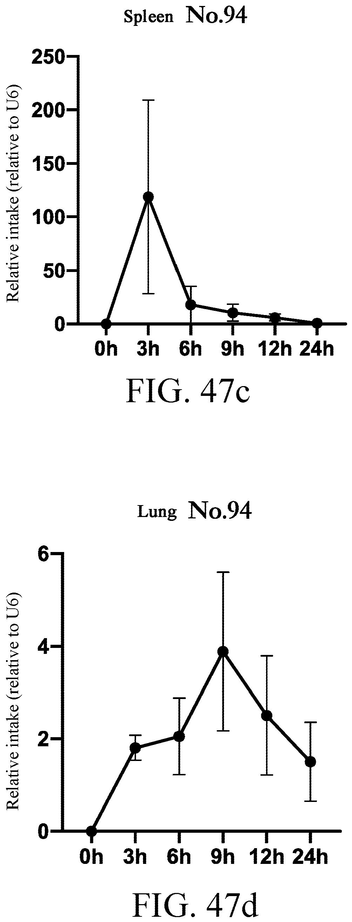

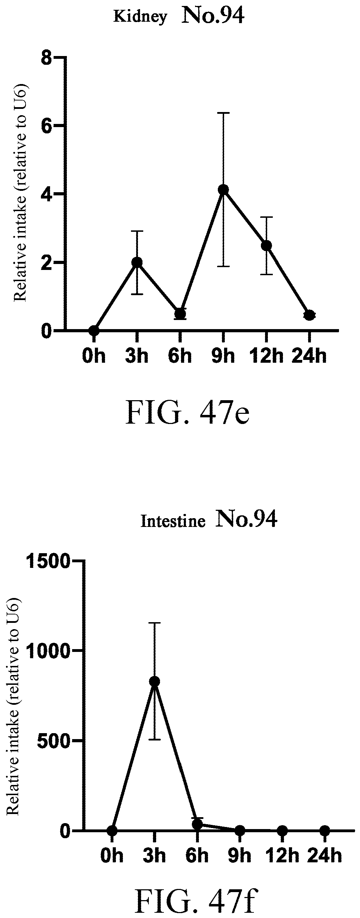

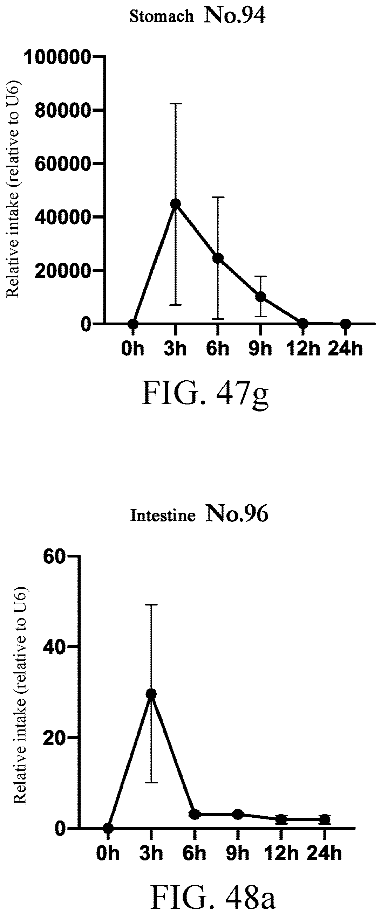

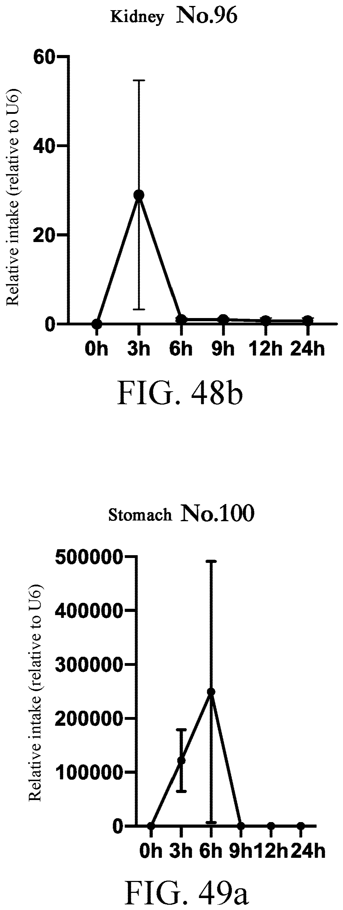

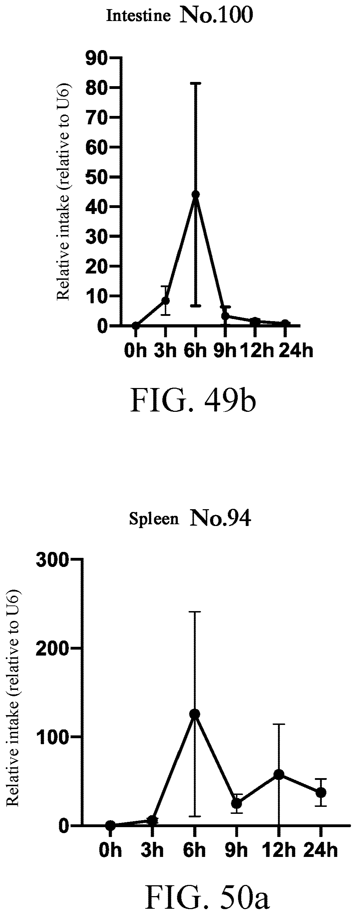

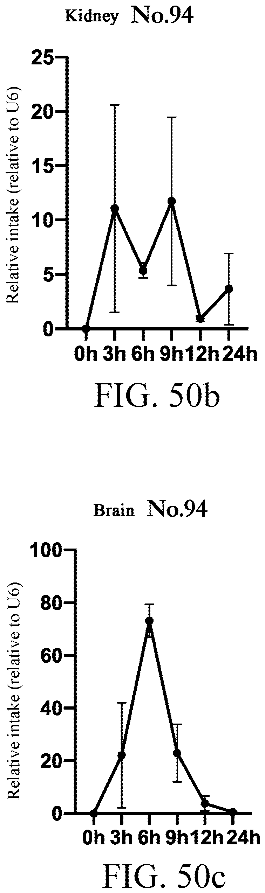

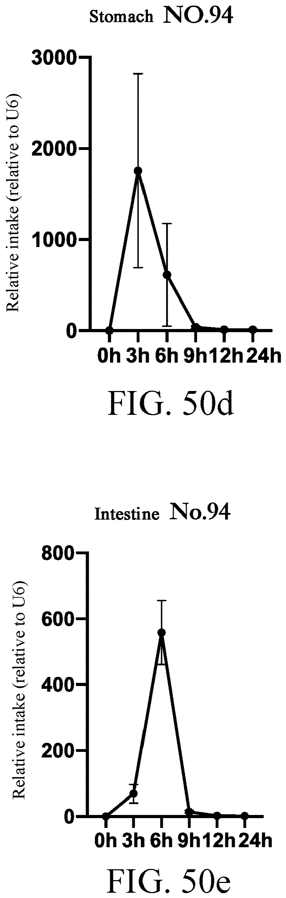

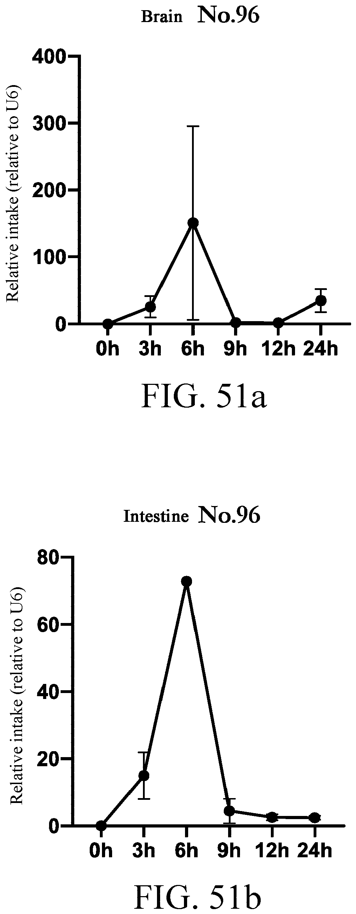

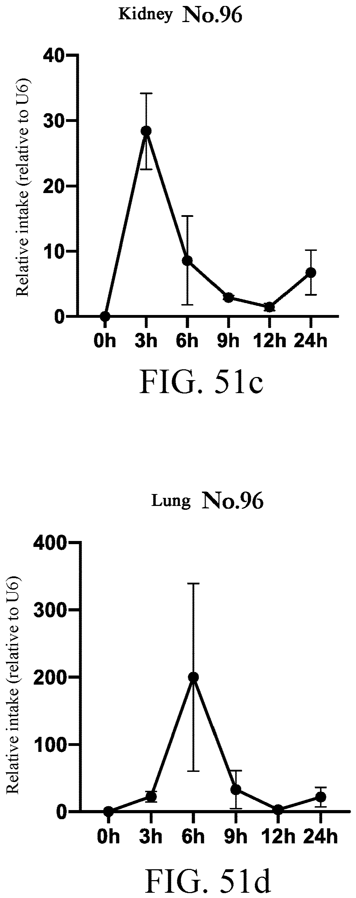

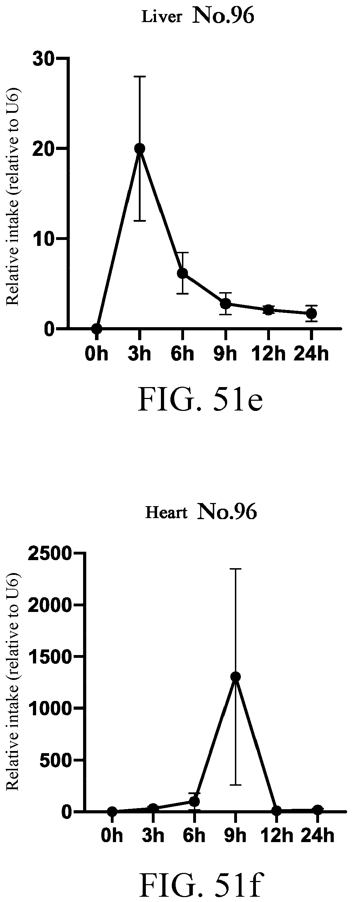

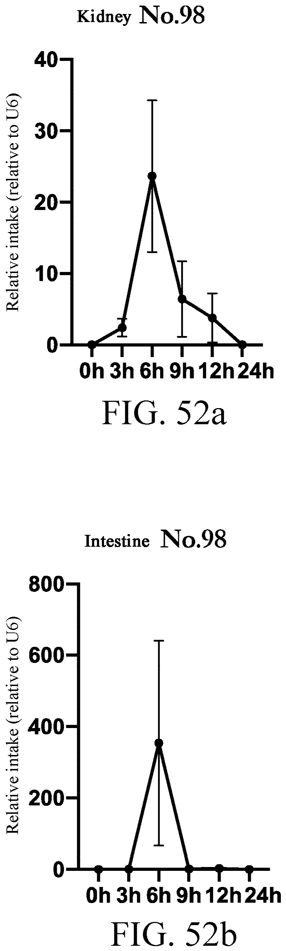

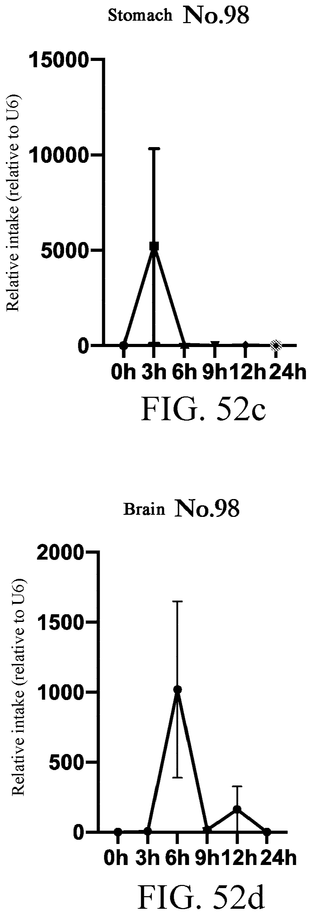

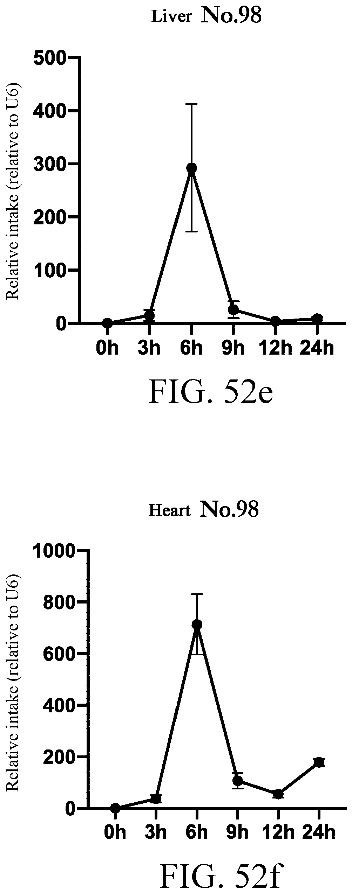

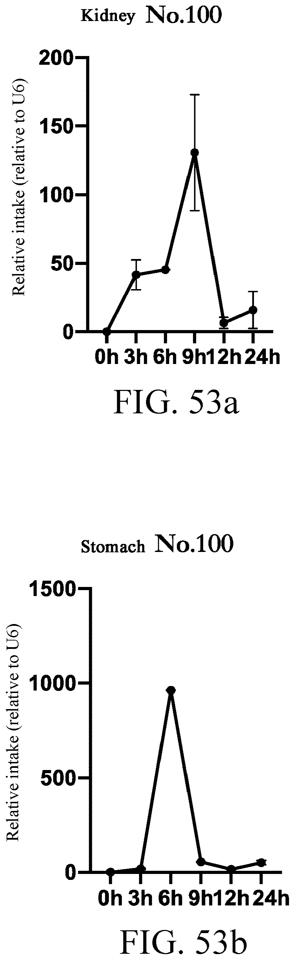

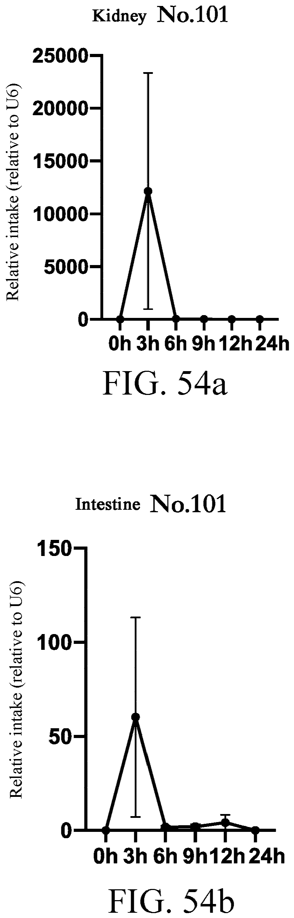

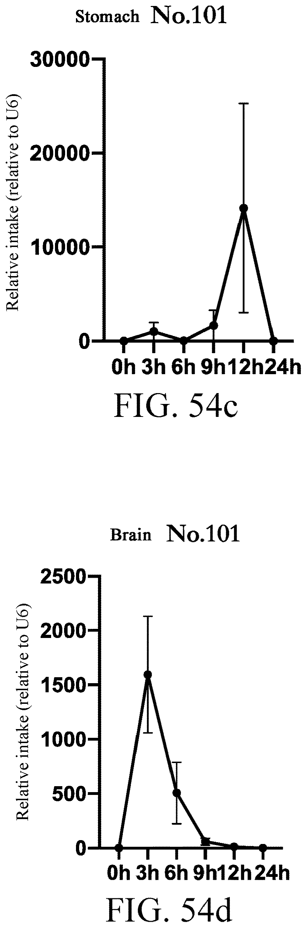

Figs. 46-49 respectively show the results of delivering single-stranded small RNA PGY-sRNA-26 into different target tissues at various time points by gavage using lipids 93, 94, 96, and 100. Figs. 50-54 respectively show the results of delivering single-stranded small RNA PGY-sRNA-26 into different target tissues at various time points by injection via the tail vein using lipids 94, 96, 98, 100, and 101.

### Conclusions:

Lipids 72, 73, 74, 75, 76, 77, 78, 79, 80 and 81 can effectively orally deliver sRNA single-chain nucleic acid into the blood, and lung, kidney, spleen, and heart tissues of mice.

Lipids 82, 83, 84, 85, 86, 87, 88, 89, 90, 91, 92, 93, 94, 95, 96, 97, 98, 99, 100, 101, 102, 103, 104, 105, 107, 109, 111, 112, 113, 114, 115, 116, 117, 118 and 119 can effectively orally deliver sRNA single-chain nucleic acid into the blood, and lung, kidney, spleen, heart, and intestinal tissue of mice.

The experimental results with significant differences compared to the control are as follows:
1. Lipid 72 can significantly efficiently orally deliver sRNA single-stranded nucleic acid into the lung tissue of mice.
2. Lipid 73 can significantly efficiently orally deliver sRNA single-stranded nucleic acid into the heart tissue of mice.
3. Lipid 75 can significantly efficiently orally deliver sRNA single-stranded nucleic acid into the blood of mice.
4. Lipid 76 can significantly efficiently orally deliver sRNA single-stranded nucleic acid into the lung and spleen tissues of mice.
5. Lipid 77 can significantly efficiently orally deliver sRNA single-stranded nucleic acid into the spleen tissue of mice.
6. Lipid 79 can significantly efficiently orally deliver sRNA single-stranded nucleic acid into the spleen tissue of mice.
7. Lipid 80 can significantly efficiently orally deliver sRNA single-stranded nucleic acid into the spleen and heart tissues of mice.
8. Lipid 81 can significantly efficiently orally deliver sRNA single-stranded nucleic acid into the spleen tissue of mice.
9. Lipid 83 can significantly efficiently orally deliver sRNA single-stranded nucleic acid into the intestinal tissue of mice.
10. Lipid 88 can significantly efficiently orally deliver sRNA single-stranded nucleic acid into the kidney tissue of mice.
11. Lipid 91 can significantly efficiently orally deliver sRNA single-stranded nucleic acid into the kidney tissue of mice.
12. Lipid 92 can significantly efficiently orally deliver sRNA single-stranded nucleic acid into the lung tissue of mice.
13. Lipid 93 can significantly efficiently orally deliver sRNA single-stranded nucleic acid into the kidney tissue of mice.
14. Lipid 94 can significantly efficiently orally deliver sRNA single-stranded nucleic acid into the lung and kidney tissues of mice.
15. Lipid 95 can significantly efficiently orally deliver sRNA single-stranded nucleic acid into the lung and spleen tissues of mice.
16. Lipid 96 can significantly efficiently orally deliver sRNA single-stranded nucleic acid into the kidney and spleen tissues of mice.
17. Lipid 97 can significantly efficiently orally deliver sRNA single-stranded nucleic acid into the lung and kidney tissues of mice.
18. Lipid 98 can significantly efficiently orally deliver sRNA single-stranded nucleic acid into the lung, kidney, and spleen tissues of mice.
19. Lipid 99 can significantly efficiently orally deliver sRNA single-stranded nucleic acid into the spleen tissue of mice.
20. Lipid 100 can significantly efficiently orally deliver sRNA single-stranded nucleic acid into the spleen tissue of mice.
21. Lipid 101 can significantly efficiently orally deliver sRNA single-stranded nucleic acid into the spleen tissue of mice.
22. Lipid 102 can significantly efficiently orally deliver sRNA single-stranded nucleic acid into the blood and spleen tissue of mice.
23. Lipid 103 can significantly efficiently orally deliver sRNA single-stranded nucleic acid into the lung tissue of mice.
24. Lipid 104 can significantly efficiently orally deliver sRNA single-stranded nucleic acid into the blood of mice.
25. Lipid 105 can significantly efficiently orally deliver sRNA single-stranded nucleic acid into the blood of mice.

Wherein lipids 73 and 80 can significantly efficiently orally deliver sRNA single-stranded nucleic acid into the heart tissue of mice. Lipids 72, 76, 92, 94, 95, 97, 98 and 103 can significantly efficiently orally deliver sRNA single-stranded nucleic acid into the lung tissue of mice. Lipids 88, 91, 93, 94, 96, 97 and 98 can significantly efficiently orally deliver sRNA single-stranded nucleic acid into the kidney tissue of mice. Lipids 76, 77, 79, 80, 81, 95, 96, 98, 99, 100, 101 and 102 can significantly efficiently orally deliver sRNA single-stranded nucleic acid into the spleen tissue of mice. Lipids 75, 102, 104 and 105 can significantly efficiently orally deliver sRNA single-stranded nucleic acid into the blood of mice. Lipid 83 can significantly efficiently orally deliver sRNA single-stranded nucleic acid into the intestinal tissue of mice.

Among all 6 organs in the experiments, spleen, lung, and kidney are the three organs to which the nucleic acid is most significantly delivered by different lipid monomers. The number of lipids having significant delivery performance into spleen is the greatest, followed by lung, and then kidney. The performance of lipid delivering nucleic acid into the blood is also very significant. Lipid 98 has the most significant delivery performance, can significantly deliver nucleic acid into spleen, lung, and kidney, and is expected to be used as a broad-spectrum nucleic acid delivery vehicle. Moreover, Lipids 97, 76, 80, 94, 95, 96, and 102 can significantly deliver nucleic acid into two organs.

Pharmacokinetics shows that the time when the nucleic acid delivered by the lipids into different organs reaches peak intake may be different. For example, when delivered by gavage, the nucleic acid delivered by lipids 93, 94, 96 and 100 into different organs reaches the peak intake at different time points, wherein the amount of nucleic acid delivered by lipid 93 into the intestinal tissue and gastric tissue reaches a maximum value at 3 hrs, the amount delivered into the lung tissue and kidney tissue reaches a maximum value at 6 hrs, the amount delivered into the brain tissue reaches a maximum value at 9 hrs, and the amount delivered into the spleen tissue reaches a maximum value at 12 hrs. The amount of nucleic acid delivered by lipid 94 into the heart, brain, intestine, spleen, and gastric tissues reaches a maximum value at 3 hrs, and the amount delivered into the kidney, and lung tissues reaches a maximum value at 9 hrs. The amount of nucleic acid delivered by lipid 96 into the kidney and intestinal tissues reaches a maximum value at 3 hrs. The amount of nucleic acid delivered by lipid 100 into the gastric and intestinal tissues reaches a maximum value at 6 hrs. When delivered by injection via the tail vein, the amount of nucleic acid delivered by lipid 94 into the gastric tissue reaches a maximum value at 3 hrs, the amount delivered into the spleen, brain, and intestinal tissues reaches a maximum value at 6 hrs, and the amount delivered into the kidney tissue reaches a maximum value at 9 hrs. The amount of nucleic acid delivered by lipid 96 into the kidney and liver tissues reaches a maximum value at 3 hrs, the amount delivered into the brain, intestine, and lung tissues reaches a maximum value at 6 hrs, and the amount delivered into the heart tissue reaches a maximum value at 9 hrs. The amount of nucleic acid delivered by lipid 98 into the gastric tissue reaches a maximum value at 3 hrs, the amount delivered into the kidney, intestine, brain, liver, and heart tissue reaches a maximum value at 6 hrs. The amount of nucleic acid delivered by lipid 100 into the gastric tissue reaches a maximum value at 6 hrs, and the amount delivered into the kidney tissue reaches a maximum value at 9 hrs. The amount of nucleic acid delivered by lipid 101 into the kidney, intestine, and brain tissue reaches a maximum value at 3 hrs, and the amount delivered into the gastric tissue reaches a maximum value at 12 hrs.

All documents mentioned in the present invention are cited as references in the present invention, as if each document is individually cited as a reference. Moreover, it should be understood that after reading the above teachings of the present invention, various changes or modifications can be made by those skilled in the art to the present invention, which also fall within the scope defined by the appended claims of the present invention.

## Claims

1. Use of a lipid composition in the manufacture of a reagent for delivering a nucleic acid, wherein the lipid composition comprises one or more compounds of Formula (I) or a salt, a hydrate or a solvate thereof: 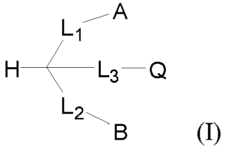 wherein
L₁ is absent, or is -CH₂-O-C(O)-, -CH₂-O- or -CR(OH)-;
L₂ is absent, or is -O-C(O)- or -NH-C(O)-;
L₃ is a linear or branched C₁₋₂₀ alkyl, a linear or branched C₁₋₂₀ alkenyl, a linear or branched C₁₋₂₀ heteroalkyl, a linear or branched C₁₋₂₀ heteroalkenyl or 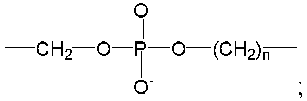
A is a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl;
B is -OH, a linear or branched C₁₋₂₀ alkyl, a linear or branched C₁₋₂₀ alkenyl, a linear or branched C₁₋₂₀ heteroalkyl or a linear or branched C₁₋₂₀ heteroalkenyl;
Q is -H, -COOH, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, -N(R)₃⁺ or 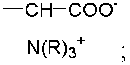
R is each independently H or a linear or branched C₁₋₂₀ alkyl; and
n is 0, 1 or 2.

2. The use according to claim 1, wherein the lipid composition is used to deliver the nucleic acid by oral administration, inhalation or injection.

3. The use according to claim 2, wherein the lipid composition is used to deliver the nucleic acid by oral administration.

4. The use according to any one of the preceding claims, wherein the delivery comprises in vitro cell delivery, or in vivo gastrointestinal delivery.

5. The use according to any one of the preceding claims, wherein the lipid composition is mixed with a nucleic acid and prepared into a lipid-nucleic acid mixture, and optionally, the lipid-nucleic acid mixture is prepared by a heating method, by a reverse evaporation method, or by direct mixing.

6. The use according to claim 5, wherein the heating method comprises mixing the lipid composition with the nucleic acid to obtain a mixture, and heating the mixture at a temperature in a range selected from the group consisting of 25°C to 100°C, 30°C to 100°C, 40°C to 100°C, 50°C to 100°C, 60°C to 100°C, 70°C to 100°C, 80°C to 100°C, 90°C to 100°C, and 95°C to 100°C.

7. The use according to claim 6, wherein the mixture is heated at a temperature selected from the group consisting of 30°C, 35°C, 37°C, 40°C, 45°C, 50°C, 55°C, 60°C, 65°C, 70°C, 75°C, 80°C, 85°C, 90°C, 95°C and 100°C.

8. The use according to any one of claims 5 to 7, wherein the mixing the lipid composition with the nucleic acid comprises adding a solution of the lipid composition in an organic solvent to an aqueous solution of the nucleic acid.

9. The use according to claim 5, wherein the reverse evaporation method comprises mixing an aqueous solution of the nucleic acid with a solution of the lipid compound in an organic solvent to obtain a mixed solution, and then removing the organic solvent in the mixed solution.

10. The use according to claim 9, wherein the organic solvent in the mixed solution is removed at a temperature in a range selected from about 25°C to about 70°C, 30°C to about 70°C, about 30°C to about 65°C, about 40°C to about 65°C, about 40°C to about 60°C, or about 50°C to about 60°C.

11. The use according to claim 9, wherein the mixing an aqueous solution of the nucleic acid with a solution of the lipid compound in an organic solvent comprises adding the aqueous solution of the nucleic acid to the solution of the lipid compound in an organic solvent.

12. The use according to any one of claims 1 to 11, wherein
L₁ is -CH₂-O-C(O)-, -CH₂-O- or -CR(OH)-;
L₂ is absent, or is -O-C(O)- or -NH-C(O)-;
L₃ is 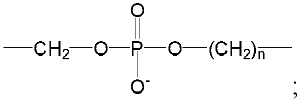 and
A, B, Q, R and n are as defined in claim 1.

13. The use according to any one of claims 1 to 11, wherein
L₁ is absent, or is -CH₂-O-C(O)- or -CH₂-O-;
L₂ is -O-C(O)-;
L₃ is a linear or branched C₁₋₂₀ alkyl or 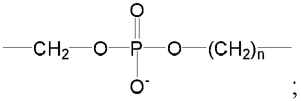
A, B, Q, R and n are as defined in claim 1.

14. The use according to any one of claims 1 to 11, wherein
L₁ is -CH₂-O-C(O)-;
L₂ is absent or is -O-C(O)-;
L₃ is 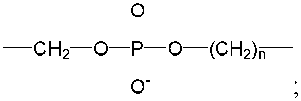 and
A, B, Q, R and n are as defined in claim 1.

15. The use according to any one of claims 1 to 11, wherein
L₁ is -CH₂-O-;
L₂ is absent or is -O-C(O)-;
L₃ is 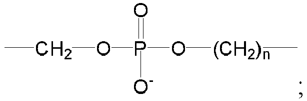 and
A, B, Q, R and n are as defined in claim 1.

16. The use according to any one of claims 1 to 11, wherein
L₁ is -CH₂-O-C(O)- or -CH₂-O-;
L₂ is absent;
L₃ is 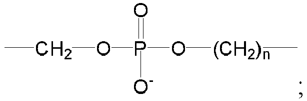 and
A, B, Q, R and n are as defined in claim 1.

17. The use according to any one of claims 1 to 11, wherein
L₁ is absent;
L₂ is -O-C(O)-;
L₃ is a linear or branched C₁₋₂₀ alkyl;
A is a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
B is -OH, a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
Q is -H, -COOH, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, -N(R)₃⁺ or 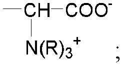
R is each independently H or a linear or branched C₁₋₂₀ alkyl; and
n is 0, 1 or 2;

18. The use according to any one of claims 1 to 11, wherein
L₁ is -CH₂-O-C(O)-;
L₂ is absent;
L₃ is 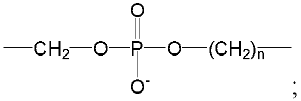
A is a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
B is -OH, a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
Q is -H, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, -N(R)₃⁺ or 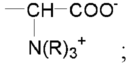
R is each independently H or a linear or branched C₁₋₂₀ alkyl; and
n is 0, 1 or 2;

19. The use according to any one of claims 1 to 11, wherein
L₁ is -CH₂-O-;
L₂ is absent;
L₃ is 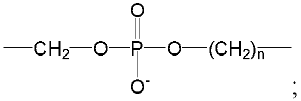
A is a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
B is -OH, a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
Q is -H, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, C₃₋₂₀ cycloalkenyl substituted by hydroxyl, -N(R)₃⁺ or 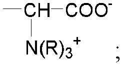
R is each independently H or a linear or branched C₁₋₂₀ alkyl; and
n is 0, 1 or 2;

20. The use according to any one of claims 1 to 11, wherein
L₁ is -CH₂-O-;
L₂ is -O-C(O)-;
L₃ is 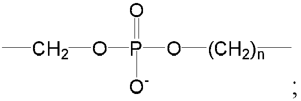
A is a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
B is -OH, a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
Q is -H, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, -N(R)₃⁺ or 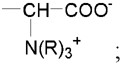
R is each independently H or a linear or branched C₁₋₂₀ alkyl; and
n is 0, 1 or 2;

21. The use according to any one of claims 1 to 11, wherein
L₁ is -CH₂-O-C(O)-;
L₂ is -O-C(O)-;
L₃ is 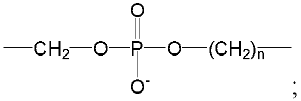
A is a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
B is a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
Q is -H, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, -N(R)₃⁺ or 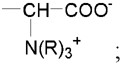
R is each independently H or a linear or branched C₁₋₂₀ alkyl; and
n is 0, 1 or 2;

22. The use according to any one of claims 1 to 11, wherein
L₁ is -CH₂-O-C(O)-;
L₂ is -O-C(O)-;
L₃ is 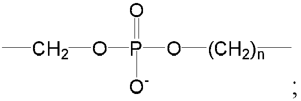
A is a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
B is -OH, a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
Q is -H, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, -N(R)₃⁺ or 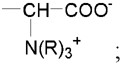
R is each independently H or a linear or branched C₁₋₂₀ alkyl; and
n is 0, 1 or 2;

23. The use according to any one of claims 1 to 11, wherein
L₁ is -CR(OH)-;
L₂ is -NH-C(O)-;
L₃ is 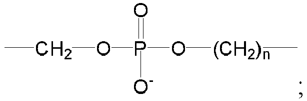
A is a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
B is -OH, a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
Q is -H, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, -N(R)₃⁺ or 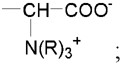
R is each independently H or a linear or branched C₁₋₂₀ alkyl; and
n is 0, 1 or 2;

24. The use according to any one of the preceding claims, wherein the compound has the following Formula (II): 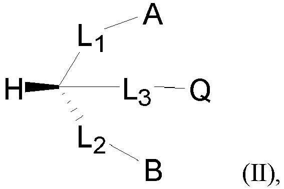 wherein L₁, L₂, L₃, A, B and Q are as defined in any one of the preceding claims.

25. The use according to any one of the preceding claims, wherein A is a linear or branched C₁₋₂₀ alkyl, or a linear or branched C₁₋₂₀ alkenyl.

26. The use according to any one of the preceding claims, wherein A is a linear or branched C₅₋₂₀ alkyl, or a linear or branched C₅₋₂₀ alkenyl.

27. The use according to any one of the preceding claims, wherein A is a linear or branched C₅₋₂₀ alkyl.

28. The use according to any one of the preceding claims, wherein A is a linear C₅₋₂₀ alkenyl having 1 to 5 double bonds.

29. The use according to any one of the preceding claims, wherein B is -OH, a linear or branched C₁₋₂₀ alkyl, or a linear or branched C₁₋₂₀ alkenyl.

30. The use according to any one of the preceding claims, wherein B is -OH, a linear or branched C₅₋₂₀ alkyl, or a linear or branched C₅₋₂₀ alkenyl.

31. The use according to any one of the preceding claims, wherein B is -OH.

32. The use according to any one of the preceding claims, wherein B is a linear or branched C₅₋₂₀ alkyl.

33. The use according to any one of the preceding claims, wherein B is a linear C₅₋₂₀ alkyl.

34. The use according to any one of the preceding claims, wherein B is a linear or branched C₅₋₂₀ alkenyl.

35. The use according to any one of the preceding claims, wherein B is a linear C₅₋₂₀ alkenyl having 1 to 5 double bonds.

36. The use according to claim 28 or 35, wherein the double bond is in a Z configuration.

37. The use according to claim 28 or 35, wherein the double bond in the linear C₅₋₂₀ alkenyl is located at a position selected from: position C1-C2, position C2-C3, position C3-C4, position C4-C5, position C5-C6, position C6-C7, position C7-C8, position C8-C9, position C9-C10, position C10-C11, position C12-C13, position C13-C14 or a combination thereof.

38. The use according to any one of the preceding claims, wherein Q is -H, -COOH, a linear or branched C₁₋₂₀ alkyl substituted by hydroxyl, a C₃₋₂₀ cycloalkyl substituted by hydroxyl, -N(R)₃⁺ or

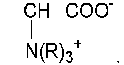

39. The use according to any one of the preceding claims, wherein Q is a linear or branched C₁₋₂₀ alkyl substituted by hydroxyl.

40. The use according to any one of the preceding claims, wherein Q is a linear or branched C₁₋₁₀ alkyl substituted by hydroxyl.

41. The use according to any one of the preceding claims, wherein Q is a linear C₁₋₅ alkyl substituted by hydroxyl.

42. The use according to any one of the preceding claims, wherein Q is -CH(OH)-CH₂OH.

43. The use according to any one of the preceding claims, wherein Q is a C₃₋₂₀ cycloalkyl substituted by hydroxyl.

44. The use according to any one of the preceding claims, wherein Q is a C₃₋₁₀ cycloalkyl substituted by hydroxyl.

45. The use according to any one of the preceding claims, wherein Q is

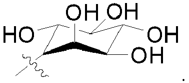

46. The use according to any one of the preceding claims, wherein Q is -N(R)₃⁺, and R is each independently H or a linear C₁₋₁₀ alkyl.

47. The use according to any one of the preceding claims, wherein Q is -N(CH₃)₃⁺.

48. The use according to any one of the preceding claims, wherein Q is 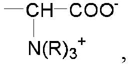 and R is each independently H or a linear C₁₋₁₀ alkyl.

49. The use according to any one of the preceding claims, wherein Q is 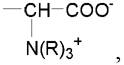 and R is H.

50. The use according to any one of claims 1 to 11, wherein the lipid composition comprises a compound having the following Formula (Ia): 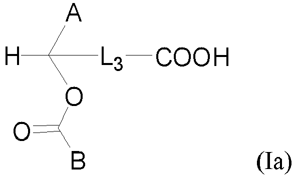 wherein
L₃ is a linear or branched C₁₋₂₀ alkyl;
A is a linear or branched C₁₋₂₀ alkyl; and
B is a linear or branched C₁₋₂₀ alkyl.

51. The use according to any one of claims 1 to 11, wherein the lipid composition comprises a compound having the following Formula (Ib): 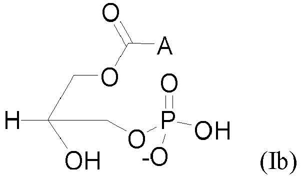 wherein A is a linear or branched C₁₋₂₀ alkyl.

52. The use according to any one of claims 1 to 11, wherein the lipid composition comprises a compound having the following Formula (Ic): 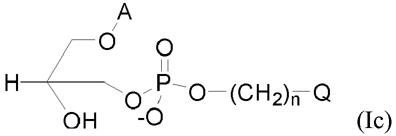 wherein
A is a linear or branched C₁₋₂₀ alkyl;
Q is -N(R)₃⁺; and
R is each independently a linear or branched C₁₋₂₀ alkyl.

53. The use according to any one of claims 1 to 11, wherein the composition comprises a compound having the following Formula (Id): 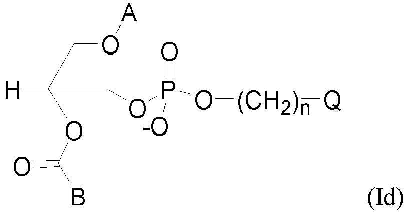 wherein
A is a linear or branched C₁₋₂₀ alkyl;
B is a linear or branched C₁₋₂₀ alkyl, or a linear or branched C₁₋₂₀ alkenyl;
Q is -N(R)₃⁺; and
R is each independently a linear or branched C₁₋₂₀ alkyl.

54. The use according to any one of claims 1 to 11, wherein the lipid composition comprises a compound having the following Formula (Ie): 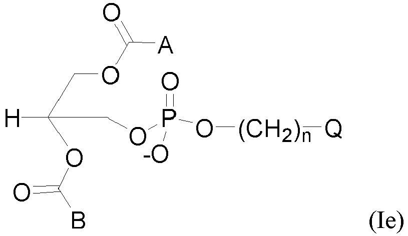 wherein
A is a linear or branched C₁₋₂₀ alkyl, or a linear or branched C₁₋₂₀ alkenyl;
B is a linear or branched C₁₋₂₀ alkenyl;
Q is a linear or branched C₁₋₂₀ alkenyl substituted by hydroxyl, a C₃₋₂₀ cycloalkyl substituted by hydroxyl, or 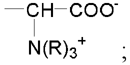 and
R is each independently a linear or branched C₁₋₂₀ alkyl.

55. The use according to any one of claims 1 to 11, wherein the lipid composition comprises a compound having the following Formula (If): 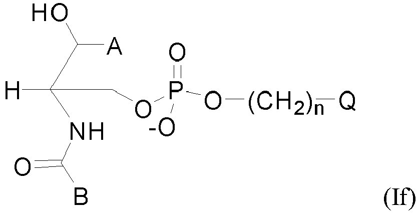 wherein
A is a linear or branched C₁₋₂₀ alkenyl;
B is a linear or branched C₁₋₂₀ alkyl, or a linear or branched C₁₋₂₀ alkenyl;
Q is -N(R)₃⁺; and
R is each independently a linear or branched C₁₋₂₀ alkyl.

56. The use according to any one of claims 1 to 11, wherein the lipid composition comprises one or more compounds selected from the group consisting of:
| Lipid Compound No. | Chemical name |
|---|---|
| 106 | 9-(palmitoyloxy)octadecanoic acid |
| 96 | 1-palmitoyl-2-hydroxy-sn-glycero-3-phosphate (sodium salt) |
| 93 | 1-O-hexadecyl-2-hydroxy-sn-glycero-3-phosphocholine |
| 94 | 1-O-octadecyl-2-hydroxy-sn-glycero-3-phosphocholine |
| 84 | β-acetyl-γ-O-hexadecyl-L-α-phosphatidylcholine hydrate |
| 85 | β-acetyl-γ-O- alkyl-L-α-phosphatidylcholine |
| 108 | 1-O-hexadecyl-2-arachidonoyl-sn-glycero-3-phosphocholine |
| 81 | 1,2-dioleoyl-sn-glycero-3-phosphatidylglycerol sodium salt |
| 88 | 1-palmitoyl-2-oleoyl-sn-glycero-3-phospho-(1'-rac-glycerol) (sodium salt) |
| 89 | 1-palmitoyl-2-oleoyl-sn-glycero-3-phosphoinositol (ammonium salt) |
| 109 | 1 ,2-di palmitoyl-sn-glycero-3 -phospho-(1 '-myo-inositol) (ammonium salt) |
| 110 | 1-palmitoyl-2-arachidonoyl-sn-glycero-3-phospho-L-serine (sodium salt) |
| 103 | N-palmitoleoyl-D-erythro-sphingosylphosphocholine |
| 104 | N-stearoyl-D-erythro-sphingosylphosphocholine |
| 105 | N-palmitoyl-D-erythro-sphingosylphosphocholine |

57. Use of a lipid composition in the manufacture of a reagent for delivering a nucleic acid, wherein the lipid composition comprises one or more compounds selected from the group consisting of:
| Lipid Compound No. | Chemical name |
|---|---|
| 72 | 1 ,2-dioleoyl-sn -glycero-3 -phosphatidylethanolamine |
| 73 | Trilinolenin |
| 74 | 1,2-palmitolein-3 -olein |
| 75 | 1,2-palmitolein-3-palmitin |
| 76 | 1,3 -palmitin-2-myristin |
| 77 | 1,2-stearin-3 -olein |
| 78 | 1,2-olein-3 -arachidin |
| 79 | 1,2-olein-3-behenin |
| 80 | 1,2-myristin-3 -palmitin |
| 82 | Q-10, Ubiquinone 50, Ubiquinone 10 |
| 83 | 1 -palmitoyl-2-hydroxy-sn-glycero-3 -phosphoethanolamine |
| 86 | 1-palmitoyl-2-oleoyl-sn-glycero-3-phosphocholine, powder |
| 87 | 1 -stearoyl -2-oleoyl-sn-glycero-3 -phosphocholine |
| 90 | N-lignoceroyl-D-erythro-sphingosine |
| 91 | N-nervonoyl-D-erythro-sphingosine |
| 92 | 1-arachidonyl-2-hydroxy-sn-glycero-3 -phosphocholine |
| 95 | 1-oleoyl-2-hydroxy-sn-glycero-3 -phosphocholine |
| 97 | D-erythro-sphingosine (C22 group) |
| 98 | D-erythro-sphingosine |
| 99 | D-erythro-sphingosine (C14 group) |
| 100 | D-erythro-sphingosine (C16 group) |
| 101 | D-erythro-sphingosine (C20 group) |
| 102 | D-erythro-sphingosine |
| 107 | 1 ,2-dilinoleoyl-sn -glycero-3 -phosphocholine |
| 111 | 1 -palmitin-3 -stearin |
| 112 | Trieicosanoin |
| 113 | 1 -palmitoyl-2-hydroxy-sn-glycero-3 -phosphatidylcholine |
| 114 | 1-octadecanoyl-2-hydroxy-sn-glycero-3-phosphocholine |
| 115 | 1-heptadecenoyl-sn-glycero-3-phosphoethanolamine |
| 116 | 1 -(9Z-octadecenoyl)-sn-glycero-3 -phosphoethanolamine |
| 117 | 1,2-bis-(9Z-octadecenoyl)-sn-glycero-3-phosphocholine |
| 118 | 1-palmitoyl-2-oleoyl-sn-glycero-3-phosphatidylcholine |
| 119 | 1 ,2-dihexadecanoyl-sn -glycero-3 -phosphocholine |
or a salt, a hydrate or a solvate thereof.

58. The use according to any one of the preceding claims, wherein the salt is a pharmaceutically acceptable salt.

59. The use according to any one of the preceding claims, wherein the compound is derived from a traditional Chinese medicine extract.

60. The use according to claim 59, wherein the traditional Chinese medicine extract is obtained by boiling and extracting the traditional Chinese medicine selected from the group consisting of decoction pieces of *Rhodiola crenulata, Taraxacum mongolicum, Andrographis paniculata* and *Lonicera japonica.*

61. The use according to claim 60, wherein the traditional Chinese medicine extract is obtained by: soaking a traditional Chinese medicine in water, sequentially performing intense heating and slow heating, concentrating the heated Chinese medicine soup, sequentially adding chloroform-methanol, chloroform and water followed by stirring, and collecting and extracting chloroform layer.

62. The use according to any one of claims 1 to 58, wherein the compound is synthetic.

63. The use according to any one of the preceding claims, wherein the nucleic acid comprises DNA or RNA; and optionally, the DNA is selected from coding DNA and noncoding DNA, and the RNA is selected from antisense nucleic acid, mRNA, lncRNA, and small RNA.

64. The use according to claim 63, wherein the nucleic acid comprises a small nucleic acid at a length of 14-32 bp, 16-28 bp or 18-24 bp.

65. The use according to any one of the preceding claims, wherein the nucleic acid is single-stranded or double-stranded.

66. The use according to any one of the preceding claims, wherein the nucleic acid has a stem-loop structure.

67. The use according to any one of the preceding claims, wherein the nucleic acid is used for treating disease.

68. The use according to claim 67, wherein the nucleic acid is used for treating cancer, inflammation, fibrotic disease, autoimmune disease or autoinflammatory disease, bacterial infection, behavioral and psychiatric disorder, hematological disease, chromosomal disorder, congenital and genetic disease, connective tissue disease, digestive disease, ear-nose-throat disease, endocrine disease, environmental illness, eye disease, female reproductive system disease, fungal infection, heart disease, hereditary cancer syndrome, immune system disorder, kidney and urinary tract disorder, pulmonary disease, male reproductive system disease, metabolic disorder, mouth disease, musculoskeletal disorder, myelodysplastic syndrome, newborn screening, nutritional disease, parasitic disease, rare cancer, rare disease, skin disease and viral infection.

69. The use according to claim 67, wherein the nucleic acid is used for treating hepatocellular carcinoma, corneal neovascularization, recurrent or refractory anaplastic astrocytoma (WHO grade III) or secondary glioblastoma (WHO grade IV), advanced squamous cell lung cancer, acromegaly, psoriasis, Duchenne muscular dystrophy, advanced non-small cell lung cancer, metastatic castration-resistant prostate cancer, cytomegalovirus retinitis, HIV infection, Hepatitis B, Hepatitis C, hyperlipoproteinemia, total knee replacement, Diabetes mellitus type II, familial amyloid polyneuropathy (FAP), wet macular degeneration (e.g., neovascular age-related macular degeneration, subfoveal neovascular age-related macular degeneration, and exudative age-related macular degeneration), hypercholesterolemia, Crohn's disease, extensive liver fibrosis, infantile spinal muscular atrophy, melanoma, neonatal coronary artery disease, mild allergic asthma, chronic lymphocytic leukemia, hypertriglyceridemia, hepatic veno-occlusive disease complicated with renal or pulmonary dysfunction after hematopoietic stem cell transplantation, and hereditary transthyretin amyloidosis.

70. A pharmaceutical composition, comprising a lipid composition and a nucleic acid, wherein the lipid composition comprises one or more compounds of Formula (I), or a pharmaceutically acceptable salt, hydrate or solvate thereof: 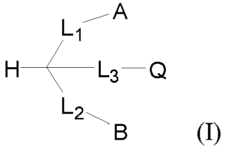 wherein
L₁ is absent, or is -CH₂-O-C(O)-, -CH₂-O- or -CR(OH)-;
L₂ is absent, or is -O-C(O)- or -NH-C(O)-;
L₃ is a linear or branched C₁₋₂₀ alkyl, a linear or branched C₁₋₂₀ alkenyl, a linear or branched C₁₋₂₀ heteroalkyl, a linear or branched C₁₋₂₀ heteroalkenyl or 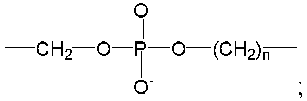
A is a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
B is -OH, a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
Q is -H, -COOH, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, -N(R)₃⁺ or 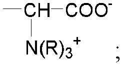
R is each independently H or a linear or branched C₁₋₂₀ alkyl; and
n is 0, 1 or 2;

71. A pharmaceutical composition comprising a lipid composition and a nucleic acid, wherein the lipid composition comprises one or more compounds selected from the group consisting of lipids 72, 73, 74, 75, 76, 77, 78, 79, 80, 82, 83, 86, 87, 90, 91, 92, 95, 97, 98, 99, 100, 101, 102, 107, 111, 112, 113, 114, 115, 116, 117, 118, and 119, or a salt, a hydrate or a solvate thereof.

72. The pharmaceutical composition according to claim 70 or 71, wherein the pharmaceutical composition is formulated for oral administration, or administration by inhalation, via the digestive tract, or via the respiratory tract.

73. The pharmaceutical composition according to claim 70 or 71, wherein the pharmaceutical composition is used to deliver the nucleic acid by in vitro cell delivery, or by in vivo gastrointestinal delivery.

74. The pharmaceutical composition according to claim 70 or 71, wherein the pharmaceutical composition is an oral pharmaceutical composition.

75. The pharmaceutical composition according to any one of claims 70 to 74, wherein at least part of or all of the lipid composition and the nucleic acid exist in the form of a lipid-nucleic acid mixture.

76. The pharmaceutical composition according to claim 75, wherein the lipid-nucleic acid mixture is prepared by a heating method, by a reverse evaporation method, or by direct mixing.

77. The pharmaceutical composition according to claim 76, wherein the heating method comprises mixing the lipid composition with the nucleic acid to obtain a mixture, and heating the mixture at a temperature in a range selected from 25°C to 100°C, 50°C to 100°C, 80°C to 100°C, or 95°C to 100°C.

78. The pharmaceutical composition according to claim 77, wherein the mixture is heated at a temperature selected from 37°C, 60°C, 80°C or 100°C.

79. The pharmaceutical composition according to any one of claims 76 to 78, wherein the mixing the lipid composition with the nucleic acid comprises adding a solution of the lipid composition in an organic solvent to an aqueous solution of the nucleic acid.

80. The pharmaceutical composition according to claim 76, wherein the reverse evaporation method comprises mixing an aqueous solution of the nucleic acid with a solution of the lipid compound in an organic solvent to obtain a mixed solution, and then removing the organic solvent in the mixed solution at a temperature of 25°C to 70°C, 30°C to 65°C, or 40°C to 60°C.

81. The pharmaceutical composition according to claim 80, wherein the organic solvent in the mixed solution is removed at a temperature of 55°C.

82. The pharmaceutical composition according to any one of claims 76 and 80 to 81, wherein the reverse evaporation method comprises adding the aqueous solution of the nucleic acid to the solution of the lipid composition in an organic solvent.

83. Use of the pharmaceutical composition according to any one of claims 70 to 82 in the manufacture of a medicament for preventing and/or treating disease that can be prevented and/or treated by the nucleic acid, or for in vivo delivering the nucleic acid to a subject in need thereof.

84. A kit comprising:
one or more compounds of Formula (I), or a pharmaceutically acceptable salt, hydrate or solvate thereof provided in a first container: 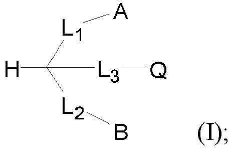 and
a nucleic acid provided in a second container;
wherein
L₁ is absent, or is -CH₂-O-C(O)-, -CH₂-O- or -CR(OH)-;
L₂ is absent, or is -O-C(O)- or -NH-C(O)-;
L₃ is a linear or branched C₁₋₂₀ alkyl, a linear or branched C₁₋₂₀ alkenyl, a linear or branched C₁₋₂₀ heteroalkyl, a linear or branched C₁₋₂₀ heteroalkenyl or 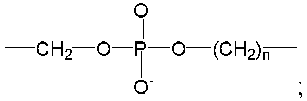
A is a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
B is -OH, a linear or branched C₁₋₂₀ alkyl, a linear or branched C₁₋₂₀ alkenyl, a linear or branched C₁₋₂₀ heteroalkyl, or a linear or branched C₁₋₂₀ heteroalkenyl;
Q is -H, -COOH, a linear or branched C₁₋₂₀ alkyl substituted by hydroxyl, a linear or branched C₁₋₂₀ alkenyl substituted by hydroxyl, a C₃₋₂₀ cycloalkyl substituted by hydroxyl, a C₃₋₂₀ cycloalkenyl substituted by hydroxyl, -N(R)₃⁺ or 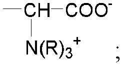
R is each independently H or a linear or branched C₁₋₂₀ alkyl; and
n is 0, 1 or 2;

85. A kit comprising
one or more compounds, or a pharmaceutically acceptable salt, hydrate or solvate thereof provided in a first container; and a nucleic acid provided in a second container, wherein the one or more compounds is/are selected from the group consisting of lipids 72, 73, 74, 75, 76, 77, 78, 79, 80, 82, 83, 86, 87, 90, 91, 92, 95, 97, 98, 99, 100, 101, 102, 107, 111, 112, 113, 114, 115, 116, 117, 118, and 119.

86. The kit according to claim 84 or 85, wherein at least part of or all of the lipid composition and the nucleic acid are prepared into a lipid-nucleic acid mixture prior to use.

87. The kit according to claim 86, wherein the lipid-nucleic acid mixture is prepared by a heating method, by a reverse evaporation method, or by direct mixing.

88. The kit according to claim 87, wherein the heating method is performed at a temperature in a range selected from 25°C to 100°C, 50°C to 100°C, 80°C to 100°C, or 95°C to 100°C.

89. The kit according to claim 88, wherein the heating method is performed at a temperature selected from 37°C, 60°C, 80°C or 100°C.

90. The kit according to any one of claims 87 to 89, wherein the heating method comprises adding a solution of the lipid composition in an organic solvent to an aqueous solution of the nucleic acid.

91. The kit according to claim 87, wherein the reverse evaporation method is performed at a temperature in a range selected from 25°C to 70°C, 30°C to 65°C, or 40°C to 60°C.

92. The kit according to claim 91, wherein the reverse evaporation method is performed at a temperature of 55°C.

93. The kit according to any one of claims 87 and 91 to 92, wherein the reverse evaporation method comprises adding an aqueous solution of the nucleic acid to a solution of the lipid composition in an organic solvent.

94. The kit according to any one of claims 84 to 93, wherein the kit is formulated into an oral pharmaceutical composition.

95. The kit according to any one of claims 84 to 93, wherein the kit is formulated for oral administration, or administration by inhalation, via the digestive tract, or via the respiratory tract.

96. The kit according to any one of claims 84 to 93, wherein the kit is formulated for delivering the nucleic acid by in vitro cell delivery, or by in vivo gastrointestinal delivery.

97. Use of the kit according to any one of claims 84 to 96 in the manufacture of a medicament for preventing and/or treating disease that can be prevented and/or treated by the nucleic acid, or for in vivo delivering the nucleic acid to a subject in need thereof.

98. A method for delivering a nucleic acid to a target cell, comprising administering, to the target cell, the pharmaceutical composition according to any one of claims 70 to 82, or a lipid-nucleic acid mixture prepared with the kit according to any one of claims 84 to 96.

99. A method for in vivo delivering a nucleic acid to a subject in need thereof, comprising administering, to the subject, the pharmaceutical composition according to any one of claims 70 to 82, or a lipid-nucleic acid mixture prepared with the kit according to any one of claims 84 to 96.

100. The method according to claim 99, wherein the subject is a human or an animal, such as a mammal.

101. The method according to claim 99 or 100, wherein the nucleic acid is delivered in vivo to the blood circulation or to a target tissue/cell in the subject.

102. The method according to any one of claims 99 to 101, wherein the medicament is administered by oral administration, inhalation or injection.

103. The method according to any one of claims 99 to 102, wherein the medicament is administered via the digestive tract, or via the respiratory tract.

104. The method according to claim 103, wherein the medicament is administered by oral administration.
